(19) Europäisches Patentamt / European Patent Office / Office européen des brevets

(11) EP 4 298 151 B1

(12) EUROPEAN PATENT SPECIFICATION

(45) Date of publication and mention of the grant of the patent:
**06.11.2024 Bulletin 2024/45**

(21) Application number: **22709674.0**

(22) Date of filing: **25.02.2022**

(51) International Patent Classification (IPC):
***C08G 71/04*** *(2006.01)* ***C08G 18/75*** *(2006.01)*
***C08G 18/48*** *(2006.01)* ***C08G 18/24*** *(2006.01)*
***C09D 175/04*** *(2006.01)*

(52) Cooperative Patent Classification (CPC):
**C08G 71/04; C08G 18/246; C08G 18/4845; C08G 18/758; C09D 175/04**

(86) International application number:
**PCT/EP2022/054854**

(87) International publication number:
**WO 2022/180235 (01.09.2022 Gazette 2022/35)**

(54) **NEW ANTIMICROBIAL AND ANTITHROMBOGENIC MEDICAL DEVICE**

NEUE ANTIMIKROBIELLE UND ANTITHROMBOGENE MEDIZINISCHE VORRICHTUNG

NOUVEAU DISPOSITIF MÉDICAL ANTIMICROBIEN ET ANTITHROMBOGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2021 EP 21159597**

(43) Date of publication of application:
**03.01.2024 Bulletin 2024/01**

(73) Proprietors:
- **Université de Liège 4000 Liège (BE)**
- **Centre Hospitalier Universitaire de Liège 4000 Liège (BE)**

(72) Inventors:
- **LANCELLOTTI, Patrizio 4000 Liège (BE)**
- **OURY, Cécile 4000 Liège (BE)**
- **JEROME, Christine 4000 Liège (BE)**
- **DETREMBLEUR, Christophe 4000 Liège (BE)**
- **AQIL, Abdelhafid 4000 Liège (BE)**
- **MELO, Sofia 4000 Liège (BE)**
- **PIERRARD, Anna 4000 Liège (BE)**

(74) Representative: **De Clercq & Partners Edgard Gevaertdreef 10a 9830 Sint-Martens-Latem (BE)**

(56) References cited:
**WO-A1-2013/060950 WO-A1-2014/185856**

- **HANNES BLATTMANN ET AL: "Isocyanate- and Phosgene-Free Routes to Polyfunctional Cyclic Carbonates and Green Polyurethanes by Fixation of Carbon Dioxide", MACROMOLECULAR RAPID COMMUNICATIONS, vol. 35, no. 14, 30 July 2014 (2014-07-30), pages 1238 - 1254, XP055180325, ISSN: 1022-1336, DOI: 10.1002/marc.201400209**
- **GABRIEL ROKICKI ET AL: "Non-isocyanate polyurethanes: synthesis, properties, and applications", POLYMERS FOR ADVANCED TECHNOLOGIES, vol. 26, no. 7, 13 May 2015 (2015-05-13), GB, pages 707 - 761, XP055364978, ISSN: 1042-7147, DOI: 10.1002/pat.3522**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 298 151 B1

## Description

### FIELD OF THE INVENTION

The present invention relates to a new medical device comprising polyhydroxyurethane, particularly a catheter or a prosthetic heart valve and a method of manufacturing thereof.

The present invention also relates to the use of the medical device to load and release a bioactive molecule, particularly an antibacterial or anti-thrombotic molecule.

### BACKGROUND OF THE INVENTION

Medical devices for implant or insertion into the body of a patient are commonly used in the medical field. Catheters and indwelling tubing or prosthetic valves represent an important market size of medical devices.

Medical devices such as implants are necessary in the treatment of patients, but can generate a risk of inflammation or infection by microorganisms due to the use of such implants or the procedure of placement of the implant in the body.

Medical devices for implant or insertion into the body of a patient may also generate in-vivo calcification, degradation and thrombosis.

The inflammation or infection of an implant begins with a colonization of the implant surface or its surrounding area with bacteria or formation of biofilm on the surface of the implant.

To minimize the risk of inflammation or infection, it is becoming common that medical devices include an antibacterial coating.

Several methods have been used to prevent microbial colonization of catheters. The most common methods involve the use of antimicrobial loaded or antimicrobial coated catheters.

Nevertheless, the coating strategies often lead to a rapid release of the antimicrobial agent. Other coating are using traditional antibiotics, raising concerns about resistance over long term use.

Frontiers in Cellular and infection microbiology 9, art 37 of February 2019 describes how Auranofin releasing antibacterial and antibiofilm polyurethane (PU) is used in intravascular catheter coatings. The PU+ auranofin coated catheter is able to inhibit Methycillin resistant *Staphylococcus aureus* (MRSA) biofilm formation.

Nevertheless, polyurethane compounds suffer from some serious issues. Polyurethane are based on isocyanate chemistry comprising a reaction between a diol and an isocyanate to form a linear polyurethane.

The isocyanate based materials are toxic because they are synthetized from a toxic and hazardous phosgene. Also polyurethane based isocyanates are sensitive to moisture that can produce unstable monosubstituted carbamic acids, which decompose in a strongly exothermic reaction to produce urea and carbon dioxide.

Moreover *in vitro* and *in vivo* evaluation of polyurethane (PU) implants revealed problems associated with long-term implantation, i.e. degradation affecting the PU surface structure is observed. Accelerated calcification due to calcified host material penetration in the PU is likely to cause premature failure of the implant.

WO 2014/185856 discloses an implantable medical device comprising a polyhydroxyurethane and the production method thereof. Hannes Blattman et al in Macromolecular Rapid Communication (2014-07-30) pages 1238-1254 describes non-isocyanate polyurethane obtained from a polycyclocarbonate and a polyamine of different structure. WO 2013/060950 discloses medical device comprising hydroxyurethane obtained from cyclic carbonates and amino-compounds. Gabriel Rockiki (Polymer for AdvancesTechnology (2015-05-13), p707-761) describes non-isocyanates polyurethanes of different structures. Each of these documents describe an implantable medical device comprising a polyhydroxyurethane. There is however no indication about their suitability to load bioactive molecules or how they should be adapted for this purpose.

Consequently, there is a need for new affordable, safe and effective medical devices, particularly for catheter and for prosthetic valves.

### SUMMARY OF THE INVENTION

We have now found non-isocyanate polyurethanes (NIPU), also called polyhydroxyurethane (PHU) for use in the manufacture of a new medical device or for coating a medical device. The new medical device comprising one or more polyhydroxyurethane (PHU) can be loaded and release, or be grafted with bioactive molecules, particularly antibacterial, anti-thrombotic molecules or anti-calcification agents.

The use of non-isocyanate polyurethanes has many attractive features for biomedical applications, including low cost, excellent biocompatibility, low toxicity, and a variety of mechanical properties that allows their use in several manufacturing methods such as for example extrusion, co-extrusion, moulding or 3D printing methods.

DETAILED DESCRIPTION

It is an object of the present invention to provide a new medical device comprising a non-isocyanate polyurethane or polyhydroxyurethane (PHU).

The term implantable medical device as used herein refers to all implantable foreign material for clinical use in host mammal such as for prosthetic joints, pacemakers, implantable cardioverter-defibrillators, catheters, such as intravascular or urinary catheters, stent including coronary stent, prosthetic heart valves, intraocular lens, dental implants, breast implants, endotracheal tubes, gastrostomy tubes and the like.

Particularly the implantable medical device comprising polyhydroxyurethane (PHU) is a catheter that can be introduced in the host mammal to deliver fluids such as blood products, glucose solutions, medications diagnostic agents and so forth. Catheters may also be used for example to withdraw blood from vasculature in order to treat blood or to drain urine from the urinary bladder or for gathering of samples.

The term "catheter" according to the invention refers to urinary catheters, central venous catheters, cardiovascular catheters, neurovascular catheters, intravenous catheters, and specialty catheters, but also endotracheal tubes, cannulae, ophthalmic catheter and the like.

The catheter according to the invention comprises one or more polyhydroxyurethanes (PHU). The catheter according to the invention may be partially or totally made of one or more polyhydroxyurethane (PHU).

Additionally or alternatively, the catheter according to the invention may be partially or totally coated with one or more polyhydroxyurethane (PHU).

Particularly the medical device comprising one or more polyhydroxyurethane (PHU) is a prosthetic heart valve such as a polymeric valve, a mechanical valve or a bioprosthetic valve.

The prosthetic heart valve comprising one or more polyhydroxyurethane (PHU) may be partially or totally made of one or more polyhydroxyurethane (PHU).

Alternatively, the prosthetic heart valve comprising one or more polyhydroxyurethane (PHU) may be partially or totally coated with one or more polyhydroxyurethane (PHU).

In particular embodiments, the valves are mechanical valves. The mechanical valvesmay be mono or bi-leaflet heart valves made of either pyrolytic carbon or titanium coated with pyrolytic carbon and further coated with one or more polyhydroxyurethane (PHU).

In particular embodiments, the valves are bioprosthetic valves. The bioprosthetic valve also called tissue valve can be a bi-leaflet or tri-leaflet heart valve having tissues components provided by a donor animal and further coated with one or more polyhydroxyurethane (PHU).

In particular embodiments, the valves are polymeric valves. The Polymeric valve can be a bi-leaflet or tri-leaflet heart valve totally or partly made from one or more polyhydroxyurethane (PHU). The polymeric valve may also be totally or partly coated on its surface with PHU, when made from another polymeric composition.

The term "host mammal" as used herein refers to a human, but can also refer to an animal.

The new medical device comprising said one or more non-isocyanate polyurethanes or polyhydroxyurethane (PHU), as a coating at the surface thereof and/or as main component of the medical device's bulk composition, is advantageously more hemocompatible, inducing less coagulation and less platelet adhesion than medical device comprising isocyanate-based Polyurethane.

The new medical device comprising said one or more non-isocyanate polyurethanes or polyhydroxyurethane (PHU), can advantageously load and release one or more bioactive molecules for local therapeutical treatment.

***In a first aspect,*** the invention provides a medical device comprising one or more polyhydroxyurethane (PHU) based on formula (1), optionally obtained **by thermal polymerisation** wherein n is an integer greater or equal to 1 (n $\geq$ 1), preferably n is between 1 and 100, more preferably between 1 and 20. The polyhydroxyurethane obtained by thermal polymerisation is either a linear or a network structure.

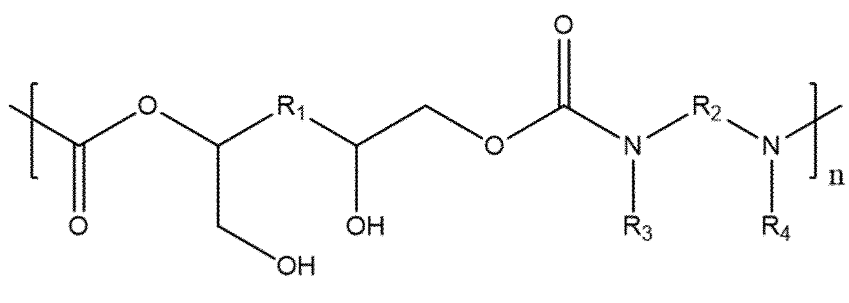

(1)

wherein $R_1$ is a linear or branched hydrocarbon chain, which may be unsubstituted or substituted and

wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms; preferably $R_1$ is polypropylene oxide, polyethylene oxide, polybutylene oxide, polytetrahydrofurane, or a mixture thereof;

wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon atoms, more preferably from 2 to 20 carbon atoms, even more preferably from 2 to 15 carbon atoms; or a mixture thereof;

$R_2$ is preferably an alkylene such as for example methylene, ethylene, methylene bis-cyclohexyl, heptamethylene, and the like;

wherein $R_3$ and $R_4$ are identical or different and represent an hydrogen atom (H), a straight or branched saturated hydrocarbon chain $C_{1-6}$-alkyl having 1 to 6 carbon atoms as for example methyl, ethyl, n-propyl, isopropyl, n-butyl, isobutyl, ter-butyl, n-pentyl, 2 methylbutyl, 3-methylbutyl, n-hexyl, 4-methylpentyl, neopentyl, n-hexyl, 1,2-dimethylpropyl, 2,2-dimethyl propyl, 1,2,2-trimethylpropyl, and the like; $C_{1-6}$-alkoxy alkyl having 1 to 6 carbon atoms as for example: methyloxy, ethyloxy, propoxy, isopropoxy, butoxy, pentoxy.

$R_3$ and $R_4$ are preferably identical and equal to hydrogen atom,

with the exception that if $R_3$ and/or $R_4$ represents an hydrogen atom, $R_1$ is not

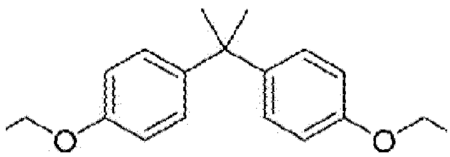
,
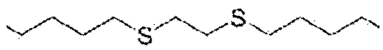
,

or

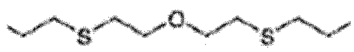
;

and with the exception that if $R_3$ and/or $R_4$ represents an hydrogen atom, then $R_2$ is not

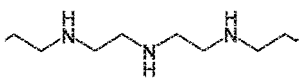
.

In some embodiments, when $R_3$ and $R_4$ represents an hydrogen atom, then $R_1$ is not

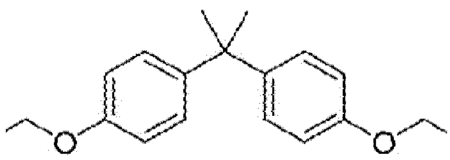
,
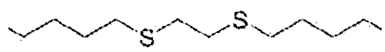
,

or

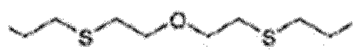

;

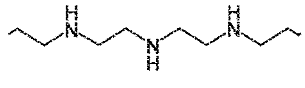

and when $R_3$ and $R_4$ represent an hydrogen atom, then $R_2$ is not

In some embodiments the medical device according to the invention is characterised in that the one or more polyhydroxyurethane (PHU) is based on formula (1)

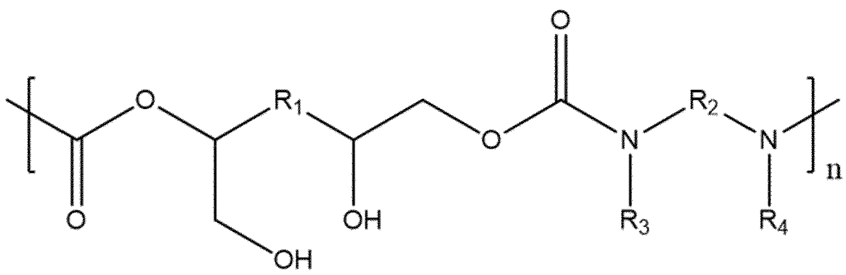

(1)

wherein $R_1$ is a linear or branched hydrocarbon chain, which may be unsubstituted or substituted and

wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms;

wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon atoms, more preferably from 2 to 20 carbon atoms, even more preferably from 2 to 15 carbon atoms, or a mixture thereof; preferably $R_2$ is an alkylene;

wherein $R_3$ and $R_4$ are identical or different and represent H, a straight or branched saturated hydrocarbon chain $C_{1-6}$-alkyl having 1 to 6 carbon atoms ; $C_{1-6}$-alkoxy alkyl having 1 to 6 carbon atoms

and wherein n is an integer $\geq 1$, preferably n is between 1 and 100, more preferably between 1 and 20.

In some embodiments the medical device according to the invention, wherein $R_1$ is polypropylene oxide, polyethylene oxide, polybutylene oxide, polytetrahydrofuran, or a mixture thereof and $R_2$ is an alkylene.

In some embodiments the medical device according to the present invention, wherein the polyhydroxyurethane based on formula (1) is obtained by thermal polyaddition of polycyclocarbonate of formula (2)

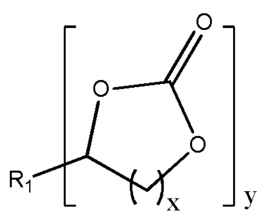

(2)

wherein R1 is a carbon bond between a cyclic carbonate ring or is a linear or branched hydrocarbon chain, which may be unsubstituted or substituted and wherein one or more hydrocarbon groups of said hydrocarbon chain may

be replaced by an heteroatom, a ketone, a cycloalkyl, an heterocycle, an aryl or a heteroaryl, each of which may be substituted or unsubstituted, said hydrocarbon chain having from 2 to 40 carbon atoms ; preferably $R_1$ is polypropylene oxide, polyethyleneoxide, polybutylene oxide, polytetrahydrofurane or a mixture thereof;

with a polyamine of formula (3)

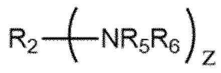

(3)

wherein z is an integer between 2 and 6;

wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms;

wherein $R_5$ and $R_6$ are identical or different and represent H, a straight or branched saturated hydrocarbon chain $C_{1-6}$-alkyl having 1 to 6 carbon atoms; preferably $R_5$ and $R_6$ are hydrogen.

In some embodiments the medical device according to the invention is characterised-in-that the polycyclocarbonate of formula (2) is a polypropylene glycol)-bis-cyclocarbonate (PPGbisCC) of formula (5) wherein m is an integer $\geq 1$, preferably between 1 and 100, most preferably between 1 and 10;

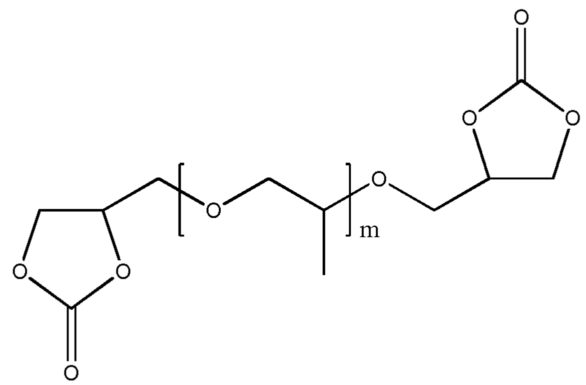

(5)

and the polyamine is 4-4'methylene-bis-(cyclohexylamine) (MBCHA) of formula(4)

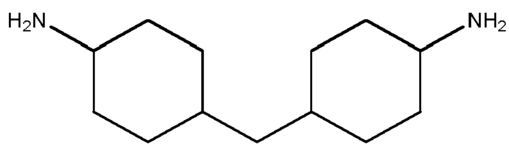

(4).

In some embodiments the medical device according to the invention is characterised-in-that the polyamine is 4,4'methylene-bis-(cyclohexylamine) of formula (4) in combination with tris(2-aminoethyl)amine (TAEA) of formula (6).

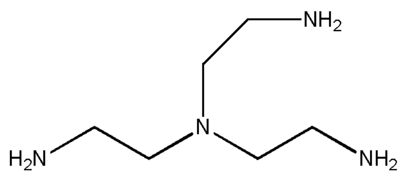

(6)

In some embodiments the medical device according to the invention is characterised-in-that the polycyclocarbonate is polytetrahydrofuranebiscyclocarbonate of formula (8) wherein p is an integer is ≥ 1, preferably between 1 and 100, most preferably between 1 and 10;

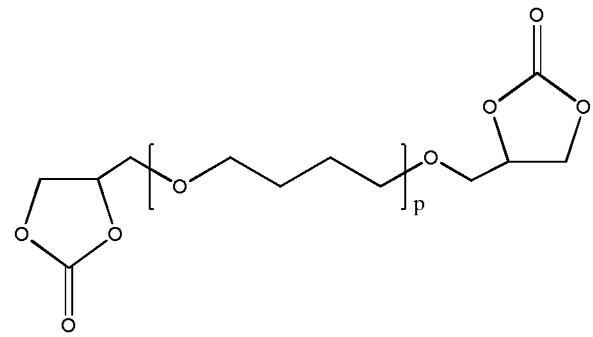
(8)

and the polyamine is 4-4'methylene-bis-(cyclohexylamine) (MBCHA) of formula (4).

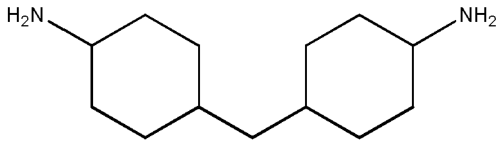
(4).

In some embodiments the medical device according to the invention, characterised-in-that the polyamine is polyamine is 4-4'methylene-bis-(cyclohexylamine) of formula (4) in combination with tris(2-aminoethyl)amine (TAEA) of formula (6)

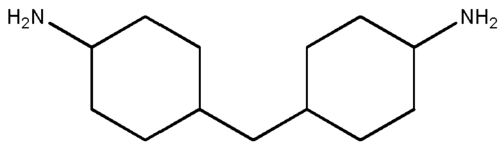
(4)
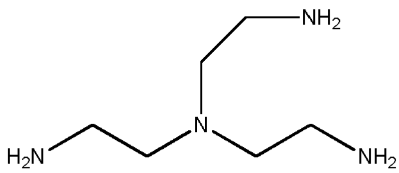
(6).

In some embodiments, the polyhydroxyurethane (PHU) of the medical device according to the invention is based on formula (9):

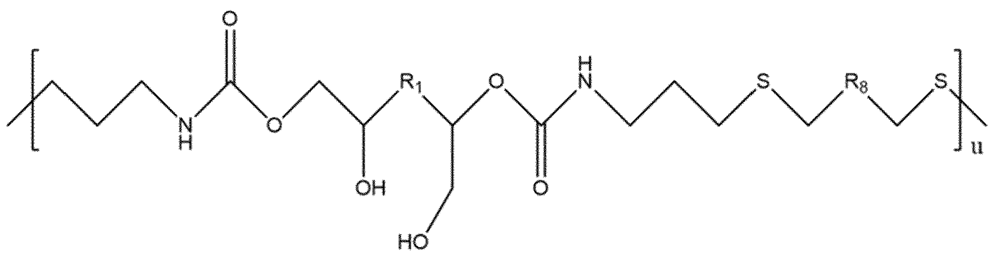
(9)

wherein $R_1$ is a linear or branched hydrocarbon chain, which may be unsubstituted or substituted and wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms ; preferably $R_1$ is polypropyleneoxide, polyetylene oxide, polybutylene oxide, polytetrahydrofurane, or a mixture thereof;
wherein $R_8$ is is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene; and

wherein u is an integer ≥ 1, preferably between 1 and 10 more preferably between 1 and 5.

In some embodiments the polyhydroxyurethane (PHU) of the medical device according to the invention is based on formula (9) and is obtained by UV polyaddition of a polyallylcarbamatehydroxy derivative of formula (10) with a dithiol of formula (11) and a photoinitiator;

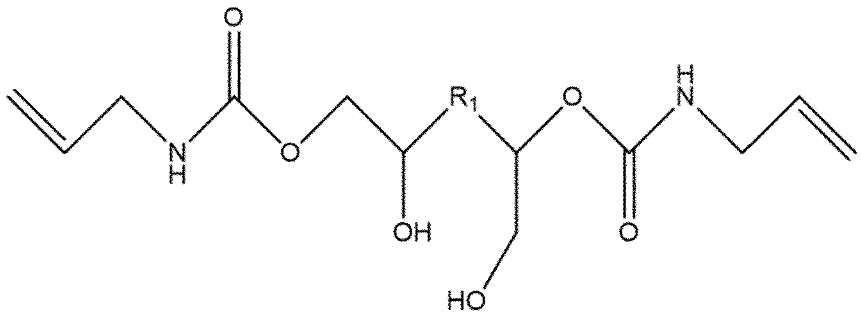

(10)

wherein $R_1$ is a linear or branched hydrocarbon chain, which may be unsubstituted or substituted and wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms; preferably $R_1$ is polypropylene oxide, polyethylene oxide, polybutylene oxide, polytetrahydrofurane, or a mixture thereof;

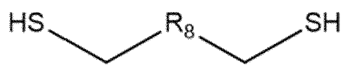

(11)

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene.

In some embodiments the medical device according to the invention is characterised in that the polyallylcarbamatehydroxy derivative is a polyallylcarbamatehydroxypropyleneoxide of formula(14)

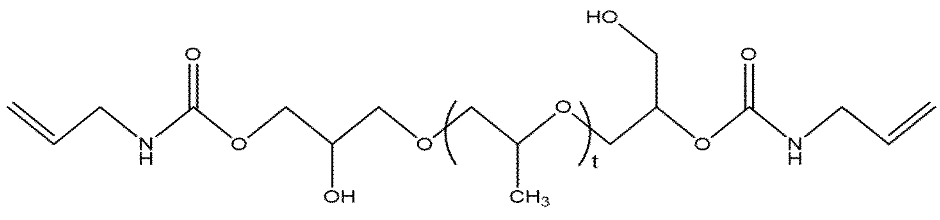

(14)

wherein t ≥ 1, preferably t is between 1 and 1000 and most preferably between 1 and 20.

In some embodiments, the medical device of the invention is a catheter or a prosthetic heart valve.

The resent invention also encompasses a method of manufacturing a medical device comprising polyhydroxyurethane as described herein; characterised in that the method comprises a mixture step (a) or (b) corresponding respectively to a thermal or UV polymerisation:

a) mixing a polycyclocarbonate of formula (2) with a polyamine of formula (3) and optionally a catalyst to obtain, by thermal polymerization, a polyhydroxyurethane based on formula (1);

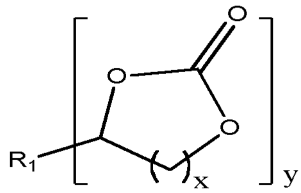 (2)

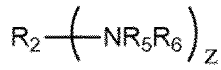 (3)

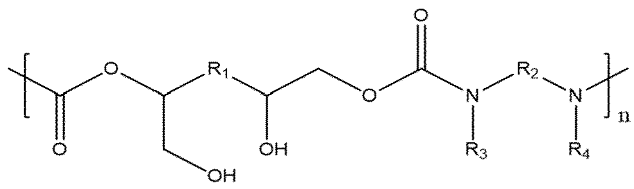 (1)

wherein $R_1$ is a carbon bond between a cyclic carbonate ring or is a linear or branched hydrocarbon chain, which may be unsubstituted or substituted and wherein one or more hydrocarbon groups of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 40 carbon atoms ; preferably $R_1$ is polypropylene oxide, polyethyleneoxide, polybutylene oxide, polytetrahydrofurane or a mixture thereof;

wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon atoms, more preferably from 2 to 20 carbon atoms, even more preferably from 2 to 15 carbon atoms;

wherein $R_5$ and $R_6$ are identical or different and represent H, a straight or branched saturated hydrocarbon chain $C_{1-6}$-alkyl having 1 to 6 carbon atoms; preferably $R_5$ and $R_6$ are hydrogen.

wherein $R_3$ and $R_4$ are identical or different and represent H, a straight or branched saturated hydrocarbon chain $C_{1-6}$-alkyl having 1 to 6 carbon atoms ; $C_{1-6}$-alkoxy alkyl having 1 to 6 carbon atoms

and wherein n is an integer $\geq 1$, preferably n is between 1 and 100, more preferably between 1 and 20.

b) mixing a polyvinylcarbamatehydroxy derivative of formula (10) with a dithiol of formula (11) and a photoiniator to obtain, by UV polymerization, a polyhydroxyurethane based on formula (9);

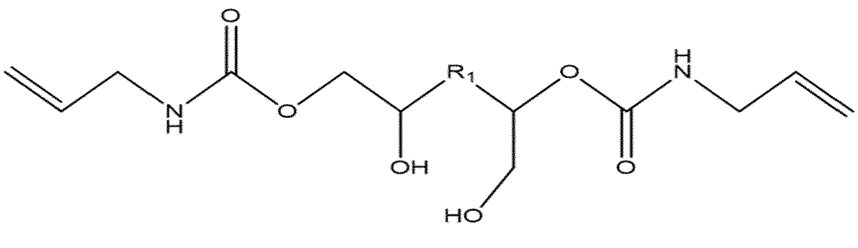 (10)

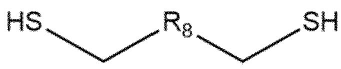 (11)

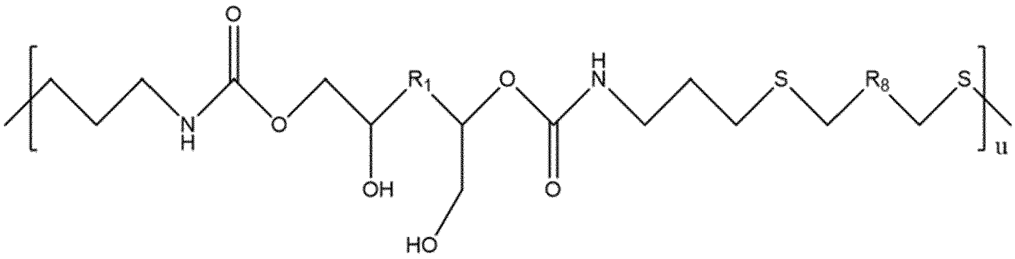

(9)

wherein $R_1$ is a linear or branched hydrocarbon chain, which may be unsubstituted or substituted and

wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms ; preferably $R_1$ is polypropyleneoxide, polyethylene oxide, polybutylene oxide, polytetrahydrofurane, or a mixture thereof;

wherein $R_8$ is is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene; and

wherein u is an integer ≥ 1, preferably between 1 and 10 more preferably between 1 and 5.

The present invention also encompasses a method of coating a medical device comprising a polyhydroxyurethane as described herein; the method comprising depositing onto a surface of the medical device at least one layer of polyhydroxyurethane based on formula (1) or on formula (9)

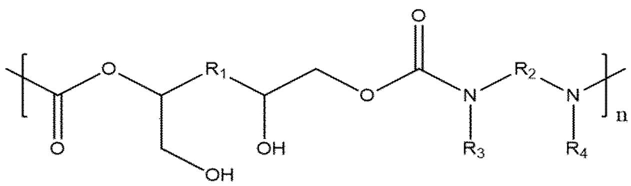

(1)

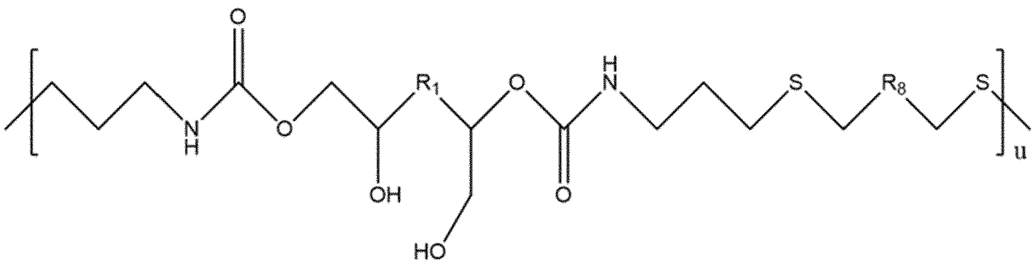

(9)

wherein $R_1$ is a carbon bond between a cyclic carbonate ring or is a linear or branched hydrocarbon chain, which may be unsubstituted or substituted and wherein one or more hydrocarbon groups of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 40 carbon atoms ; preferably $R_1$ is polypropylene oxide, polyethyleneoxide, polybutylene oxide, polytetrahydrofurane or a mixture thereof;

wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon

atoms, more preferably from 2 to 20 carbon atoms, even more preferably from 2 to 15 carbon atoms;

wherein $R_3$ and $R_4$ are identical or different and represent H, a straight or branched saturated hydrocarbon chain $C_{1-6}$-alkyl having 1 to 6 carbon atoms; $C_{1-6}$-alkoxy alkyl having 1 to 6 carbon atoms;

wherein $R_8$ is is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene;

wherein u is an integer ≥ 1, preferably between 1 and 10 more preferably between 1 and 5;

and wherein n is an integer ≥ 1, preferably n is between 1 and 100, more preferably between 1 and 20.

In some embodiments, the medical device according to the invention further comprises a bioactive molecule, preferably an antibacterial or an anti-thrombotic agent.

PHUs based on formula (1) with a linear structure such as when $R_3$ and $R_4$ represents an hydrogen atom can advantageously be used in Fused Filament Fabrication(FFF) or Fused Deposition Modeling (FDM), also called 3D printing

In some embodiments the polyhydroxyurethane (PHU) of formula (1) is obtained by thermal polyaddition of a polyamine of formula (3) with a polycyclocarbonate of formula (2)

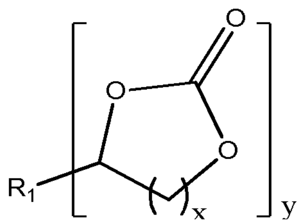
(2)

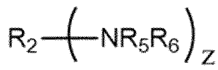
(3)

wherein $R_1$ is a carbon bond between a cyclic carbonate ring or is a linear or branched hydrocarbon chain, which may be unsubstituted or substituted and wherein one or more hydrocarbon groups of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms;

preferably $R_1$ is polypropylene oxide, polyethylene oxide, polybutylene oxide, polytetrahydrofurane, or a mixture thereof;

wherein y is an integer superior or equal to 2; preferably from 2 to 6; more preferably 2 or 3; and

wherein x is an integer between 1 and 4 which represent the number of methylene groups . According to the invention, when x assumes values of 1 to 4, the resulting cyclic carbonate groups is made of five, six, seven and / or eight atoms;

preferably, the integer x is 1, in a pentacyclic carbonate group.

Examples of polycyclocarbonates used in the present invention are:

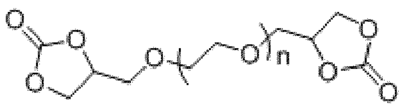

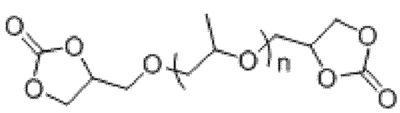

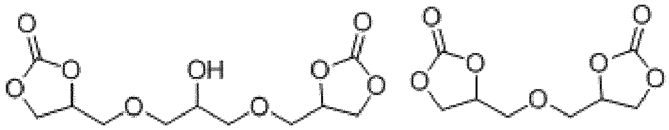

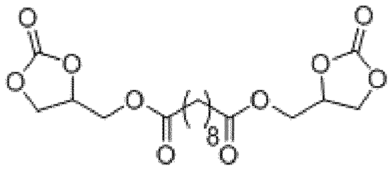

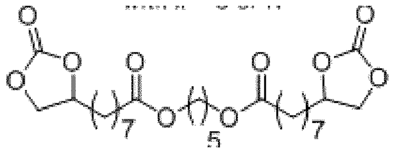

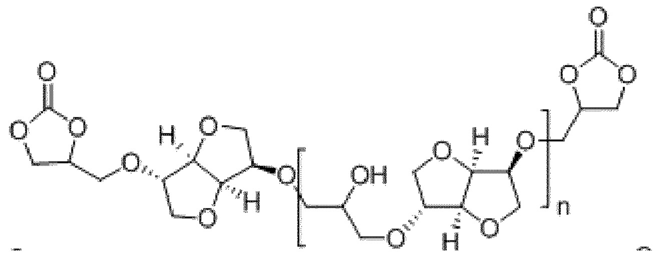

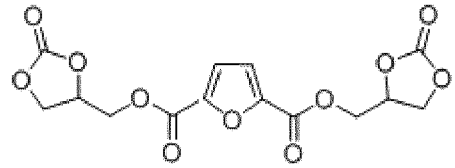

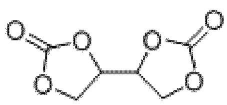

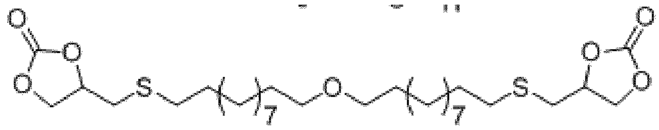

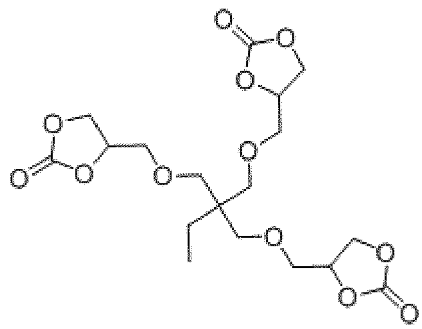

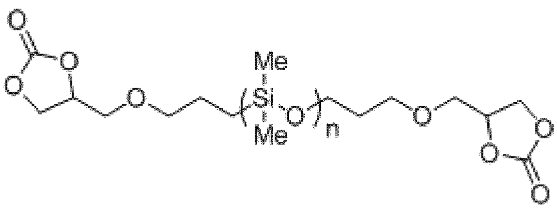

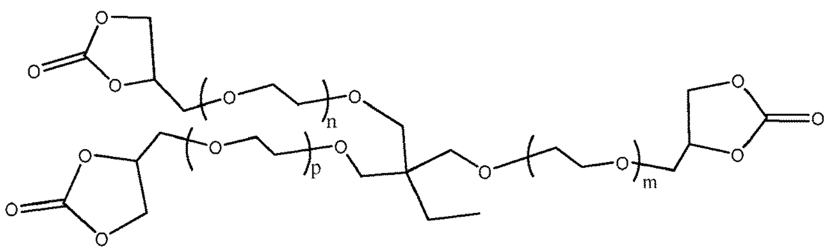

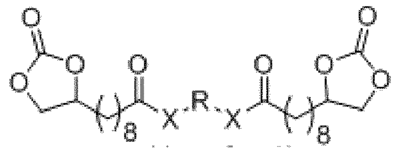

wherein X is an oxygen atom (O) or a nitrogen atom of (N);

and wherein R is one of the group selected from:

$-CH_2)_n-$

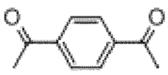

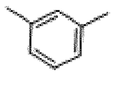

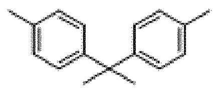

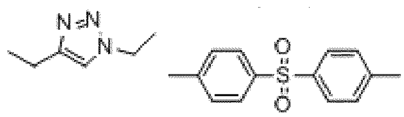

The polycyclocarbonates can be prepared by any methods known by the art, for instance from polyols by converting all or part of the alcohol functions of said polyol into glydicylether functions, followed by carbonation of said glycidylether functions as described in EP 3 199 569A1, or by epoxidation of molecules bearing at least 2 external double bonds, followed by the (organo)catalyzed carbon dioxide coupling reaction as described by L.-N. He & al., "One-pot stepwise synthesis of cyclic carbonates directly from olefins with CO2 promoted by K2S2O8/NaBr", J. CO2 Util., 2016, 16, 313-317 and by R. Wang & al., "Direct Synthetic Processes for Cyclic Carbonates from Olefins and CO2", Catal. Surv. from Asia, 2011, 15, 49-54, and by C. Detrembleur & al., "Organocatalyzed coupling of carbon dioxide with epoxides for the synthesis of cyclic carbonates: catalyst design and mechanistic studies", Catal. Sci. Technol., 2017, 7, 2651.

In the polyamine of formula (3),

$$R_2 \left( NR_5R_6 \right)_z \quad (3)$$

z is an integer greater or equal to 2; preferably between 2 to 6; most preferably equal to 2 or 3;

$R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon atoms, more preferably from 2 to 20 carbon atoms, even more preferably from 2 to 15 carbon atoms; preferably $R_2$ is an alkylene group such as methylene, ethylene, methylene-bis-cyclohexyl, heptamethylene, a polyether with a molecular weight preferably varying from 300 to 10,000g/mol, a polyester with a molecular weight varying from 300 to 10,000g/mol, a polysiloxane with a molecular weight varying from 300 to 10,000g/mol, or a mixture thereof;

$R_5$ and $R_6$ are identical or different and represent H, a straight or branched saturated hydrocarbon chain $C_{1-6}$-alkyl having 1 to 6 carbon atoms as for example methyl, ethyl, n-propyl, isopropyl, n-butyl, isobutyl, ter-butyl, n-pentyl, 2 methylbutyl, 3-methylbutyl, n-hexyl, 4-methylpentyl, neopentyl, n-hexyl, 1,2-dimethylpropyl, 2,2-dimethyl propyl, 1,2,2-trimethylpropyl, and the like, or a $C_{3-8}$ - cycloalkyl as for example cyclopropyl, cyclobutyl, cyclopentyl, cyclohexyl, cycloheptyl or cyclooptyl;

$R_5$ and $R_6$ are preferably hydrogen, with the exception that if $R_5$ and/or $R_6$ are hydrogen, then $R_2$ is not

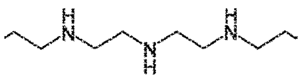

.

The polyamine of formula (3) can be a primary, a secondary or a tertiary polyamine.

The polyamines of formula (3), are for example diamines, in particular linear aliphatic diamines, such as 1,2-diaminoethane, 1,3-diaminopropane, butane-1,4-diamine, pentane-1,5-diamine, 1,6-diaminohexane, or 1,12-diaminododecane, hexamethylenediamine, 4,9-dioxa-1,12-dodecanediamine, 2,2'-(ethane-1,2-diylbis(oxy))diethanamine or cyclic aliphatic diamines, such as isophoronediamine (IPDA), triamines, such as tris(2-aminoethyl)amine (TAEA), or any other polyamines, such as polyethylene imine or dimeric fatty acid diamines or aromatic diamine, such as o-xylylenediamine, m-xylylenediamine, p-xylylenediamine or 1,2-diphenylethylenediamine and the like. The use of multifunctional amines with long chain segments and/or a low number of $-NH_2$ functionalities may yield a linear and flexible structure; while the use of polyamine with short chain segments and a higher number of $-NH_2$ functionalities may yield crosslinked and more rigid network structure.

Preferably the polyamine of formula (3) is a primary polydiamine, most preferably 4-4'-methylenebis(cyclohexylamine) (MBCHA) of formula (4)

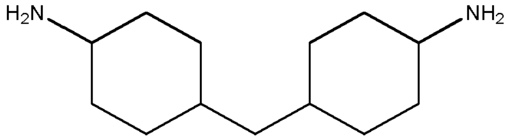

(4)

to provide a polyhydroxyurethane PHU of formula (1) wherein $R_2$ is a methylenebis(cyclohexyl) group

Alternatively a primary polyamine can be used in combination with another primary polyamine or in combination with a secondary or tertiary polyamine.

The term "primary polyamine" as used herein refers to a polyamine of formula (3) wherein $R_5$ and $R_6$ are hydrogen.

The term "secondary polyamine" as used herein refers to a polyamine of formula (3) wherein either $R_5$ or $R_6$ is hydrogen.

The term "ternary polyamine" as used herein refers to a polyamine of formula (3) wherein $R_5$ and $R_6$ are both different from hydrogen.

The thermal polyaddition is a ring opening reaction of the polycyclocarbonate of formula (2) with a polyamine of formula (3) under temperature between 10 to 100°C, preferably between 65 and 80°C.

The polycyclocarbonate of formula (2) reacts with the polyamine used as a nucleophile (Nu)).

The thermal polyaddition is preferably carried out with a catalyst used to increase the kinetics of the carbonate/amine reaction and to permit the decarboxylation of the cyclic carbonate. A wide range of catalysts can be used (see for instance Blain et al., Green Chemistry 2014, 16, 4286). The choice of suitable catalyst depends on the specific formulation used but also on the temperature used to form the poyhydroxyurethane. As non-limiting examples, the catalyst may be chosen from amine catalysts, such as triazabicyclodecene (TBD), 1,4-diazabicyclo[2.2.2]octane (DABCO), 1,8-diazabicyclo[5.4.0]undec-7-ene (DBU), 7-Methyl-1,5,7-triazabicyclo[4.4.0]dec-5-ene (MTBD), or other guanidines and amidines, trimethylhydroxyethyl ethylene diamine, trimethylaminopropylethanolamine, dimethylethanolamine, bis(2-dimethylaminoethyl) ether, triethylenediamine, dimethylaminocyclohexane, N-methyl morpholine, dimethylaminopyridine (DMAP), trimethylamine ($NEt_3$), trimethylamine, phosphazenes, phosphines (triaryl and trialkylphosphines).

The catalyst may also be chosen from ionic salts or ionic liquids composed of a combination of a cation and an anion. The cation may be selected from alkali metals such as $Na^+$, $Li^+$, $K^+$, $Cs^+$ or other metal such as $Mg^{2+}$, $Ca^{2+}$ or organic cations including ammonium , phosphonium , imidazolium, pirazolium, triazolium, tetrazolium, pyridinium, piperidinium, pyrrolidinium, guanidinium or amidinium as described in WO2021/004993.

The term "polyether" as used herein refers to a straight or branched aliphatic or aromatic polymeric chain comprising two or more ether (-O- ) linkage.

The term polyether particularly refers to polyalkylene oxide $C_{2n}H_{4n}O_{n+1}$ such as polypropylene oxide also called polyproylene glycol, polyethylene oxide also called polyethylene glycol, polybutylene oxide also called polybutylene glycol or a mixture thereof.

The term polyethylene oxide refers to:

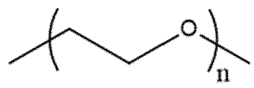

wherein n is an integer that is sufficiently large to have molecular weight from about 300 to about 10,000g/mol.

The term polypropylene oxide refers to:

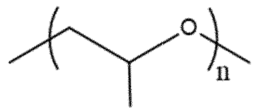

wherein n is an integer that is sufficiently large to have molecular weight from about 300 to about 10,000g/mol.

The term polybutylene oxide refers to:

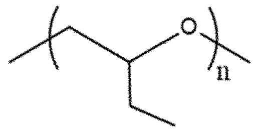

wherein n is an integer that is sufficiently large to have molecular weight from about 300 to about 10,000 g/mol.

The term "polyester" as used herein refers to a straight or branched aliphatic or aromatic polymeric chain comprising two or more carboxyl (-COO- ) linkages.

The term "polyol" as used herein refers to a straight or branched aliphatic or aromatic polymeric chain comprising two or more alcohol group.

The term "polysiloxane" or silicone as used herein refers to a polymer consisting of a silicon-oxygen backbone of formula (18)
wherein n is an integer that is sufficiently large to have molecular weight from about 300 to about 10000g/mol.

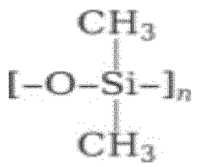

(18)

The term "$C_{1-6}$-alkyl" as used herein, alone or in combination, refers to a straight or branched, saturated hydrocarbon chain having 1 to 6 carbon atoms such as e.g. methyl, ethyl, n-propyl, isopropyl, n-butyl, sec-butyl, isobutyl, tert-butyl, n-pentyl, 2-methylbutyl, 3-methylbutyl, n-hexyl, 4-methylpentyl, neopentyl, n-hexyl, 1,2-dimethylpropyl, 2,2-dimethylpropyl and 1,2,2-trimethylpropyl.

The term "$C_{1-6}$-alkoxy" as used herein, alone or in combination, refers to a straight or branched monovalent substituent comprising a $C_{1-6}$-alkyl group linked through an ether oxygen having its free valence bond from the ether oxygen and having 1 to 6 carbon atoms e.g. methoxy, ethoxy, propoxy, isopropoxy, butoxy, pentoxy.

The term "ketone" as used herein refers to a C=O group.

The term "heteroatom" as used herein refers to an atom selected from N, O, S, Si and S(O)n (where n is 0, 1 or 2), SiO.

The term "cycloalkyl" as used herein refers to a monovalent or bivalent 3 to 8 membered carbon ring, for example cyclopropyl, cyclobutyl, cyclopentyl, cyclohexyl, cycloheptyl or cyclooctyl.

The term "heterocycle" as used herein refers monovalent or bivalent non-aromatic mono- or bi-cyclic radical of four to nine ring atoms in which one to three ring atoms are heteroatoms independently selected from N, O and S(O)n (where n is 0, 1 or 2), with the remaining ring atoms being C. Particular is piperidyl or a cyclic carbonate.

The term "aryl" as used herein refers a monovalent or bivalent aromatic carbocyclic group containing 6 to 14, particularly 6 to 10, carbon atoms and having at least one aromatic ring or multiple condensed rings in which at least one ring is aromatic. Examples include phenyl, benzyl, naphthyl, biphenyl, anthryl, azalenyl or indanyl.

The term "heteroaryl" as used herein refers a monovalent or bivalent cyclic aromatic group containing 1, 2 or 3 heteroatoms, having at least one aromatic ring or multiple condensed rings in which at least one ring is aromatic. The

aromatic ring may be a 6 membered ring, such as pyridinyl, or a 5-membered ring, such as thiazolyl, isoxazolyl, isothiazolyl, oxadiazolyl, imidazolyl, triazolyl or thiadiazolyl.

The term "allyl" as used herein refers to a $CH_2$=CH-$CH_2$- group

*In one particular embodiment,* the present invention provides a medical device comprising one or more polyhydroxyurethane (PHU) based on formula (7)

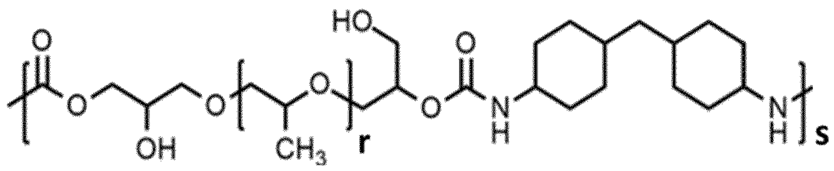

(7)

wherein r is an integer equal or superior to 1 (r >, 1) and s is an integer equal or superior to 1 (s ≥ 1) . Preferably r is between 1 and 100, more preferably between 1 and 10. Preferably s is between 1 and 500, more preferably between 1 and 20, most preferably between 1 and 6.

The polyhydroxyurethane (PHU) based on formula (7) also called polypropylene glycol based PHU may be obtained by thermal polyaddition between polypropylene glycol bis(cyclo-carbonate) (PPG biscc) of formula (5) wherein m is an integer equal or superior to 1 (m ≥ 1); preferably m is between 1 and 1000, more preferably between 1 and 20;

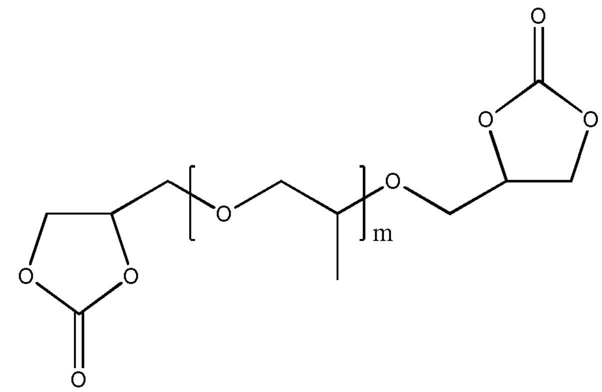

(5)

and 4-4'methylene-bis-(cyclohexylamine) (MBCHA) of formula (4)

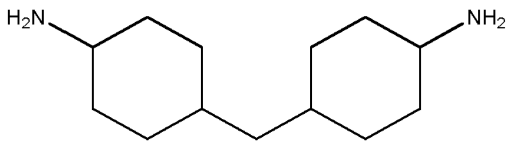

(4)

The Polypropylene glycol based PHU as described herein has the advantage to be more hemocompatible inducing less coagulation and platelet adhesion and less prone to bacterial infection than the corresponding polyurethane (PU) or polypropylene glycol based PU of formula (17) obtained by a reaction illustrated in figure 1 and wherein m and q are equal or superior to 1 (m ≥ 1) and (q >, 1).

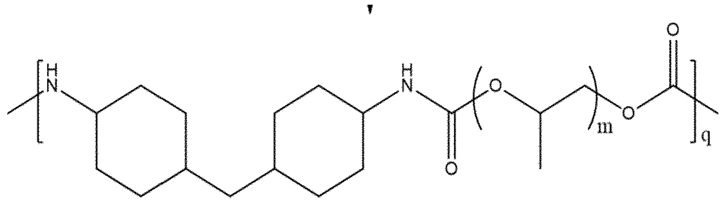

(PPG based PU)(17)

In a preferred embodiment, the present invention provides a medical device comprising one or more polyhydroxyurethane (PHU) obtained by polyaddition between polypropylene glycol bis(cyclo-carbonate) (PPG bisCC) of formula (5) and a diamine, preferably 4-4'methylene-bis-(cyclohexylamine) (MBCHA) of formula (4) and with a triamine, preferably tris(2-aminoethyl)amine (TAEA) of formula (6).

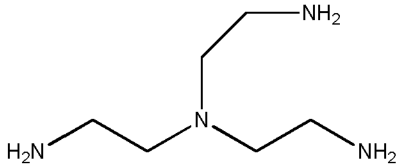

(6)

Thanks to the triamine addition, the resulting PHU becomes an interconnected 3D network with better mechanical properties.

*In another particular embodiment,* the present invention provides a medical device comprising one or more polyhydroxyurethane (PHU) obtained by thermal polyaddition of polytetrahydrofurane biscyclocarbonate (PTHF bisCC) of formula (8)

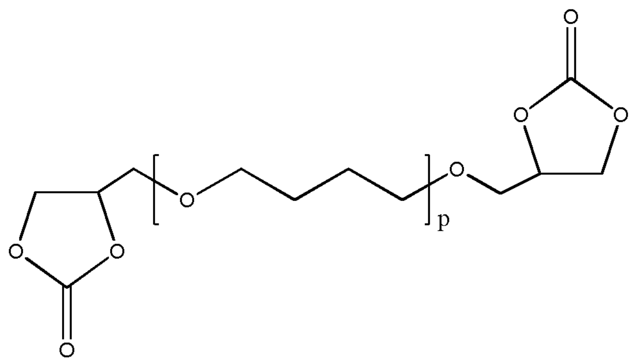

(8)

wherein p is an integer greater or equal to 1 ( ≥ 1 ) preferably p is between 1 and 100; most preferably p is between 1 and 10;

with a diamine preferably 4-4'-methylenebis(cyclohexylamine) of formula (4):

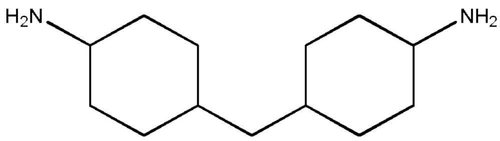

(4)

The resulting polytetrahydrofurane based PHU obtained by thermal polyaddition has the advantage to be more hemocompatible inducing less coagulation and platelet adhesion and less prone to bacterial infection than the corresponding polyurethane (PTHF based PU).

In another preferred embodiment, the present invention provides a medical device comprising one or more polyhydroxyurethane (PHU) obtained by thermal polyaddition of polytetrahydrofuran biscyclocarbonate of formula (8) with a diamine, preferably 4-4'-methylenebis(cyclohexylamine) of formula (4) and with a triamine, preferably tris(2-aminoethyl)amine (TAEA) of formula (6):

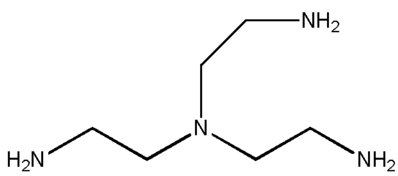

(6)

*In a second aspect,* the invention provides a medical device comprising one or more polyhydroxyurethane

(PHU) based on formula (9) and obtained by UV **polymerisation.**

When using UV polymerisation, the resulting polyhydroxyurethane based on formula (9) is a network structure.

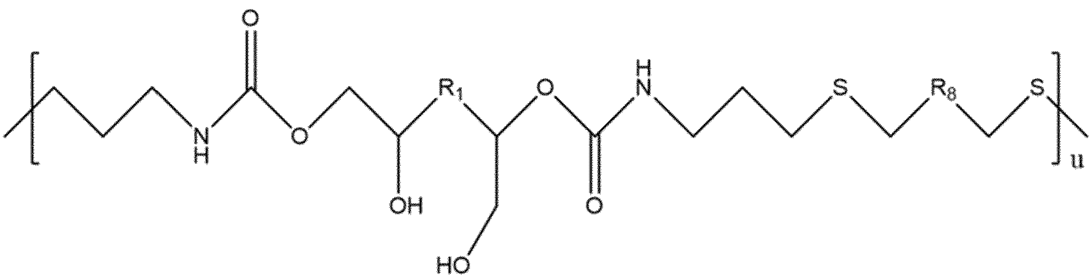

(9)

wherein u is an integer equal or superior to 1 (n >, 1); preferably n is between 1 and 100, more preferably between 1 and 20;

wherein $R_1$ is a linear or branched hydrocarbon chain, which may be unsubstituted or substituted and wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms;

preferably $R_1$ is polypropylene oxide, polyethylene oxide, polybutylene oxide, polytetrahydrofurane, or a mixture thereof;

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof;

$R_8$ is preferably an alkylene.

The polyhydroxyurethane (PHU) based on formula (9) is obtained by UV polymerisation in a two steps reaction: The first step is an aminolysis of polycarbonate of formula (2)

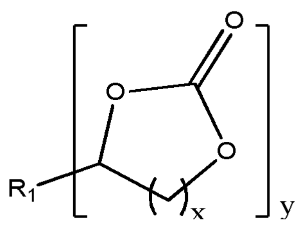

(2)

wherein $R_1$ is a linear or branched hydrocarbon chain, which may be unsubstituted or substituted and

wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms;

preferably $R_1$ is polypropylene oxide, polyethylene oxide, polybutylene oxide, polytetrahydrofurane, or a mixture thereof;

wherein x is an integer between 1 and 4 ; preferably equal to 1; and y is an integer equal or superior to 2; preferably from 2 to 6;

with a polyamine of formula (15) to obtain a polycarbamatehydroxy derivative bearing photoresponsive groups

$$R_2 - (NR_5R_6)_z \quad (15);$$

wherein $R_2$ is a linear or branched hydrocarbon unsaturated chain with 2 to 40 carbon atoms, said linear or branched hydrocarbon unsaturated chain bearing at least a photoresponsive group;

wherein $R_5$ is hydrogen and $R_6$ is hydrogen, a straigt or branched saturated hydrocarbon chain C $_{1-6}$ alkyl having 1 to 6 carbon atoms and

wherein z is an integer ≥ 1, preferably between 1 and 6;

Preferably with an amine containing one or more olefins such as for example an allylamine to obtain a polyallylcarbamate hydroxy derivative bearing photoresponsive groups on the linear or branched hydrocarbon chain, particularly at the extremities of the main chain.

In a preferred embodiment, the first step of aminolysis of polycarbonate of formula (2) is carried out with an allylamine to obtain a polyallylcarbamate hydroxy derivative of formula (10)

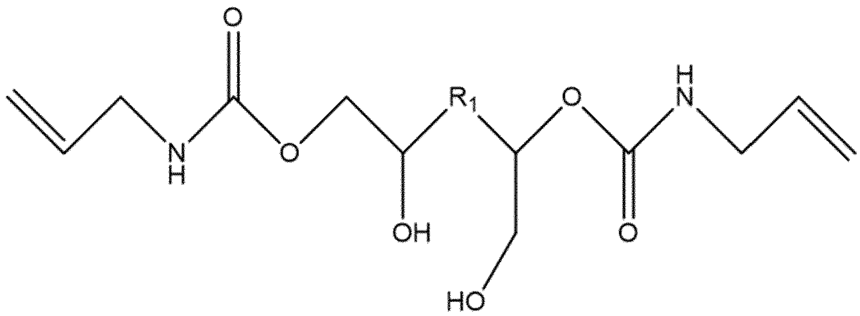

(10)

wherein $R_1$ is a linear or branched hydrocarbon chain, which may be unsubstituted or substituted and
wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms;
preferably $R_1$ is polypropylene oxide, polyethylene oxide, polybutylene oxide, polytetrahydrofurane, or a mixture thereof.

The polyallylcarbamate hydroxy derivative of formula (10) contains vinyl photoresponsive groups at the extremities of the main linear or branched hydrocarbon chain, but may also contain vinyl photoresponsive groups on the main chain.

The aminolysis may be carried out at a temperature between 20 and 100°C, preferably between 40°C and 60°C, most preferably 40°C.

The aminolysis may be catalysed with DBU, DMAP, DABCO, TBD, MTBD and DBN as illustrated hereafter:

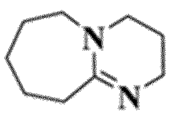 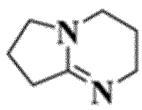 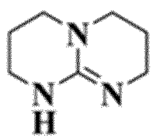 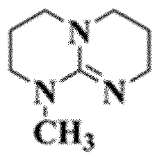 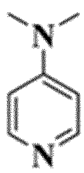

DBU DBN TBD MTBD DMAP

The aminolysis is preferably carried out with DBU catalyst or 2,3,4,6,7,8,9,10-octahydropyrimido[1,2-a]azepine (DBU),

In the second step, the resulting polyallylcarbamate hydroxy derivative of formula (10) is then UV polymerised in presence of a thiol of formula (16) preferably of formula (11) as crosslinker and a photoinitiator.

$R_8\text{-}(SH)_k$ (16)

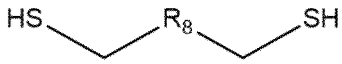

(11)

wherein k is an integer >,2 preferably between 2 and 6;

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof;

$R_8$ is preferably an alkylene.

The term thiol as used herein refers to bi-thiols, tri-thiols, tetra-thiols, or hexa-thiol preferably pentaerythrityl tetrathiol, thiol trimethyol propane, pentaerythritol tetra (3-mercaptopropionate), trimethylolpropane tri (3-mercaptopropionate), pentaerythritol tetrathioglycolate and/or trimethylolpropane thioglycolate, tris [2-(3-mercaptopropionyloxy)ethyl] isocyanurate. It also refers to a telechelic, branched or multiarms polymer bearing thiol groups at each chain-end (such as polyethylene glycol dithiol or polypropylene glycol dithiol) or along the polymer backbone as pendant groups, or a peptide or protein containing at least two thiols.

Preferred di-thiol and trithiol are for example 2-2'(ethylenedioxy)diethanethiol of formula (12) and 2,2-bis(3-sulfanylpropanoyloxymetyl)butyl -sufanylpropanoate of formula (13).

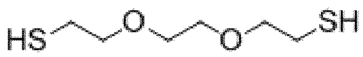

(DiTh)

(12)

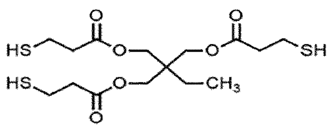

(13)

The term "photoinitiator" as used herein refers for example to bis(2,4,6-trimethylbenzoyl)-phenylphosphineoxide also called Irgacure 819; bis-(4-methoxybenzoyl)diethylgermanium also called Ivocerin; 2,3-bornanedione also called camphorquinone; Lithium phenyl-2,4,6-trimethylbenzoylphosphinate also called LAP, Riboflavin-5-phosphate. Preferably the photoiniator is Irgacure 819.

The photoinitiator may be used at a low concentration between 0 and 0.1%, preferably below 0.1%.

According to some embodiments, UV polymerization occurs at a wavelength between 300 and 400nm preferably at 365 nm. Optionally UV polymerization may be further combined with heating to increase the crosslinking and evaporate trace of solvent. Such additional heating may be carried out between 40 and 100°C, preferably 70°C.

The resulting polyhydroxyurethane of formula (9) comprises hydroxyl groups and other groups that can advantageously be used to load and release bioactive molecules for local medical treatment.

*In one particular embodiment,* the first step is an aminolysis of polypropyleneoxidebicyclocarbonate of formula (5), also called PPGbisCC

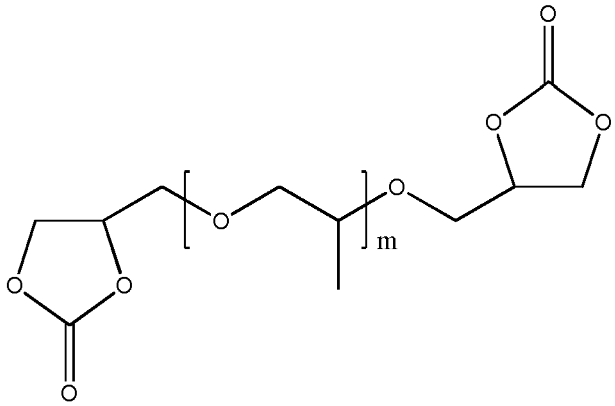

(5)

wherein m is an integer equal or superior to 1, preferably m is between 1 and 1000, more preferably between 1 and 20.

In some embodiments the bicyclocarbonate of formula (5) is first reacted with an allylamine to obtain a polyallylcarbamatehydroxypropyleneoxide of formula (14) also called PPG-allyl

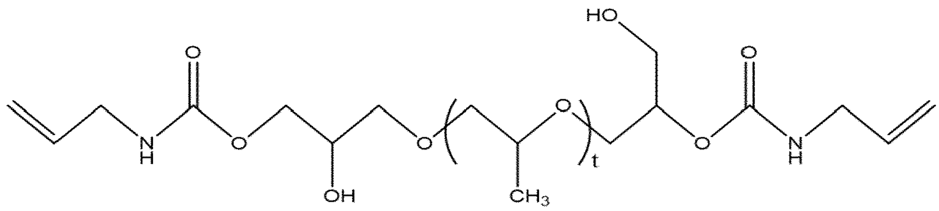

(14)

wherein t is an integer superior or equal to 1, preferably t is between 1 and 1000, more preferably between 1 and 20

In the second step, the polyallylcarbamatehydroxypropyleneoxide of formula (14) is then crosslinked with a dithiol of formula (13) in presence of a photoinitiator such bis(2,4,6-trimethylbenzoyl)-phenylphosphineoxide under UV radiation preferably from 300 to 400nm, most preferably 365nm.

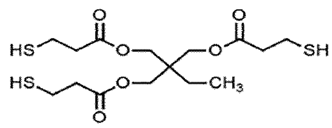

(13)

***In a particular aspect,*** the medical device is made or partly made of one or more polyhydroxyurethane (PHU), particularly of PHU based on formula (1) or of PHU based on formula (9). The medical device made or partly made of polyhydroxyurethane (PHU) may optionally be reinforced with high surface area particles or fibers to improve mechanical properties, such as for example tensile strength, elongation at break, Elastic and shear modulus.

The Polyhydroxyurethane (PHU) structure based on formula (1) may optionally be reinforced by addition of high surface area particles or fibers such as for example silica, functionalized silica, polypropylene terephthalate mesh or polyethylene mesh or a mixture thereof. The addition of high surface area particles or fibers is made in the mixture of polycarbonate of formula (2) with polyamine of formula (3) before thermal polymerization.

The polyhydroxyurethane (PHU) structure based on formula (9) may optionally be reinforced by addition of high surface area particles or fibers such as for example silica, functionalized silica, polypropylene terephthalate mesh or polyethylene mesh or a mixture thereof. The addition of high surface area particles or fibers is made in the mixture of polyallylcarbamate hydroxy derivative of formula (10) with thiol of formula (11) before UV polymerization.

The medical device made or partly made of one or more polyhydroxyurethane (PHU) optionally with high surface area particles may be carried out by casting, injection molding, extrusion, coextrusion, spraying, 3D printing, coating or additive manufacturing to produce the medical device according to the invention.

The medical device partly or totally made of one or more PHU is advantageously more hemocompatible inducing less coagulation and platelet adhesion and less prone to bacterial infection than corresponding medical device comprising

polyurethane (PU)

The medical device partly or totally made of one or more PHU may advantageously load and release bioactive molecules, thereby providing a local medical treatment.

***In another particular aspect of the invention,*** the medical device comprises one or more polyhydroxyurethane (PHU); particulary PHU based on formula (1) or PHU based on formula (9) coated at least partly or totally on the surface of the medical device, for example on the surface of a catheter or of a prosthetic heart valve.

The polyhydroxyurethane (PHU) used to coat at least partly or totally the surface of a medical device may be anchored or attached onto the surface of the medical device, using various physical or chemical methods, such as surface irradiation, layer-by-layer (LbL) assembly, spin coating, plasma deposition and the like.

The medical device partly or totally coated with one or more PHU is advantageously more hemocompatible inducing less coagulation and platelet adhesion and less prone to bacterial infection than corresponding medical device coated with polyurethane (PU).

The at least partly or totally PHU coated medical device may advantageously be loaded and release, or be grafted with bioactive molecules, thereby providing a local medical treatment.

*In a particular embodiment,* the medical device is a catheter comprising a shaft made of one or more polyhydroxyurethane (PHU) or is a catheter coated with one or more polyhydroxyurethane (PHU). The shaft may be a single lumen shaft, shafts with multiple lumens, such as for example two, three, four or even more. The shaft is prepared by extrusion of one or more polyhydroxyurethane (PHU) according to methods well known in the art, during a monolayer or multi-layer co-extrusion process.

*In another particular embodiment,* the medical device is a prosthetic valve made or partly made of one or more polyhydroxyurethane (PHU). PHU may be synthetised directly in molds designed to mimic native valve having symmetric tri-leaflet design as reproduced in figure 2.

In order to meet the properties of a native valve, polyhydroxyurethane (PHU) should have mechanical properties strong enough such as a Young’s modulus around 2-3MPa, a tensile strength superior to 1.5MPa and an elastic deformation superior to 35%.

Such properties are met with polyhydroxyurethane (PHU), but may be improved after incorporation of a reinforced particle such as silica by creation of crosslinking nodes to which polyhydroxyurethane are attached by their OH groups or after incorporation of reinforced polyester mesh to improve tensile strength. Examples of reinforced mesh are polyethylene or polypropylene terephthalate mesh.

Prosthetic heart valve may be made by 3D printing with a computer program that was optimized for valve designs to meet computational fluid dynamics.

Indeed, it has been learned through experience that not only is the choice of prosthetic material important, but also the design must result in flow that is as laminar as possible to diminish turbulence and also incorporate sufficient diastolic washing of valve components to minimize microthrombi.

The new medical device made or coated with one or more PHU can advantageously comprise bioactive molecules that are further released progressively over time, thereby providing medical treatment such as for example improved anti-infectious or anti-thrombotic effect. In particular embodiments the PHU is impregnated with one or more bioactive molecules. Accordingly, in particular embodiments, the medical device comprises one or more PHU impregnated with bioactive molecules. In other particular embodiments, the medical device comprises one or more PHU grafted with bioactive molecules. Moreover the new medical device comprising one or more PHU impregnated with bioactive molecules advantageously provide localised delivery to provide rapid therapeutic activity while minimizing offsite toxicity and lowering susceptibility to resistance.

Indeed polyhydroxyurethane (PHU) according to the invention can load bioactive molecules by immersion in a solvent containing the bioactive molecules.

The polyhydroxyurethane (PHU) according to the invention swells when in contact of the solvent allowing the loading of the bioactive molecules. The solvent of immersion may be alcohol, water, THF, $CH_2Cl_2$ or a mixture thereof or any other solvent mixture which can solubilize the bioactive molecules and swell the PHU. The loading of the bioactive molecules occurs through H-binding with OH groups of PHU.

After elimination of the solvent, the polyhydroxyurethane (PHU) of the present invention can release the bioactive molecules over time upon implementation in the human body.

Alternatively the polyhydroxyurethane (PHU) of the present invention can be grafted with bioactive molecules by methods known in the art.

The term "bioactive molecule" as used herein refers for example to antibacterial agent, anti-biofilm formation agent, anti-platelet agents, anti-coagulants, anti-thrombotic agents, and anti-calcification agents. The bioactive molecule may be incorporated within the bulk of the medical device made of one or more polyhydroxyurethane (PHU) or at the surface of the medical device coated with one or more polyhydroxyurethane.

Bioactive molecule may include any agent which is desired to be delivered to cells, tissues or organs for modulating or otherwise modifying cell function, including for therapeutic effects. Bioactive molecules include, but are

not limited to, pharmaceutically active compounds or diagnostic compounds. Bioactive molecules include, but are not limited to, nucleotides (aptamers, RNAi, antisense oligonucleotides), peptides, oligopeptides, proteins, apoproteins, glycoproteins, antigens and antibodies or antibody fragments thereto, receptors and other membrane proteins, retro-inverso oligopeptides, protein analogs in which at least one non-peptide linkage replaces a peptide linkage, enzymes, coenzymes, enzyme inhibitors, amino acids and their derivatives, hormones, lipids, phospholipids, liposomes, ricin or ricin fragments; toxins such as aflatoxin, digoxin, xanthotoxin, rubratoxin; analgesics such as aspirin, ibuprofen and acetaminophen; bronchodilators such as theophylline and albuterol; beta-blockers such as propranolol, metoprolol, atenolol, labetolol, timolol, penbutolol and pindolol; antimicrobial agents such as those described above and ciprofloxacin, cinoxacin and norfloxacin; antihypertensive agents such as clonidine, methyldopa, prazosin, verapamil, nifedipine, aptopril and enalapril; cardiovascular agents including antiarrhythmics, cardiac glycosides, antianginals and vasodilators; central nervous system agents including stimulants, psychotropics, antimanics and depressants; antiviral agents; antihistamines such as chlorphenirmine and brompheniramine; cancer drugs including chemotherapeutic agents, such as chlorambucil, carboplatin, derivatives of busulfan, doxorubicin, etoposide, topotecan (TPT); tranquilizers such as diazepam, chordiazepoxide, oxazepam, alprazolam and triazolam, anti-depressants such as fluoxetine, amitriptyline, nortriptyline and imipramine; H-2 antagonists such as nizatidine, cimetidine, famotidine and ranitidine; anticonvulsants; antinauseants; prostaglandins; muscle relaxants; anti-inflammatory substances; stimulants; decongestants; antiemetics; diuretics; antispasmodics; antiasthmatics; anti-Parkinson agents; expectorants; cough suppressants; mucolytics; vitamins; and mineral and nutritional additives. Other molecules include nucleotides; oligonucleotides; polynucleotides; and their art-recognized and biologically functional analogs and derivatives including, for example, methylated polynucleotides and nucleotide analogs having phosphorothioate linkages; plasmids, cosmids, artificial chromosomes, other nucleic acid vectors; antisense polynucleotides including those substantially complementary to at least one endogenous nucleic acid or those having sequences with a sense opposed to at least portions of selected viral or retroviral genomes; promoters; enhancers; inhibitors; other ligands for regulating gene transcription and translation.

The bioactive molecule may be an anti-infective agent. Anti-infective agents include, but are not limited to antibiotics, such as amikacin, gentamicin, kanamycin, neomycin, netilmicin, tobramycin, paromomycin, streptomycin, spectinomycin, geldanamycin, herbimycin, rifaximin, loracarbef, ertapenem, dorpenem, imipenem/cilastatin, meropenem, cefadroxil, cefazolin, cefalotin, cephalexin, cefaclor, cefamandole, cefoxitin, cefproxil, cefuroxime, cefixime, cefdinir, cedfitoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, daibavancin, oritavancin, clindamycin, lincomycin, daptomycin, azithromycin, clarithromycin, dirithromycin, erythromycin, roxithromycin, troleandomycin, telithromycin, spiramycin, aztreonam, furazolidone, nitrofurantoin, linezolid, amoxicillin, ampicillin, piperacillin, ticarcillin, bacitracin, colistin, polymyxin B, ciprofloxacin, enoxacin, gatifloxacin, gemifloxacin, levoflaxicin, lomefloxacilin, moxifloxacin, nalidixic acid, norfloxacin, ofloxacin, mafenide, sulfacetamide, sulfadizine, silver sulfadizine, sulfadimethoxine, sulfamethizole, sulfamethoxazole, sulfanilimide, sulfisoxazole, trimethoprim-sulfamethoxazole, sulfonamidochrysoidine, demeclocyline, doxycycline, minocycline, oxytetracycline, tetracycline, clofazimine, dapsone, rifampicin, rifabutin, arspehnamine, chloramphenicol, fosfomycin, metronidazole, thiamphenicol, tigecycline, tinidazole, trimethoprim; but also to pyrimidine derivatives as defined in WO2019/158655 or Triazolo(4,5-d)pyrimidine derivatives as defined in EP3292867B1 that possess antibacterial activity and are also called antibacterial agents.

Anti-biofilm formation agents include, but are not limited to naturally occurring peptides such as human cathelicidin LL-37 or the bovine peptide indolicidin, or synthetic peptides such as 1018, natural compounds with 2-aminoimidazole moiety, 2-aminoimidazole based inhibitors, benzimidazoles analogs, indole-triazo-amide analogs, plant-derived biofilm inhibitors such as emodin, phloretin, casbane diterpene, resveratrol and its oligomers, sulphur derivatives, brominated furanone analogs, bromopyrrole alkaloids, skyllamycins and (-)-ageloxime D structures, cembranoids, N-acyl homoserine lactone analogs, carolacton, molecules that interfere with the formation of amyloid-like fibres, fatty acids, nitric oxide donors, ionic liquids as 1-alkyl-3-methyl imidazolium chloride, 1-alkylquinolinium bromide, all these agents can be used in combination with conventional antibiotics or with pyrimidine derivatives as defined in WO2019/158655 or Triazolo(4,5-d)pyrimidine derivatives as defined in EP3292867B1 that possess antibacterial activity and are also called antibacterial agents.

Anti-platelet agents include, but are not limited to, irreversible cyclooxygenase inhibitors such as aspirin and trifusal (Disgren), Adenosine diphosphate (ADP) receptor inhibitors such as Clopidogrel (Plavix), prasugrel (Effient), ticagrelor (Brilinta), ticlopidine (Ticlid), Phosphodiesterase inhibitors such as cilostazol (Pletal), Protease-activated receptor-1 (PAR-1) antagonists such as vorapaxar (Zontivity), glycoprotein IIB/IIIA inhibitors (intravenous use only) such as abciximab (ReoPro), eptifibatide (Integrilin), tirofiban (Aggrastat), Adenosine reuptake inhibitors such as dipyridamole (Persantine), thromboxane inhibitors, thromboxane synthase inhibitors and thromboxane receptor antagonists such as terutroban, glycoprotein VI inhibitors such as Revacept, glycoprotein Ib inhibitors, and von Willebrand factor inhibitors.

Anti-coagulants include, but are not limited, to acenocoumarol, coumatetralyl, dicoumarol, ethyl biscoumacetate, phenprocoumon, warfarin, clorindione, dipjenadione, phenindione, ticlomarol, bemiparin, certoparin, ardeparin, dalteparin, enoxaparin, nadroparin, parnaparin, reviparin, dabigatran, apixaban, betrixabaan, darexaban, edoxaban,

otamixaban, rivaroxaban, alteplase, danaparoid, tinzaparin, and fondaparinux.

Thrombolytic agents include, but are not limited to, alteplase, reteplase, tenecteplase, saruplase, urokinase, anistreplase, monteplase, streptokinase, ancrod, brinase and fibrinolysin.

Anti-thrombotic agents comprises anti-coagulants and anti-platelet agents.

Anti-calcification agents include, but are not limited to, bisphosphonates, aluminium salts, glutaraldehyde, amino oleic acid, and metalloproteinase inhibitors.

It is another object of the invention to provide a method of manufacturing the medical device comprising one or more polyhydroxyurethane (PHU); the method comprising a step (a) or (b) corresponding respectively to a thermal or a UV polymerisation or a combination thereof:

a) mixing a polycyclocarbonate of formula (2) with a polyamine of formula (3) and optionally a catalyst to obtain, by thermal polymerization, a polyhydroxyurethane based on formula (1);

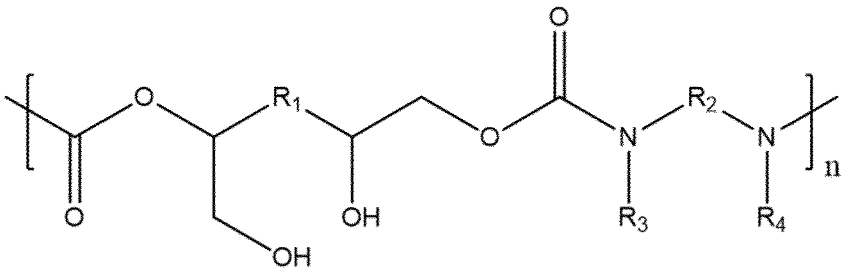

(1)

wherein n, $R_1$, $R_2$ are as defined above ; or

b) mixing a polycarbamate hydroxyl derivative, bearing two or more photoresponsive group at the extremity or pendant to the main hydrocarbon chain with a dithiol as crosslinker and a photoiniator to obtain, by UV polymerization, a polyhydroxyurethane based on formula (9);

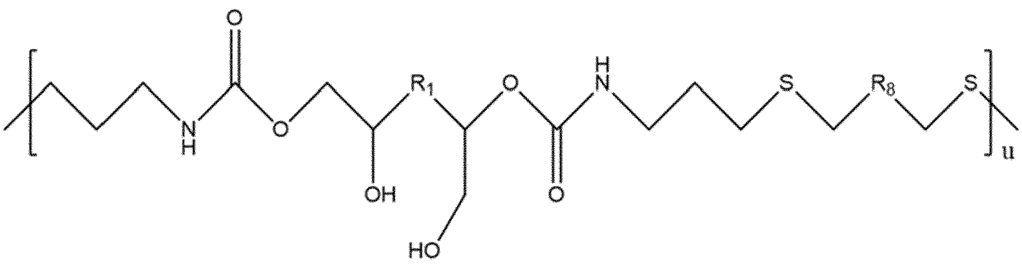

(9)

wherein u and $R_8$ are as defined above.

The method of manufacturing the medical device comprising one or more polyhydroxyurethane (PHU) may optionally further comprise an addition of reinforced particle or fiber in the mixture step (a) or (b).

The obtained mixture of step (a) or (b) may be then further processed either by moulding, extrusion, coextrusion or 3D printing to obtain respectively by thermal or UV polymerisation a medical device comprising a polyhydroxyurethane based respectively on formula (1) or (9) .

The term "photoresponsive group" as used herein refers to one or more vinyl or allyl groups.

The invention will now be illustrated with reference to the following Figures which are not intended to limit the scope of the claimed invention:

Figure 1: diagram of synthesis of polypropylene glycol based polyurethane

Figure 2: tri-leaflet valve made by molding polyhydroxyurethane according to example 22.

Figure 3a: diagram of synthesis for Polyethylene glycol) bicyclocarbonate (PEGbisCC) of example 1.

Figure 3b: diagram of thermal polymerisation of PPGbisCC728 or PHU1 according to example 2

Figure 4: diagram of UV synthesis of polyhydroxyurethane according to example 5.

Figure 5: PHU characterisations by $^1$H NMR spectra and FTIR-ATR spectra

Figure 5a: 1H NMR spectra of PPGDE and PPG bisCC.

Figure 5b: FTIR-ATR spectra of PPGDE and bisCC PPG.

Figure 5c: *FTIR-ATR spectra of bisCC PPG and PHU.*

Figure 5d: 1H NMR spectra of PTHFDE and PTHF bisCC.

Figure 5e: FTIR-ATR spectra of PTHF and PTHFDE

Figure 5f: FTIR-ATR spectra of PTHFDE and PTHF bisCC

Figure 5g: FTIR-ATR spectra of PTHF bisCC and PHU

Figure 5h: 1H NMR spectra of PPG bisCC and PPG-allyl

Figure 5i: FTIR-ATR spectra of PPG-allyl and PHU A6b.

Figure 6: comparison minocycline release over time for PTHF PHU, PPG PHU and PTHF- PU and PPG-PU

Figure 7: comparison % hemolysis for PU, PPG-PHU: **PPG-PHU and PTFH-PHU do not cause hemolysis.** A medical-grade PU was used as a reference control. Hemolysis rates below 2% are considered non-hemolytic (dashed line).

Figure 8: comparison PU, PPG-PHU in complement activation test: **PPG-PHU does not activate the complement cascade in human whole blood.** A medical-grade PU was used as a reference control, and zymosan as a positive control.

Figure 9: Comparison PU, PPG-PHU and PTHF-PHU in contact phase activation of coagulation test: **PPG-PHU and PTHF-PHU induce less contact phase activation of coagulation than medical grade PU.** Kaolin was used as a positive control.

Figure 10: Comparison PU, PPG-PHU in human platelet adherence (UV absorbance test): **Human platelets do not adhere onto PPG-PHU.** A medical-grade PU was used as a reference control, and fibrinogen-coated polystyrene as a positive control.

Figure 11: comparison PU, PPG-PHU in human plasma proteins adsorption : **Less plasma proteins adsorption onto PPG-PHU than onto medical grade PU.**

Figure 12: dynamic rheology: storage and loss moduli of UV cured PHU1(bis CC PPG468) and PHU2(bisCC PPG728).

**Example 1: Synthesis of Poly(ethyleneglycol)bicyclcarbonate (PEGbisCC) 588g/mole**

Poly(ethylene glycol) biscyclocarbonate was synthetised by coupling $CO_2$ with PEG diglycidyl ether 500g/mol (0.06 mol, 30g, 1eq.) from Sigma Aldrich, using a bicomponent organocatalyst that combined tetra butyl ammoniumiodide (TBAI 0.03 mol, 0.05eq) from Sigma Aldrich as the catalyst in 80 ml highpressure cell equipped with a mechanical stirrer (Autoclave Top Industrie). The coupling reaction illustrated in Figure 3 was performed at 80°C and 100 bar using 5 mole % of catalyst ([TBAI]). These conditions ensured a complete conversion of the terminal epoxy rings into the corresponding cyclic carbonates in 24 hours.

After depressurization of the reactor, Poly(ethylene glycol) biscyclocarbonate was collected and TBAI was removed by liquid-liquid extractions. Polyethylene glycol) biscyclocarbonate was first solubilized in 400ml of chloroform, washed with 4 × 400ml of Milli-Qwater and finally 400 ml of Milli-Qwater with ammonium sulfate (10 g/l). The organic fraction iwas collected and rotavaped.

The same procedure may be used to prepare a polypropylene glycol)bis cyclo-carbonate using PPG diglycidyl ether 380g/mol (0.079 moles) from Sigma Aldrich and 0.004 moles of tetrabutylammonium iodide from Sigma Aldrich.

**Example 2: thermal synthesis of polyhydroxyurethane ($PHU_{728}$ and $PHU_{468}$) from polypropylene glycol biscyclo carbonate PPG bisCC (728** and 468). **ANPIA1bL6 and ANPIA1bL7**

**ANPIA1bL6:** PPG bisCC 728 g/mol (1.92 mmol, 1.40 g, 1 eq., prepared from PPGDE 640 g/mol (see example 1)), 4-((4-aminocyclohexyl)methyl)cyclohexanamine (MBCHA, 0.38 mmol, 0.08 g, 0.2 eq.) from Sigma Aldrich and Tris(2-aminoethyl)amine (TAEA, 1.02 mmol, 0.15 g, 0.53 eq.) from Sigma Aldrich were introduced in a vial and magnetically stirred at 40 °C for 10 minutes. The resulting mixture was poured into a flat Teflon mold (5 x 2.5 x 0.1 cm) and placed at 70 °C in the oven for 24 hours.

**ANPIA1bL7:**The same procedure was used with PPG bisCC 468 g/mol (prepared from PPGDE 380 g/mol) by adapting the quantities of the other reagents to keep the same molar ratios (PPGDE/MBCHA/TAEA: 1/0,2/0,53).

**Example 3: thermal synthesis of Polypropylene glycol based-polyurethane PU(L25C).**

4,4'-methylene bis(cyclohexyl isocyanate) (1.08 g, 4.12mmol) from Sigma Aldrich, triethanolamine (0.16 g, 1.07mmol) and polypropylene glycol ( 1g, 2.5mmol) from Sigma Aldrich were charged into a round bottom flask equipped with a dry N2 purge and stirred to form a homogeneous solution. After 10 min, dibutyltin dilaurate (5 mg) was added, and the round bottom flask was placed in an oil bath at 30 °C under vacuum. The reaction proceeded for 5min, and the resultant melt was poured onto greased Kapton paper and pressed between two aluminum plates equipped with 1 mm spacers at 80 °C and 5 psi for 3 hr. After removing from the mold, the sample was post-cured at 100 °C for 24 h.

**Example 4: thermal synthesis of Polypropylene glycol based-polyurethane PU(L25D) also called PPG based PU.**

Following the same procedure as for the synthesis of L25C of example 3, but using a different ratio (1/0.8) between 4,4'-methylene bis(cyclohexyl isocyanate)/PPG , PPG based-PU (L25D) was prepared by mixing 4,4'-methylene bis(cyclohexyl isocyanate) (0.82 g, 3.13mmol) from Sigma Aldrich, triethanolamine (0.062 g, 0.41mmol) from Sigma Aldrich and polypropylene glycol (1g, 2.5mmol) from Sigma Aldrich.

**Example 5: UV synthesis of PHU.**

The second route to obtain a PHU network is the UV crosslinking. In this example, UV-PHU was synthetized in two steps according to the chemical reaction scheme illustrated in figure 4.

The first step is a grafting of allyl functions at the end of PPG bisCC chains. Allylamine was thus added in excess in order to obtain quantitative functionalization, and 2,3,4,7,8,9-octahydropyrimido(1,2-a)azepine (DBU) was used to catalyse the reaction. The first step is also described in more detail at example 16. The conversion of PPG bisCC into PPG-allyl was checked by 1H NMR spectroscopy *(Figure 5h*). The disappearance of peaks assigned to the cyclocarbonates of PPG bisCC 468 at 4.8 and 4.5 ppm is visible, so as the appearance of peaks corresponding to PPG-allyl (5.9 - 5.8 and 5.2 - 5.1 ppm).

In the second step, the PPG-allyl obtained in the first step reacts with the thiol groups of 2,2-bis(3-sulfanylpropanoyloxymethyl) butyl 3-sulfanylpropanoate as crosslinking agent and Irgacure 819 (BASF) as photoinitiator under UV irradiation (365 nm). This step led therefore to the PHU network. The second step is also described in more detail at example 17.

Each PHU solution was prepared and poured into a flat Teflon mold and put under the UV lamp 15 minutes before staying overnight at 70°C in the oven. After removal of the mold the product was characterised by FTIP-ATR spectroscopy with -OH and - NH groups (3200-3500 cm-1), and CO (1711 $cm^{-1}$), NH (1535 $cm^{-1}$), N-CO-O and C-O-C (1256 $cm^{-1}$) of urethane group as shown in figure 5i.

**Example 6: mechanical properties - comparison Polypropylene glycol based-PU and Polypropylene glycol based-PHU**

Mechanical properties were measured at room temperature under dried conditions according to ASTMD638. The tensile strength and elongation of HPU films were measured and compared to PU films on a testing machine (Instron 5566, USA) at a speed of 10mm/min, at a pressure of 2,7 bar and with a static load cell of 10N. The test involves putting a sample, 1 cm of width and 3 cm of length, between two clamps stretched until the film ruptures.

The results are illustrated in table 1. Other mechanical tests such as swelling and contact angle were also carried out.

The swelling ratio of dry networks was measured after immersion for 24 hours in a phosphate-buffered saline water (PBS, pH = 7.2). This solution was chosen for its pH (= 7.2) close to the physiological pH.

The swelling ratio was determined using the following equation: swelling = $(W_{s,w}- W_i)/W_i \times 100$ %

where $W_{s,w}$ is the weight of the swollen sample in PBS and $W_i$ the weight of the dried sample.

The Contact angles were measured using a contact angle meter DGD Fast/60 and the software WINDROP by GBX Instruments in surface energy mode. For each measurement, a droplet of 15 μl of Milli-Qwater was deposited on the surface of the material. Contact angles were then determined after 180 seconds. The measurements were repeated three times on three different spots.

The results are also reported in table 1.

**Table 1**

| Polymer code | Expansion (thickness/ width/leng th)% | swelling (%) after 24h | Contact | Elastic modulus E (MPa) | Tensile strength $\sigma_R$ (MPa) | Strain $\varepsilon_R$(%) |
|---|---|---|---|---|---|---|
| | | | Angle after 3min | | | |
| | | | | | | |
| ANPIA1bL6 b | 12,25/8,10/8,11 | 35.41 | 56 | 0,80 | 0,48 | 129,1 1 |
| ANPIA1bL7 | 9,6/8,5/8,1 | 26.21 | 55 | 15,62 | 1,08 | 233,9 6 |
| L25C | - | -- | 100 | 80,31 | 26,93 | 3,58 |
| L25D | - | 1 | 96 | 45,67 | 38,54 | 6,38 |
| | | | | | | |

**Example 7: synthesis of Polyhydroxyurethane as a crosslinked network**

*First step: Synthesis of bis(cyclocarbonate) polypropylene glycol) (PPG bisCC) 728g/mol:*

Polypropylene glycol) diglycidyl ether (PPGDE) 640 g/mol (0.047 mol, 30 g, 1 eq.) from Sigma Aldrich and tetrabutylammonium iodide (TBAI, 0.047 mol, 1.73g, 0.05 eq.) from Sigma Aldrich were introduced in an 80 ml high pressure autoclave (Top Industrie). The stainless-steel reactor was then filled with 100 bars of $CO_2$ from Air Liquide and heated up to 80 °C for 24 hours. After depressurization of the reactor, the resulting product is collected and TBAI was removed by liquid-liquid extractions. PPG bisCC is first solubilized in 400 ml of chloroform, washed with 4 x 400 ml of Milli-Q water and finally 400 ml of Milli-Q water with ammonium sulfate (10 g/l) from Sigma Aldrich. The organic fraction was collected and the solvent removed by a rotary evaporator. The same procedure was used with PPGDE 380 g/mol (0.079 mol) from Sigma Aldrich and 0.079 moles of tetrabutylammonium iodide from Sigma Aldrich.

Figure 5a shows the $^1H$ NMR spectra of PPGDE 640 g/mol and PPG bisCC after purification of the catalyst. The conversion of PPGDE to PPG bisCC is clearly shown as the peaks corresponding to epoxy groups in the range 2.6-3.2 ppm have completely disappeared in the PGG bisCC spectrum, while the peaks assigned to the cyclo-carbonates of PPG bisCC appear at 4.5 and 4.8 ppm. As additional evidence of conversion, the FTR-ATR spectra of PPGDE and PPG bisCC are also shown in Figure 5b. The total disappearance of the peaks assigned to the epoxy groups of PPGDE at 1250 $cm^{-1}$ and 907 $cm^{-1}$ is visible. The peaks assigned to C=O of cyclocarbonates are also visible at 1797 $cm^{-1}$ and 1162 $cm^{-1}$.

*Second step: Synthesis of a crosslinked poly(hydroxyurethane) networks:*

Bis(cyclocarbonate) polypropylene glycol) (PPG bisCC) 728 g/mol (1.92 mmol, 1.40 g, 1 eq., prepared from PPGDE 640 g/mol, 4-((4-aminocyclohexyl)methyl)cyclohexanamine (MBCHA, 0.38 mmol, 0.08 g, 0.2 eq.) and N',N'-bis(2-aminoethyl)ethane-1,2-diamine (TAEA, 1.02 mmol, 0.15 g, 0.53 eq.) were introduced in a vial and magnetically stirred at 40 °C for 10 minutes. This solution was then poured into a flat Teflon mold (5 x 2.5 x 0.1 cm) and placed at 70 °C in the oven for 24 hours before being characterized by FTIR-ATR spectra.

The conversion of cyclocarbonates to PHUs was checked and confirmed by IR spectroscopy . Figure 5c shows a FTIR-ATR spectra of bisCC PPG and PHU wherein the peaks corresponding to cyclo-carbonate functional groups of PTHF bisCC at 1797 $cm^{-1}$ and 1166 $cm^{-1}$ have completely disappeared, and new peaks corresponding to -OH and -NH groups (3340 $cm^{-1}$), and CO (1698 $cm^{-1}$), NH (1552 $cm^{-1}$), N-CO-O and C-O-C (1342 $cm^{-1}$) of urethane groups are visible.

The peak assigned to residual unreacted cyclocarbonates is also visible at 1797 $cm^{-1}$.

The same procedure was used with PPG bisCC 468 g/mol (prepared from PPGDE 380 g/mol) by adapting the quantities of the other reagents to keep the same molar ratios (2.99 mmol, 1.40 g and 1 eq. of BisCC PPG, 0.60 mmol, 0.13 g and 0.2 eq. of MBCHA and 1.58 mmol, 0.23 g and 0.53 eq. of TAEA). The amines used were sometimes varied but used in the same molar ratios.

**Example 8: thermal synthesis of Polytetrahydrofuran based-PHU**

*First step: Synthesis of di-epoxy polytetrahydrofuran (DEPTHF) molecular weight* 760 g/mol.

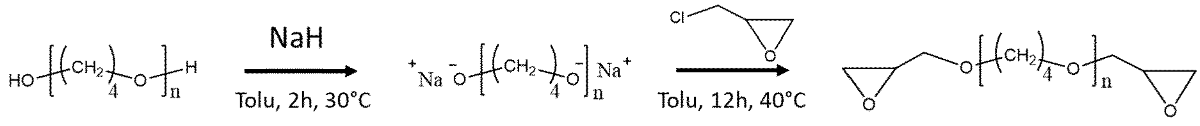


Polytetrahydrofuran (PTHF) (20 g, 650g/mol, 30 mmol) from Sigma-Aldrich was dissolved in 250 mL of anhydrous toluene from Sigma-Aldrich and dried by 3X azeotropic distillation of the solvent toluene. The O-hydroxy end group of PTHF was then converted into sodium alkoxide by reaction with sodium hydride (4 g) at 30 °C for 2h. 8 eq of epichlorohydrin (ECH) (19 mL, 92,5 g/mol, ) were added to the solution and reacted at 40 °C for 12h. DEPTHF was then recovered by evaporation under vacuum of the excess of ECH and redissolved in dichloromethane (200 mL) from Aldrich. The CH2Cl2 solution was extracted twice with water, followed by drying over anhydrous magnesium sulfate, filtration, and elimination of dichloromethane.

Figure 5d shows a 1H NMR spectra of PTHF and PTHF bisCC wherein the conversion of PTHF to DEPTHF was proved by the apparition of the peaks corresponding to epoxy groups in the range 2.6-3.2 ppm. Almost quantitative yield (91-97%) was observed after 12h of reaction at 40°C.

Figure 5e shows a FTIR-ATR spectra of PTHF and PTHFDE wherein functionalization of PTHF was confirmed by apparition of a new peaks at 1250 $cm^{-1}$ and 910 $cm^{-1}$ corresponding to the epoxy groups and by the disappearance of the peaks at 3400 $cm^{-1}$assigned to the -OH groups of PTHF .

*Step 2: Synthesis of polytetrahydrofuran bis(cyclo-carbonate) (PTHF bisCC) 850g/mol:*

DEPTHF 760 g/mol (25 g, 1 eq.) and tetrabutylammonium iodide (TBAI, 0.6g, 0.05 eq.) were introduced in an 80 ml high pressure autoclave. The stainless-steel reactor was then filled with 100 bar of $CO_2$ from Air Liquide and heated up to 80 °C for 24 hours. After depressurization of the reactor, the resulting product was collected and TBAI was removed from it by liquid-liquid extractions. PTHF bisCC was thus first solubilized in 400 ml of chloroform, washed with 4 x 400 ml of Milli-Q water and finally 400 ml of Milli-Q water with ammonium sulfate (10 g/l)from Sigma Aldrich. The organic fraction was collected and rotavaped.

Figure 5d shows the NMR analysis of PTHF and PTHF bisCC, wherein the total conversion of PTHFDE to PTHF bisCC is clearly confirmed, as the peaks corresponding to epoxy groups in the range 2.6-3.2 ppm have completely disappeared, while the peaks assigned to the cyclocarbonates of PTHF bisCC appear at 4.5 and 4.8 ppm.

Figure 5f shows the FTR-ATR spectra of PTHFDE and PTHF bisCC wherein the apparition of the peaks assigned to the cyclocarbonate groups of PTHF bisCC are recorded at 1797 $cm^{-1}$ and 1166 $cm^{-1}$.

*3rd step: Synthesis* of linear *poly(hydroxyurethane):*

PTHF bisCC 850g/mol (1eq.) and 1,12-diaminododecane (eg.) from Sigma Aldrich were introduced in a vial and magnetically stirred at 120°C for 10 minutes. The resulting solution was then poured into a flat Teflon mold (5 x2.5 x0.1 cm) and placed at 40°C in an oven for 24 hours before being characterized by differential scanning calorimetry (DSC).

Glass-transition (Tg) and melting (Tm) temperatures were determined using differential scanning calorimetry with TA Instruments DSC Q100 thermal analyser calibrated with indium, and the curves were analysed using the TA Instruments Universal Analysis 2000 software. The procedure after insertion of the sample was the following: equilibration at -80 °C and isothermal step for 2 min, heating with a ramp of 10 °C/min up to 100 °C (1st heating cycle) followed by an isothermal step of 2 min, cooling with a ramp of 10 °C/min down to -80 °C (1st cooling cycle) followed by an isothermal step of 2 min, heating with a ramp of 10 °C/min up to 150 °C (2nd heating cycle) followed by an isothermal step of 2 min, cooling with a ramp of 10 °C/min down to -80 °C (2nd cooling cycle) followed by an isothermal step of 2 min, heating with a ramp of 10 °C/min up to 150 °C (3rd heating cycle) followed by an isothermal step of 2 min.

The DSC analysis shows a Tg around -61 °C and a Tm that ranges from ~8 °C to ~14 °C, characteristic of a semi-crystalline polymer.

*3rd step: Synthesis of crosslinked poly(hydroxyurethane) networks:*

PTHF bisCC 850 g/mol (1 g, 1 eq.), 4-((4-aminocyclohexyl) methyl) cyclohexanamine (MBCHA, 0.05 g, 0.2 eq.) from Sigma Aldrich and N',N'-bis(2-aminoethyl)ethane-1,2-diamine (TAEA, 0.09 g, 0.53 eq.) from Sigma Aldrich were introduced in a vial and magnetically stirred at 40 °C for 10 minutes. This solution was then poured into a flat Teflon mold (5 x 2.5 x 0.1 cm) and placed at 70 °C in the oven for 24 hours before being characterized by IR spectroscopy.

The conversion of cyclocarbonates to PHUs was checked and confirmed by IR spectroscopy.

Figure 5f shows a FTIR-ATR spectra of PTHF bisCC and PHU, wherein the peaks corresponding to cyclocarbonate functional groups of PTHF bisCC at 1797 $cm^{-1}$ and 1166 $cm^{-1}$ were completely disappeared, and new peaks corresponding to -OH and -NH groups (3340 $cm^{-1}$), and CO (1698 $cm^{-1}$), NH (1552 $cm^{-1}$), N-CO-O and C-O-C (1342 $cm^{-1}$) of urethane groups are visible.

**Example 9: loading and releasing of Minocycline over time - comparison polypropylene glycol based PU / polypropylene glycol based PHU and polytetrahydrofuran based-PU / polytetrahydrofuran based-PHU**

Polypropylene glycol based PU ( 380g/mol), Polytetrahydrofuran based-PU ( 650g/mol); Polypropylene glycol based PHU(468g/mol) and Polytetrahydrofuran based PHU (850g/mol) were immersed into 1 mL of a CH2Cl2/C2H5OH (1:1) solution of minocycline (5 mg/mL) for 25min.

PHU discs (7 mm) were immersed into 1 mL of a $CH_2Cl_2/C_2H_5OH$ (1:1) solution of minocycline (5 mg/mL) for 25min.

The swollen discs (7 mm) were wiped with filter paper and dried in a vacuum oven at room temperature for 2 h. The amount of minocycline loaded in the PHU discs were determined by soaking the films in PBS buffer solution (1 mL) for unloading under static condition. The release solutions were collected and completely replaced with fresh medium before the Minocycline was quantified/analyzed by HPLC spectrometry and compared to a standard concentration curve of minocycline in CH2Cl2/C2H5OH (1:1) solution.

Figure 6 illustrates a much better release for PTHF PHU and PPG PHU with respectively 1 mg and 0,6 mg release after 6000min compared to PTHF based PU and PPG based PPU with respectively 0,01mg and no release after 6000min.

**Example 10: hemocompatibility, coagulation and platelet adhesion tests -comparison polypropylene glycol based PU(PPG-PU)/ polypropylene glycol based PHU (PPG-PHU) and polytetrahydrofuran based-PU(PTHF-PU) / polytetrahydrofuran based-PHU (PTHF-PHU).**

The present example describes five protocols of testing polymer hemocompatibility to further illustrate in examples 11-15 that the medical device comprising Polyhydroxyurethane according to the invention is more hemocompatible, inducing less coagulation and less platelet adhesion than medical device comprising medical grade polyurethanes used as reference.

Test materials used in the following tests:

0.5 $cm^2$-polymeric patches of polyurethane (PU) (medical grade urinary catheter, SpeediCath®, Coloplast and Carbothane™ ref PC3585A, Lubrizol) and PHU (PPG-PHU, PTHF-PHU).

Prior to use, UV-sterilized patches were washed with sterile saline solution under shaking for 3 days. The washing solution was changed every day.

**a. Hemolysis test using washed red blood cells (RBCs): direct contact**

Materials:

- Surface-area-to-volume ratios and hemolysis rate calculations comply with the standard ISO 10993-12, ISO 10993-4 and ASTM Standard F756-13
- Blood collection tubes: sodium citrate Vacutainer tubes (3.2% citrate, BD Biosciences)
- Non-binding 48-well and 96-well plates for suspension culture (polystyrene, Greiner Bio-One)

- Spectrophotometer Spectra max PLUS 384 (Molecular Devices).

Protocol:

1. Collect blood from healthy volunteers by venipuncture into citrated tube. Blood is drawn by using a 21G needle. The first 2 ml are discarded. Blood samples are used within 1 hour post-collection.
2. Centrifuge whole blood at 100xg for 15 min at room temperature (RT)
3. Remove the upper layer (platelet-rich-plasma and leucocyte layer)
4. Resuspend the red blood cell-containing lower layer in two volumes of phosphate buffered saline (PBS), and invert the tube to mix
5. Centrifuge for 10 min at 900xg (Jouan B4i series, S-40 High Throughput Swing-Out Rotor) for 15 min at room temperature (RT) and discard the supernatant
6. Repeat steps 7 and 8 twice for a total of 3 washes or until the supernatant is clear
7. Completely remove the supernatant and use the red blood cells (RBCs) for the hemolysis test
8. Resuspend 1 mL of RBCs concentrate (cut the end of the tip) in 7 mL of PBS and mix the tube by inverting
9. Add 300μl (micro liter) of the mix RBC/PBS in the wells of a 48-well plate containing PU or PHU patches and in an empty well (negative control). For the positive control resuspend 1 mL of RBCs concentrate in 7 mL of distilled $H_2O$ and use 300 μL per well
10. Incubate for 1 or 3 h at 37 °C with gentle agitation 100 rpm using an orbital shaker
11. After the 3-h incubation, transfer samples to test tubes and centrifuge for 15 min at 900xg (Eppendorf bench-top centrifuge) at RT
12. Transfer the supernatant in a new tube. Avoid any solid impurities while aspirating. Pipette 100 μl aliquots to a 96-well polystyrene plate.
13. Measure the absorbance at 545 nm in a multiwell plate reader (optical hemoglobin detection method) according to the ISO 10993-4
14. The hemolysis rate (%) is calculated using the following formula:

$$\text{Hemolysis}(\%) = \frac{(OD_{\text{sample}} - OD_{\text{negativ control}})}{(OD_{\text{pozitive control}} - OD_{\text{negativ control}})} \times 100$$

Materials resulting in over 5% hemolysis are classified hemolytic, between 5 and 2% as slightly hemolytic, and below 2% as nonhemolytic (Totea G et al. Cent. Eur. J. Chem. 12(7) 2014 796-803)

**b. Plasma protein adsorption assay**

Materials:

- Micro BCA protein assay kit from ThermoFisher Ref 23235
- Citrated plasma: pooled normal human plasma from Cryopep (CCN-10)
- Polymeric patches, PU (medical grade urinary catheter, SpeediCath®, Coloplast and Carbothane™ ref PC3585A, Lubrizol) and test PHU (PPG-PHU, PTHF-PHU), 0.5 cm2 area
- Non-binding 48-well plates for suspension culture (polystyrene, Greiner Bio-One)
- Spectrophotometer Spectra max PLUS 384 (Molecular Devices).

Conditions: Negative control: empty well; Positive control: glass cover slip

Protocol:

1. Incubate plasma aliquots with the polymeric patches for 15 min and 2 hours at 37°C in a 1.5mL Eppendorf so that the patches are fully immersed in the plasma.

2. Transfer the polymer patches in a 48 well plate.
3. Wash gently with 300μl of PBS.
4. Proteins are extracted with 350μl PBS containing 1% SDS for 1 hour at 4°C. The Micro BCA (Bicinchonic Acid) Assay is a highly sensitive method detecting down to 5ng/ml of protein. Bicinchonic acid binds to $Cu^+$ ions with a 2:1 stoichiometry delivering high sensitivity.
5. Protein concentration is determined in a spectrophotometer by measuring the absorbance at 562 nm according to the manufacturer's instructions.

c. Contact phase coagulation assay (NaPTT test)

Material:

- 5-CLOT NAPTT Reagent from 5-Diagnostics (5D-51426)
- Kaolin (aluminum silicate hydroxide) (Sigma-Aldrich 1332-58-7)
- Stago STart® 4 Hemostasis Analyzer
- Citrated plasma: pooled normal human plasma from Cryopep (CCN-10)
- $CaCl_2$ solution

Protocol

The NaPTT reagent is a synthetic phospholipid platelet substitute intended for the study of activation of contact phase of coagulation. The Stago Analyzer is a semi-automated system integrated with an electro-mechanical clot detection method (Viscosity-based detection system). Clot formation in citrated human standard plasma is catalyzed by the addition of $Ca^{2+}$ ions as well as by phospholipids. Kaolin was used as a positive control for contact phase activation.

Conditions: Negative control: plasma alone; Positive control: 100mg/mL kaolin

1. Plasma aliquots are defrosted in a water bath at 37°C
2. Incubate plasma aliquots with the polymer patches for 10 min at 37°C in 1.5 mL Eppendorf tubes so that the patches are fully immersed in the plasma. For positive control, incubate 100 μl of plasma with 100mg/mL kaolin for 10 min at 37°C in 1.5 mL Eppendorf tubes.
3. Plasma is then snap frozen in a dry ice/ acetone bath (-78°C) and stored at -80°C until analysis.
4. On the day of the test, plasma is defrosted at 37°C and immediately processed in the Stago STart® 4 apparatus.
5. The 37°C pre-warmed Nodia Reagent is added to 100μl of pre-warmed plasma in a cuvette containing a coated metal bead before initiating coagulation by addition of a pre-warmed calcium solution (8.3mM).
6. The clotting end point is measured by the pendular movement of the bead alimented by an electromagnetic field. Such movement, which is influenced by the viscosity of the plasma, stops when the viscosity becomes maximal, i.e. when plasma coagulation occurs.

d. Platelet adhesion assay

Materials

- Blood collection tubes: sodium citrate Vacutainer tubes (3.2% citrate, BD Biosciences)
- Non-binding 24-well and 96-well plates for suspension culture (polystyrene, Greiner Bio-One)
- Spectrophotometer Spectra max PLUS 384 (Molecular Devices)
- Human Fibrinogen ref 341576 (Calbiochem)
- 2X pNPP buffer (0.1M citrate buffer pH5.4, 10mM p-nitrophenylphosphate, 2% Triton X-100)

Protocol

Conditions: Negative control: BSA-coated wells; Positive control: Fibrinogen-coated wells

Platelet adhesion on surfaces is analysed by using the p-NitroPhenil Phosphate (pNPP) test. Values obtained with this test are directly proportional to the number of platelets adhering to the surface. The pNPP test measures the levels of alkaline and acid phosphatases released upon platelet lysis. The hydrolysis of pNPP, a substrate of these phosphatases, produces p-nitrophenol, which has a maximal absorbance at 405nm and is proportional to the amount of platelets bound to the surface.

1. Blood is drawn from healthy volunteers by venipuncture with a 21-gauge needle into citrated tube. The first 2 ml are discarded. Blood samples are used within 1hour post-collection.
2. Platelet rich plasma (PRP) is prepared by centrifugation at 100xg
3. Platelet density is adjusted to 250,000 platelets/μl by using autologous platelet poor plasma (PPP)
4. Polymer patches are added in BSA-coated wells of 24-well plates.
5. 350μl(micro liter) of platelet rich plasma (PRP) or PPP is incubated with PU or PHU polymer patches for 45 min at 37°C or in empty BSA- or fibrinogen-coated wells under shaking conditions (100rpm).
6. Surfaces are washed 3 times with saline.
7. Lysis of adhered platelets is achieved by adding 200μL of pNPP buffer and incubating for 1hour under shaking and light-protecting conditions.
8. 140μl of a solution of 2M NaOH is added to each well
9. 100μl is transferred in a 96-well plate before measuring the absorbance at 405nm in a spectrophotometer.
10. Background readings from the PPP incubation is subtracted from the PRP reads.
11. Numbers of platelets are determined from a calibration curve performed with increasing platelet densities.

**e. Complement activation assay**

Material:

- Blood collection tubes: sodium citrate Vacutainer tubes (3.2% citrate, BD Biosciences)
- Zymosan (Merck ref 58856-93-2)
- Anti-C5a ELISA kit (ThermoFisher Scientific ref BMS2088)
- Non-binding 24-well plates for suspension culture (polystyrene, Greiner Bio-One)

Conditions: Negative control: blood incubated in empty wells; Positive control: blood incubated with zymosan 10μg/ml

Protocol:

1. Polymer patches are added in BSA-coated wells of 24-well plates
2. 400μL of blood is incubated for 30 min with the test polymer or zymosan at 37°C under 100 rpm shaking
3. Blood is centrifuged 15min at 900xg at RT. Plasma is transferred into a new tube and centrifuged again for 15min at 900xg. Plasma samples are aliquoted and frozen at -80C until analysis.
4. Plasma levels of activated complement (C5a) are determined by ELISA according to the manufacturer's instructions.

**Example 11:** Comparison PU, PPG-PHU, PTHF-PHU in % hemolysis :

**Direct contact of human red blood cells with PPG-PHU or PTHF-PHU do not cause hemolysis**

0.5 $cm^2$-polymeric patches of polyurethane (PU) (medical grade urinary catheter, SpeediCath®, Coloplast) or of polyhydroxyurethane (PHU): PPG (468g/mL)-PHU or PTHF (850g/mL)-PHU were incubated with 300μl of human red blood cell (RBC) concentrates for 1h or 3h at 37°C under shaking (100rpm).

Hemolysis rates were determined as described in the protocol of testing of example 10 at point a.

As shown in Figure 7, hemolysis rates were all below 2%, which is considered non-hemolytic. Thus, neither

PPG-PHU nor PTHF-PHU polymers caused hemolysis. The hemolysis rate was similar to that obtained in direct contact with a medical-grade PU, used as a reference control.

**Example 12:** comparison PU, PPG-PHU in complement activation test:

Complement activation is an immune system's reaction to inflammation and bacterial infection.

**Direct contact of anticoagulated human whole blood with PPG-PHU does not activate the complement cascade**

0.5 $cm^2$-polymeric patches of polyurethane (PU) (medical grade urinary catheter, SpeediCath®, Coloplast) or of polyhydroxyurethane (PHU): PPG (468g/mL)-PHU were incubated with 400μL of human citrated-whole blood for 5, 15 and 30 min at 37°C under 100 rpm shaking. Zymosan (1mg/mL) was used as a positive control.

Plasma was separated and levels of activated complement 5 (C5a) were determined by ELISA as described in the protocol of testing of example 10 at point e.

As shown in Figure 8, PPG-PHU, like medical grade PU, did not activate the complement cascade in contrast to zymosan which induced a clear increase in plasma C5a levels.

**Example 13:** comparison PU, PPG-PHU, PTHF-PHU in contact phase activation coagulation test:

**Less contact phase coagulation activation upon direct contact of human plasma with PPG-PHU or PTHF-PHU than medical grade PU**

0.5 $cm^2$-polymeric patches of polyurethane (PU) (medical grade urinary catheter, SpeediCath®, Coloplast) or of polyhydroxyurethane (PHU): PPG (468g/mL)-PHU, PPG (728g/mL)-PHU or PTHF (850g/mL)-PHU were immersed in human citrated-plasma for 10 min at 37°C. Kaolin (100mg/mL) was used as a coagulation activating stimuli (positive control).

Coagulation activation was assessed in a coagulometer upon plasma recalcification by using the NaPTT reagent as described in the protocol of testing of example 10 at point c. As shown in Figure 9, PPG-PHU (made of PPG of two different molecular weight) induced less coagulation activation than medical grade PU, as shown by more prolonged clotting times. The positive control kaolin led to significantly shorter clotting times than the polymers. Baseline corresponds to plasma that was not put in contact with polymers.

**Example 14:** comparison PU, PPG-PHU in human platelet adherence (absorbance test): **Human platelets do not adhere on PPG-PHU**

0.5 $cm^2$-polymeric patches of polyurethane (PU) (medical grade Carbothane™ ref PC3585A, Lubrizol) or of polyhydroxyurethane (PHU): PPG (468g/mL)-PHU or PPG (728g/mL)-PHU were immersed in human citrated-platelet rich plasma (PRP) for 45 min at 37°C. The positive control for platelet adhesion was PRP added into fibrinogen-coated polystyrene wells for 45 min at 37°C under shaking conditions (100rpm).

Platelet adhesion was analysed by using the p-NitroPhenil Phosphate (pNPP) assay as described in the protocol of testing of example 10 at point b

As shown in Figure 10, human platelets did not adhere to PPG-PHU nor to medical grade PU, while adhesion was observed on fibrinogen.

**Example 15:** comparison PU, PPG-PHU in human protein absorption:

**Less adsorption of human plasma proteins on PPG-PHU than on medical grade PU**

0.5 $cm^2$-polymeric patches of polyurethane (PU) (medical grade Carbothane™ ref PC3585A, Lubrizol) or of polyhydroxyurethane (PHU): PPG (468g/mL)-PHU or PPG (728g/mL)-PHU were immersed in human citrated-plasma for 2 hours at 37°C.

Protein adsorption was analysed by using the Micro BCA (Bicinchonic Acid) assay as described in the protocol of testing of example 10 at point b).

As shown in Figure 11, there was less proteins adsorption onto PPG-PHU (made of PPG of two different molecular weight) than onto medical grade PU.

**Example 16: synthesis allyl polypropylene glycol biscyclo carbonate PPG bisCC Mw 728 and 468**

Allyl functions were grafted on PPG bisCC 728 g/mol by mixing PPG bisCC 728 (1.30 mmol, 1 g, 1 eq.), allylamine (3.25 mmol, 0.19 g, 2.5 eq.) and 2,3,4,6,7,8,9,10-octahydropyrimido[1,2-a]azepine (DBU, 0.13 mmol, 0.02 g, 0.1 eq.) in a vial at 40 °C for 3-4 days. The mixture was then drawn under vacuum for several hours to remove the allylamine excess and obtained PPG with allyl at both extremities of the PPG chain.

The same procedure was used with PPG bisCC 468 g/mol by keeping the same molar ratios.

**Example 17: UV synthesis of polyhydroxy urethane (PHU)** : **polypropylene glycol biscyclo carbonate PPG bisCC 728**

PPG-allyl (1.19 mmol, 1 g, 1 eq.), 2,2-bis(3-sulfanylpropanoyloxymethyl)butyl 3-sulfanylpropanoate (0.79 mmol, 0.31 g, 0.67 eq.) and Irgacure 819 (0.19 mmol, 0.05 g, 0.1 eq., previously solubilized in a few drops of chloroform), were mixed together and homogenized before being poured into a flat Teflon mold (5 x 2.5 x 0.1 cm) and placed 15 minutes under a UV lamp (Omnicure Series 2000, 200 W, Hg, wavelength = 365 nm). The mold was then placed at 70 °C in the oven for 24 hours. The same procedure was used with PPG bisCC 468 g/mol by adapting the quantities of the other reagents to keep the same molar ratios. (PPG-allyl (1.72 mmol, 1g, 1 eq.), 2,2-bis(3-sulfanylpropanoyloxymethyl)butyl 3-sulfanylpropanoate (1.15 mmol, 0.45 g, 0.67 eq.) and Irgacure 819 (0.115 mmol, 0.048 g, 0.1 eq.)

PPGbisCC728 and PPGbisCC468 were then removed from the molds and characterized by different techniques. The conversion of bisCC PPG into PPG-PHU-allyl was checked by $^1$H NMR spectroscopy (figure 5h). The disappearance of the peaks assigned to the cyclo-carbonates of bisCC PPG located at 4.8 and 4.5 ppm is visible, so as the appearance of peaks corresponding to PPG-PHU-allyl in 5.9 - 5.8 and 5.2 - 5.1 ppm. The conversion of PPG-PHU-allyl into UV cured PPG-PHU networks was then checked and confirmed by IR spectroscopy. FTIR-ATR spectra of PPG-PHU-allyl and UV cured network are shown in figure 5i. The disappearance of the peaks assigned to allyl functions is visible at 1642 cm$^{-1}$ (C=C), 991 cm$^{-1}$ (C-H) and 909 cm$^{-1}$(allyl). As additional evidence of the conversion of bisCC PPG during the previous step, the peaks corresponding to functional groups of PPG-PHU are also shown, namely -OH and -NH groups (3200-3500 cm$^{-1}$), and CO (1711 cm$^{-1}$), NH (1535 cm$^{-1}$), N-CO-O and C-O-C (1256 cm$^{-1}$) of urethane groups.

**Example 18: glass-transition temperature** - **comparison thermal and UV cured PPGbisCC728 (PHU1) and PGbisCC468(PHU2).**

Glass-transition temperature was determined using differential scanning calorimetry with TA Instruments DSC Q100 thermal analyzer calibrated with indium, and the curves were analysed using the TA Instruments Universal Analysis 2000 software. The procedure after insertion of the sample was the following: equilibration at -60 °C and isothermal step for 2 min (cycle 1), heating with a ramp of 10 °C/min up to 120 °C followed by an isothermal step for 2 min (cycle 2), equilibration at -60 °C and isothermal step for 2 min (cycle 3), and finally, heating with a ramp of 10 °C/min up to 200 °C (cycle 4). Thermograms were recorded during the fourth cycle.

Differential scanning calorimetry and thermogravimetric analyses were performed on UV cured PHUs and thermal cured PHUs. Transition glass temperatures of UV-PHU$_1$ and UV-PHU$_2$ turned out to be similar to those of thermal PHUs. Indeed, as shown in **Table 3,** UV-PHUs 1 and 2 have $T_g$s of -23.1 and -4.7°C, respectively; while PHUs 1 and 2 have $T_g$s of -25.1 and -1.3°C as show in **Table 2.** The same tendency was observed in thermogravimetry. The TGA curves of UV-PHUs 1 and 2 show thermal degradations similar to those of PHUs 1 and 2, i.e. between 300 and 350 °C.

**Example 19: Equilibrium Water Absorption** - **comparison thermal and UV cured PPGbisCC728(PHU1) and PGbisCC468(PHU2).**

Weight gain of dry PHUs networks was measured after immersion for 4, 8 and 24 hours in a solution of phosphate-buffered saline water (PBS, pH = 7.2). This solution was chosen for its pH (= 7.2) close to the physiological pH (important when considering the medical application).

The equilibrium water absorption (EWA) was determined using the following equation:

$$EWA = (W_{s,w} - W_i)/W_i \times 100\%$$

where $W_{s,w}$ is the weight of the swollen sample in PBS and $W_i$ the weight of the dried sample.

EWAs of thermal and UV cured PPGbisCC728(PHU1) and PGbisCC468(PHU2) have been reported in respectively Tables 4 and 5.

**Example 20: Rheological properties: comparison thermal and UV cured PPGbisCC728(PHU1) and PGbisCC468(PHU2).**

Rheological properties were measured using two types of strain-controlled rheometer. The first one was used for thermal dynamic rheology (ARES from Rheometric Scientific) at 70 °C with the software TA Orchestrator, and the second was used for UV dynamic rheology (ARES G2 from TA Instruments) at room temperature and with the TRIOS program. The UV lamp was the same than for the UV syntheses, namely an Omnicure Series 2000 of 200 W at 365 nm.

Crosslinking formation for both UV cured PHUs prepared according to example 17, were followed by rheology experiments under UV conditions. The storage and loss moduli of the UV cured higher mass (UV-PH728 or UV-$PHU_1$) and lower mass (UV-PH468 or UV-$PHU_2$) PHUs are represented in Figure 12. It can be seen that the gel points corresponding to both UV-$PHU_1$ and UV-$PHU_2$ occur after only 4 minutes, which is earlier than for the corresponding thermal cured PHUs, i.e. 23 and 16 minutes for $PHU_1$ and $PHU_2$, respectively. Crosslinking networks are thereby formed more rapidly under UV than thermal conditions, probably due to the high reactivity of thiols towards allyl functions. However, just like in the thermal approach, the gel point of UV-$PHU_1$ ($GP_1$) is lower than the gel point of $PHU_2$ ($GP_2$), which means that the crosslinking network is formed faster with the polymer of smaller masses. The storage modulus of $PHU_2$ being higher than the one of $PHU_1$ also indicates that its crosslinking degree is superior because the molar masses between the crosslinking nodes of $PHU_2$ are smaller.

Mechanical properties such as Young's modulus, stress at break and elongation at break were carried out under dry and hydrated state. Under dry condition, UV-$PHU_1$ are practically identical of those of $PHU_1$, the similar PHU obtained by thermal crosslinking route (cf. **Tables 2 and 3).** However, this is not the case for UV-$PHU_2$ and $PHU_2$. Indeed, while $PHU_2$ has a Young's modulus of 15.62 MPa, UV-$PHU_2$ shows an elastic modulus of only 1.53 MPa. Its stress and elongation at break are also lower compared to $PHU_2$.

As shown in **Table 5,** UV-$PHU_2$ presents a higher $T_g$ value and higher stress and elongation at break in the hydrated state than UV-$PHU_1$. The difference between the Young's modulus values obtained under dry and wet conditions is also much less significant than with thermal cured PHUs. This can be explained by a higher hydrophobic content of UV cured PHUs, due to the insertion of allyl groups and 2,2-bis(3-sulfanylpropanoyloxymethyl) butyl 3-sulfanylpropanoate. Indeed, UV cured PHUs contain as much -OH groups as thermal cured PHUs. However, the ratio between polar and non-polar groups decreases drastically in UV cured PHUs. This hypothesis of higher hydrophobic content, preventing water from interacting with hydroxyl groups along the chains, is confirmed by low EWAs (Equilibrium Water Absorption) obtained with UV cured PHUs (around 10%, cf. **Table 5).**

**Table 2:** *Network characteristics of thermal cured PHU1 et PHU2*

| ***Code*** | ***PPG*** | ***$T_g$ (°C)*** | ***Swelling ratio*** (%) | ***Gel time*** (s) | ***G' plateau (Pa)*** | ***Young's Modulus (MPa)*** | ***Stress at break (MPa)*** | ***Elongation at break*** (%) |
|---|---|---|---|---|---|---|---|---|
| **$PHU_1$** | 1 | -25.1 | 403 | 988 | $2.1 \times 10^4$ | 0.98 | 0.53 | 143.82 |
| **$PHU_2$** | 2 | -1.3 | 348 | 4188 | $4.3 \times 10^5$ | 15.62 | 1.44 | 233.96 |

***Table 3.*** *Network characteristics of UV-$PHU_1$ and UV-$PHU_2$.*

| ***Code*** | ***PPG*** | ***$T_g$ (°C)*** | ***Swelling ratio*** (%) | ***Gel time (s)*** | ***G' plateau (Pa)*** | ***Young's Modulus (MPa)*** | ***Stress at break (MPa)*** | ***Elongation at break*** (%) |
|---|---|---|---|---|---|---|---|---|
| **UV-$PHU_1$** | **1** | -23.1 | 397 | 258 | $2.6 \times 10^5$ | 1.10 | 0.60 | 149.69 |
| **UV-$PHU_2$** | 2 | -4.7 | 196 | 241 | $5.1 \times 10^5$ | 1.53 | 0.95 | 166.16 |

***Table 4.*** *Network characteristics of* **$PHU_1$** *and* $PHU_2$ *in the hydrated state.*

| ***Code*** | ***Contact angle (°)*** | ***EWA*** (%) | ***Wet Young's Modulus (MPa)*** | ***Wet stress at break (MPa)*** | ***Wet elongation at break*** (%) |
|---|---|---|---|---|---|
| **$PHU_1$** | 59 | 35 | 0.70 | 0.20 | 45.50 |
| **$PHU_2$** | 55 | 26 | 1.51 | 0.17 | 16.73 |

**Table** 5. *Network characteristics of UV-$PHU_1$ and* UV-$PHU_2$ *in the hydrated state.*

| ***Code*** | ***Contact angle (°)*** | ***EWA (%)*** | ***Wet Young's Modulus (MPa)*** | ***Wet stress at break (MPa)*** | ***Wet elongation at break*** (%) |
|---|---|---|---|---|---|
| **UV-$PHU_1$** | 60 | 12 | 1.18 | 0.53 | 98.77 |
| **UV-$PHU_2$** | 47 | 9 | 1.08 | 0.41 | 75.52 |

**Example 21: 3D printing of UV-$PHU_1$ and UV-$PHU_2$**

Thanks to the fast crosslinking rate of UV cured PHUs, solutions were prepared with the same protocols as UV-$PHU_1$ and UV-$PHU_2$ of example 17 in order to maintain the same conditions.

A sheet of PHUs was printed on a heating plate of a 3D printer. The parameters of 3D printing were a flow rate of 0.03 ml/min on a plate heated up to 70 °C and UV light exposure percentage of 50%. For the printing tests, UV-$PHU_2$ was chosen since it showed the best mechanical properties. After removal of the printed PHUs from the plate, it is placed at 60°C in a UV oven for 10 minutes. The 3D printed PHU showing the best results is shown in Table 6 (3D-$PHU_1$), in which 3D-PHU1 is compared to UV-$PHU_2$, the same polymer but crosslinked without being 3D printed (cf. example 17). 3D-$PHU_1$ showed the same EWA than UV-$PHU_2$ (only 10%) and even higher mechanical properties, in both dry and hydrated states. These results therefore pave the way for the preparation of medical device made of UV-PHUs.

**Table 6.** *Network characteristics of the 3D printed PHU in dry and hydrated state.*

| ***Code*** | ***Swelling ratio (%)*** | ***EWA (%)*** | ***Young's Modulus (MPa)*** | ***Wet Young's Modulus (MPa)*** | ***Stress at break (MPa)*** | ***Wet stress at break (MPa)*** | ***Elongation at break (%)*** | ***Wet elongation at break (%)*** |
|---|---|---|---|---|---|---|---|---|
| **UV-$PHU_2$** | 196 | 9 | 1.53 | 1.08 | 0.95 | 0.41 | 166.16 | 75.52 |
| **3D-$PHU_1$** | 289 | 10 | 2.52 | 1.34 | 0.97 | 0.62 | 143.39 | 100.02 |

**Example 22: preparation of a prosthetic heart valve based on PHU.**

A tri-leaflet prosthetic heart valve prototype based on polyhydroxyurethane has been prepared by compression molding. The valve is at size 24 mm with a leaflet thickness of 0,5mm and is reproduced at Figure 2.

Bis(cyclocarbonate) polypropylene glycol) (bisCC PPG) 728 g/mol (1.92 mmol, 1.40 g, 1 eq., 4-((4-aminocyclohexyl)methyl) cyclohexanamine (MBCHA, 0.38 mmol, 0.08 g, 0.2 eq.) and N',N'-bis(2-aminoethyl)ethane-1,2-diamine (TAEA, 1.02 mmol, 0.15 g, 0.53 eq.) were introduced in a vial and magnetically stirred at 40 °C for 10 minutes. This solution was then injected inside a mold and placed at 70 °C in an oven for 24 hours.

The valve prototype was then tested in a pulse duplicator from Vivitro Labs, product code 18363 for its hydrodynamic properties and according to ISO 5840 requirements. Flow and pressure data were acquired under simulated cardiac conditions.

Data were acquired using a mean arterial pressure equal to 100mmHg and a heart rate of 70 beat per minute. The effective orifice area (EOA) and regurge fraction were determined and compared to commercially available bioprotheses (AvalusTM, MosaicTM from Medtronic and TrifectaTM from Abbott). The valve prototype is obtained with ISO criteria in terms of EOA (ISO standard > 1.25cm2) and regurge fraction percentage (ISO standard < 10%).

The project leading to this application has received funding from the Interreg Euregio Meuse-Rhine (grant agreement EMR100 PolyValve).

## Claims

1. An implantable medical device comprising one or more polyhydroxyurethane (PHU) 1 **characterised in that** the one or more polyhydroxyurethane (PHU) is based on formula (1) or on formula (9)

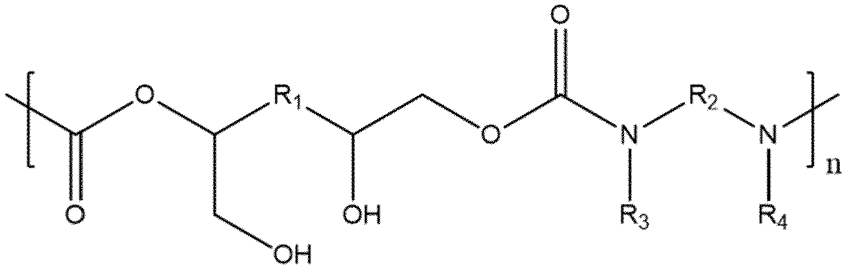

(1)

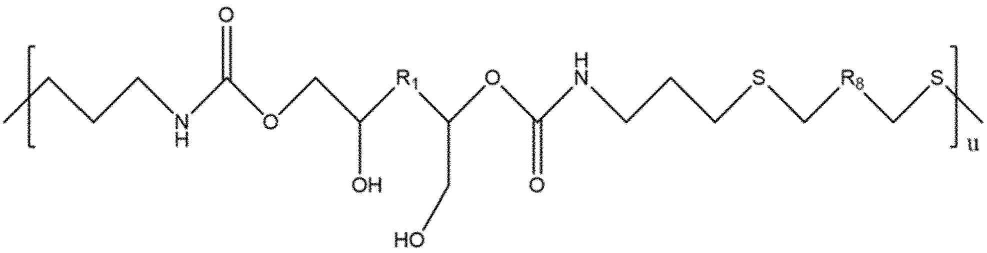

(9)

wherein $R_1$ is a linear or branched hydrocarbon chain, which may be unsubstituted or substituted and wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms;
wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon atoms, more preferably from 2 to 20 carbon atoms, even more preferably from 2 to 15 carbon atoms, or a mixture thereof; preferably $R_2$ is an alkylene;
wherein $R_3$ and $R_4$ are identical or different and represent H, a straight or branched saturated hydrocarbon chain $C_{1-6}$-alkyl having 1 to 6 carbon atoms; $C_{1-6}$-alkoxy alkyl having 1 to 6 carbon atoms
and wherein n is an integer ≥ 1, preferably n is between 1 and 100, more preferably between 1 and 20;
with the exception that if $R_3$ and/or $R_4$ represents an hydrogen atom, than $R_1$ is not

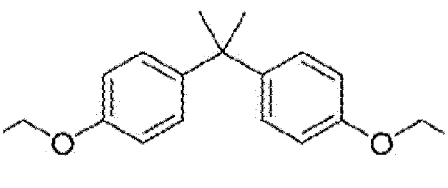

,

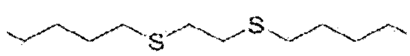

,

or

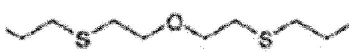

;

and with the exception that if $R_3$ and/or $R_4$ represents an hydrogen atom, then $R_2$ is not

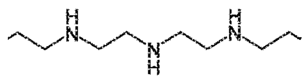

;

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene; and
wherein u is an integer ≥ 1, preferably between 1 and 10 more preferably between 1 and 5.

2. The medical device according to claim 1 wherein $R_1$ is polypropylene oxide, polyethylene oxide, polybutylene oxide, polytetrahydrofuran, or a mixture thereof.

3. The medical device according to claim 1 comprising polyhydroxyurethane (PHU) based on formula (1) wherein $R_1$ is polypropylene oxide, polyethylene oxide, polybutylene oxide, polytetrahydrofuran, or a mixture thereof and $R_2$ is an alkylene.

4. The medical device according to claim 1 wherein the polyhydroxyurethane based on formula (1) is obtained by thermal polyaddition

of polycyclocarbonate of formula (2)

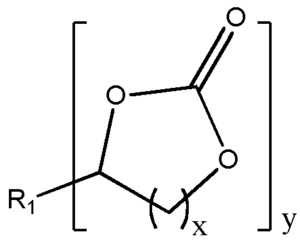

(2)

wherein R1 is a carbon bond between a cyclic carbonate ring or is a linear or branched hydrocarbon chain, which may be unsubstituted or substituted and wherein one or more hydrocarbon groups of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 40 carbon atoms ; preferably $R_1$ is polypropylene oxide, polyethyleneoxide, polybutylene oxide, polytetrahydrofurane or a mixture thereof;
wherein x is an integer between 1 and 4;
wherein y is an integer superior or equal to 2;
with a polyamine of formula (3)

$$R_2\left(NR_5R_6\right)_z \quad (3)$$

wherein z is an integer between 2 and 6;
wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms; preferably $R_2$ is an alkylene;

wherein $R_5$ and $R_6$ are identical or different and represent H, a straight or branched saturated hydrocarbon chain $C_{1-6}$-alkyl having 1 to 6 carbon atoms; preferably $R_5$ and $R_6$ are hydrogen;
with the exception that if $R_5$ and/or $R_6$ are hydrogen, then $R_2$ is not

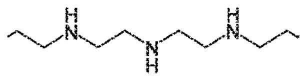

;

with the exception that if $R_5$ and/or $R_6$ are hydrogen atom, then $R_1$ is not

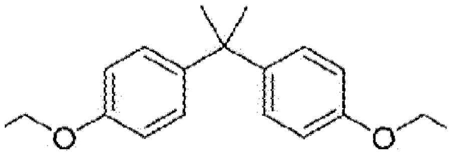

,

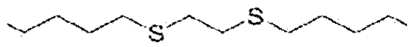

,

or

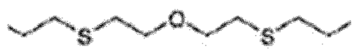

.

**5.** The medical device according to claim 4, characterised-in-that the polycylocarbonate of formula (2) is a polypropylene glycol)-bis-cyclocarbonate (PPGbisCC) of formula (5) wherein m is an integer ≥ 1, preferably between 1 and 100, most preferably between 1 and 10;

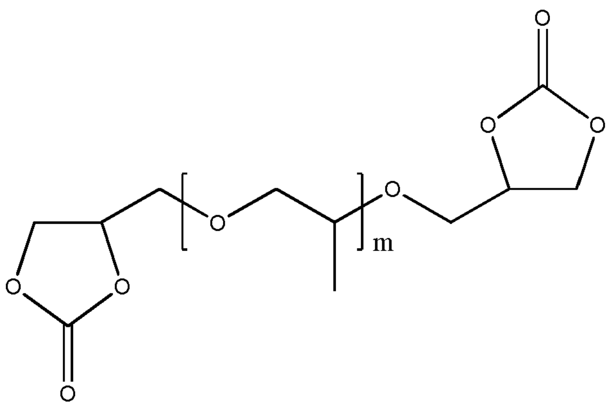

(5)

and the polyamine is 4-4'methylene-bis-(cyclohexylamine) (MBCHA) of formula (4)

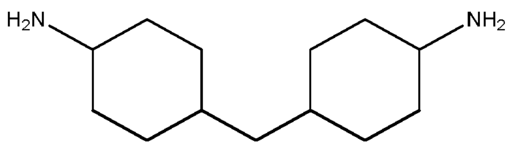

(4).

**6.** The medical device according to claim 5, characterised-in-that the polyamine is 4,4'methylene-bis-(cyclohexylamine) of formula (4) in combination with tris(2-aminoethyl)amine (TAEA) of formula (6).

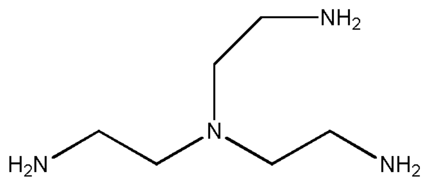
(6)

7. The medical device according to claim 4, characterised-in-that the polycyclocarbonate is polytetrahydrofuranebiscyclocarbonate of formula (8) wherein p is an integer is ≥ 1, preferably between 1 and 100, most preferably between 1 and 10;

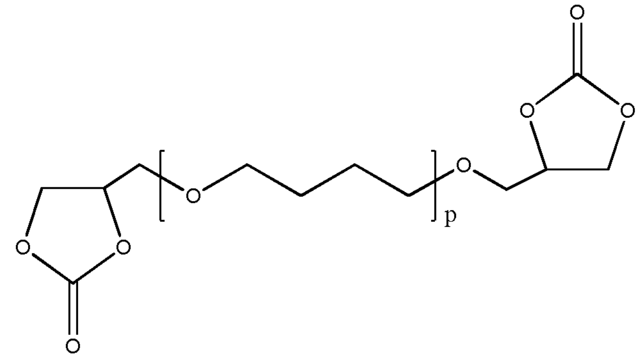
(8)

and the polyamine is 4-4'methylene-bis-(cyclohexylamine) (MBCHA) of formula (4)

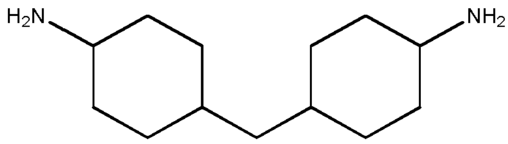
(4).

8. The medical device according to claim 7, characterised-in-that the polyamine is polyamine is 4-4'methylene-bis-(cyclohexylamine) of formula (4) in combination with tris(2-aminoethyl)amine (TAEA) of formula (6)

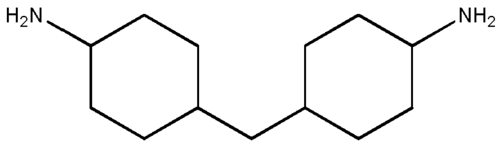
(4)
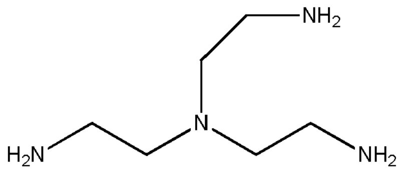
(6).

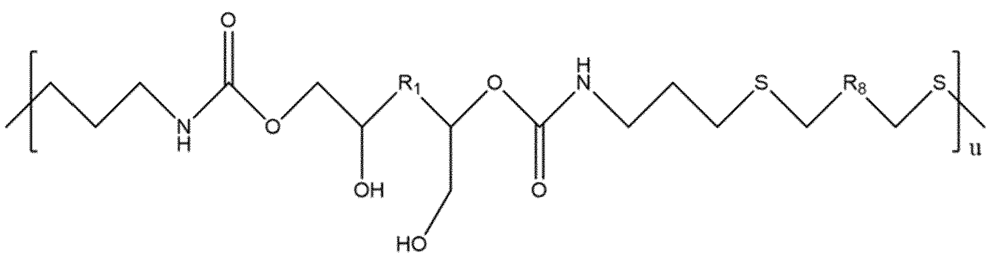
(9).

9. The medical device according to claim 1, wherein the polyhydroxyurethane (PHU) based on formula (9) is obtained by thermal addition of polycarbonate of formula (2)

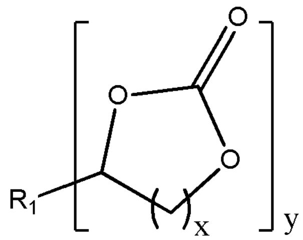
(2)

wherein $R_1$ is a carbon bond between a cyclic carbonate ring or is a linear or branched hydrocarbon chain, which may be unsubstituted or substituted and wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms;
wherein y is an integer equal or superior to 2;
wherein X is an integer between 1 and 4;
with a polyamine of formula (15) to obtain a polycarbamatehydroxy derivative bearing photoresponsive groups

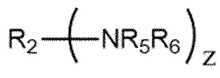
(15);

wherein $R_2$ is a linear or branched hydrocarbon unsaturated chain with 2 to 40 carbon atoms, said linear or branched hydrocarbon unsaturated chain bearing at least a photoresponsive group;
wherein $R_5$ is hydrogen and $R_6$ is hydrogen, a straight or branched saturated hydrocarbon chain $C_{1-6}$ alkyl having 1 to 6 carbon atoms, preferably $R_6$ is hydrogen; and
wherein z is an integer ≥ 1, preferably between 1 and 6;
and the polycarbamatehydroxy derivative bearing photoresponsive groups is further reacted by UV polyaddition with a thiol of formula (16), preferably a dithiol of formula (11) and a photoinitiator;

$R_8\text{-}(SH)_k$ (16);

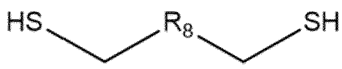
(11)

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene;
wherein k is an integer higher or equal to 2, preferably between 2 and 6.

**10.** The medical device according to claim 9, wherein the photoresponsive group is an allyl group and the polycarbamatehydroxy derivative bearing photoresponsive groups is polyallylcarbamatehydroxy derivative of formula (10)

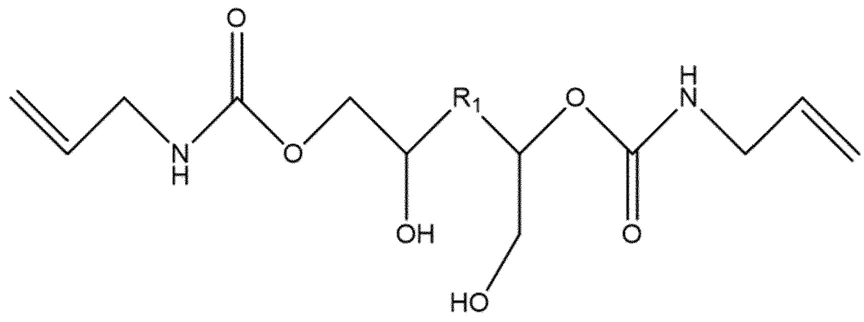

(10)

wherein $R_1$ is a linear or branched hydrocarbon chain, which may be unsubstituted or substituted and wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms; preferably $R_1$ is polypropylene oxide, polyethylene oxide, polybutylene oxide, polytetrahydrofurane, or a mixture thereof;
and the thiol is a dithiol of formula (11)

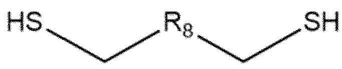

(11)

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene.

**11.** The medical device according to claim 10 characterised-in- that the polyallylcarbamatehydroxy derivative is a polyallylcarbamatehydroxypropyleneoxide of formula (14)

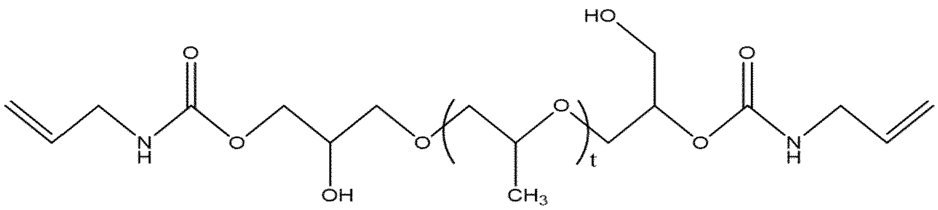

(14)

wherein $t \geq 1$, preferably t is between 1 and 1000 and most preferably between 1 and 20.

**12.** The medical device according to anyone of claim 1 to 11, which is a catheter or a prosthetic heart valve.

**13.** A Method of manufacturing an implantable medical device comprising polyhydroxyurethane according to any one of claim 1-11; **characterised in that** the method comprises step (a) or (b) corresponding respectively to a thermal or UV polymerisation or a combination of both:

a) mixing a polycyclocarbonate of formula (2) with a polyamine of formula (3) and optionally a catalyst to obtain, by thermal polymerization, a polyhydroxyurethane based on formula (1);

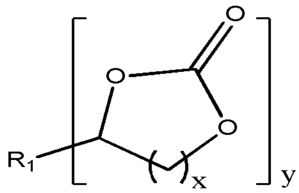 (2)

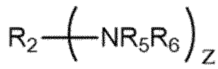 (3)

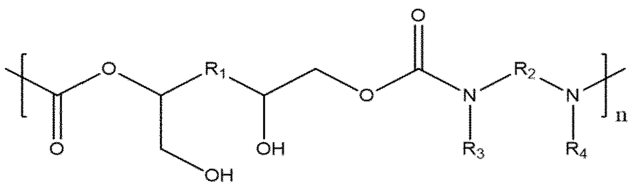

(1)

wherein $R_1$ is a carbon bond between a cyclic carbonate ring or is a linear or branched hydrocarbon chain, which may be unsubstituted or substituted and wherein one or more hydrocarbon groups of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 40 carbon atoms ; preferably $R_1$ is polypropylene oxide, polyethyleneoxide, polybutylene oxide, polytetrahydrofurane or a mixture thereof;
wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon atoms, more preferably from 2 to 20 carbon atoms, even more preferably from 2 to 15 carbon atoms;
wherein $R_5$ and $R_6$ are identical or different and represent H, a straight or branched saturated hydrocarbon chain $C_{1-6}$-alkyl having 1 to 6 carbon atoms; preferably $R_5$ and $R_6$ are hydrogen,
with the exception that if $R_5$ and/or $R_6$ are hydrogen then $R_2$ is not

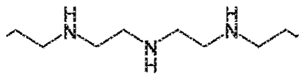

;

and with the exception that if $R_5$ and/or $R_6$ are hydrogen, then $R_1$ is not

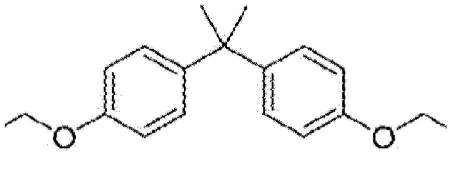

,

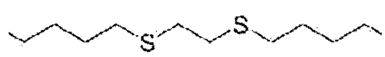

,

or

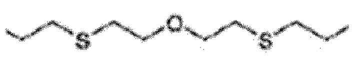

;

wherein $R_3$ and $R_4$ are identical or different and represent H, a straight or branched saturated hydrocarbon chain $C_{1-6}$-alkyl having 1 to 6 carbon atoms ; $C_{1-6}$-alkoxy alkyl having 1 to 6 carbon atoms;
and wherein n is an integer ≥ 1, preferably n is between 1 and 100, more preferably between 1 and 20;
with the exception that if $R_3$ and/or $R_4$ represents an hydrogen atom, $R_1$ is not

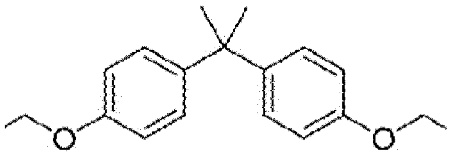

,

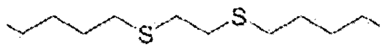

,

or

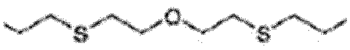

;

b) mixing a polyvinylcarbamatehydroxy derivative bearing photoresponsive groups, preferably of formula (10) with a thiol of formula (16), preferably of formula(11) and a photoiniator to obtain, by UV polymerization, a polyhydroxyurethane based on formula (9);

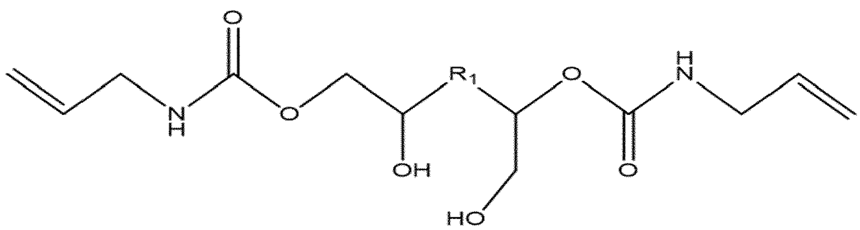

(10)

$R_8$-$(SH)_k$ (16);

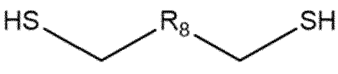

(11)

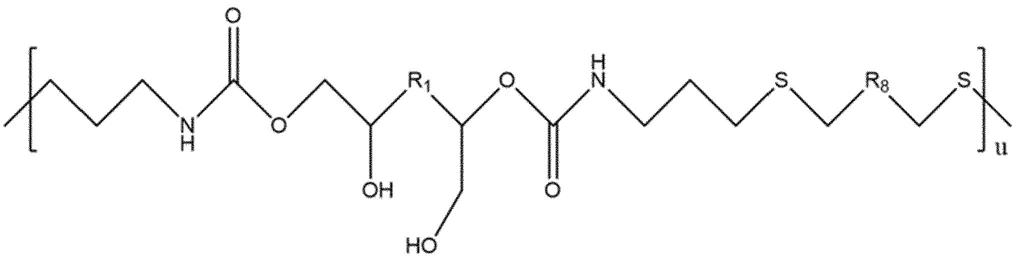

(9)

wherein $R_1$ is a linear or branched hydrocarbon chain, which may be unsubstituted or substituted and wherein one or more hydrocarbon group of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 60 carbon atoms ; preferably $R_1$ is polypropyleneoxide, polyethylene oxide, polybutylene oxide, polytetrahydrofurane, or a mixture thereof;

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene; and

wherein k is an integer $\geq 2$, preferably between 2 and 6;

wherein u is an integer $\geq 1$, preferably between 1 and 10 more preferably between 1 and 5.

**14.** A method of coating an implantable medical device comprising a polyhydroxyurethane according to any one of claim 1-11; the method comprising depositing onto a surface of the medical device at least one layer of polyhydroxyurethane based on formula (1) and/or on formula (9)

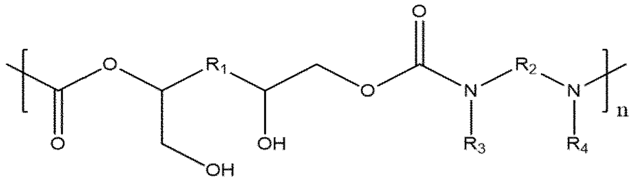

(1)

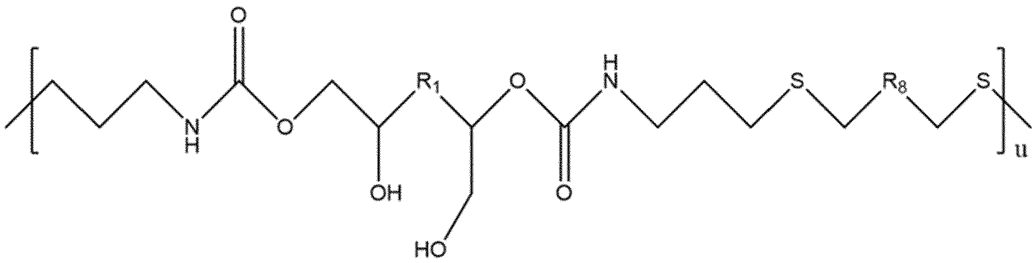

(9)

wherein $R_1$ is a carbon bond between a cyclic carbonate ring or is a linear or branched hydrocarbon chain, which may be unsubstituted or substituted and wherein one or more hydrocarbon groups of said hydrocarbon chain may be replaced by an heteroatom, a ketone, a cycloalkyl, an heterocycle, an aryl or a heteroaryl, each of which may be substituted or unsubstituted, said hydrocarbon chain having from 2 to 40 carbon atoms ; preferably $R_1$ is polypropylene oxide, polyethyleneoxide, polybutylene oxide, polytetrahydrofurane or a mixture thereof;
wherein $R_2$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, preferably from 2 to 60 carbon atoms, more preferably from 2 to 20 carbon atoms, even more preferably from 2 to 15 carbon atoms;
wherein $R_3$ and $R_4$ are identical or different and represent H, a straight or branched saturated hydrocarbon chain $C_{1-6}$-alkyl having 1 to 6 carbon atoms; $C_{1-6}$-alkoxy alkyl having 1 to 6 carbon atoms;
with the exception that if $R_3$ and/or $R_4$ represents an hydrogen atom, then $R_1$ is not

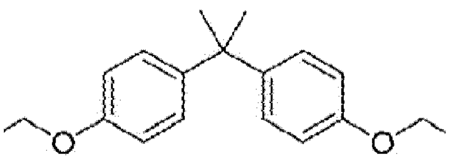

,

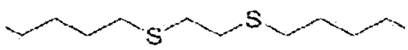

,

or

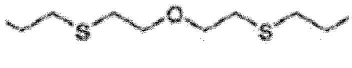

,

and with the exception that if $R_3$ and/or $R_4$ represents an hydrogen atom, then $R_2$ is not

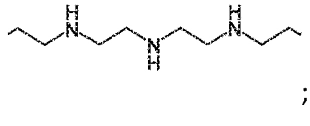

;

wherein $R_8$ is an aryl or a heteroaryl, each of which may be unsubstituted or substituted, or a linear or branched hydrocarbon chain, which may be unsubstituted or substituted, and wherein one or several hydrocarbon groups of said hydrocarbon chain may be replaced by a heteroatom, a cycloalkyl or an heterocycle, each of which may be unsubstituted or substituted, said hydrocarbon chain having at least 2 carbon atoms, in particular from 2 to 60 carbon atoms, more particularly from 2 to 20 carbon atoms, even more particularly from 2 to 15 carbon atoms, or a mixture thereof; preferably $R_8$ is an alkylene;
wherein u is an integer ≥ 1, preferably between 1 and 10 more preferably between 1 and 5; and wherein n is an integer ≥ 1, preferably n is between 1 and 100, more preferably between 1 and 20.

**15.** The medical device according to anyone of claim 1 to 12 which further comprises a bioactive molecule, preferably an antibacterial or an anti-thrombotic agent.

**Patentansprüche**

**1.** Implantierbare medizinische Vorrichtung, umfassend ein oder mehrere Polyhydroxyurethane (PHU) 1, **dadurch gekennzeichnet, dass** das eine oder die mehreren Polyhydroxyurethane (PHU) auf Formel (1) oder auf Formel (9) basieren:

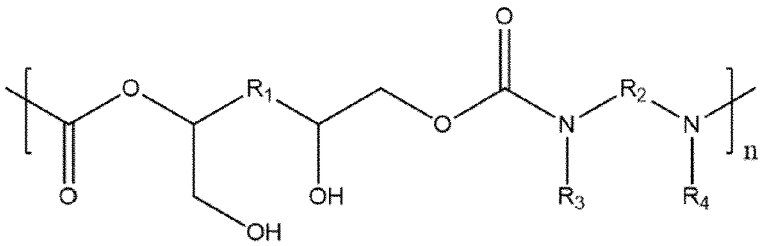

(1)

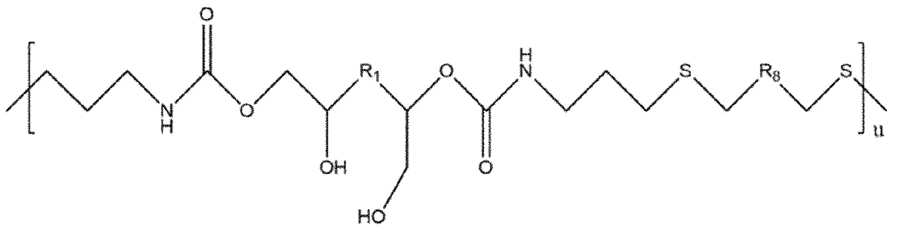

(9)

wobei $R_1$ für eine lineare oder verzweigte Kohlenwasserstoffkette steht, die unsubstituiert oder substituiert sein kann, und wobei eine oder mehrere Kohlenwasserstoffgruppen der Kohlenwasserstoffkette durch ein Heteroatom, ein Keton, ein Cycloalkyl, einen Heterocyclus, ein Aryl oder ein Heteroaryl ersetzt sein können, wobei jede dieser Gruppen substituiert oder unsubstituiert sein kann, wobei die Kohlenwasserstoffkette 2 bis 60 Kohlenstoffatome aufweist;
wobei $R_2$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, vorzugsweise 2 bis 60 Kohlenstoffatome, weiter bevorzugt 2 bis 20 Kohlenstoffatome, noch weiter bevorzugt 2 bis 15 Kohlenstoffatome, aufweist, oder eine Mischung davon steht; vorzugsweise $R_2$ für ein Alkylen steht;
wobei $R_3$ und $R_4$ gleich oder verschieden sind und für H, eine gerade oder verzweigte gesättigte Kohlenwasserstoffkette $C_{1-6}$-Alkyl mit 1 bis 6 Kohlenstoffatomen; $C_{1-6}$-Alkoxyalkyl mit 1 bis 6 Kohlenstoffatomen stehen und wobei n für eine ganze Zahl ≥ 1 steht, vorzugsweise n zwischen 1 und 100, weiter bevorzugt zwischen 1 und 20, steht;
mit der Ausnahme, dass dann, wenn $R_3$ und/oder $R_4$ für ein Wasserstoffatom stehen, $R_3$ nicht für

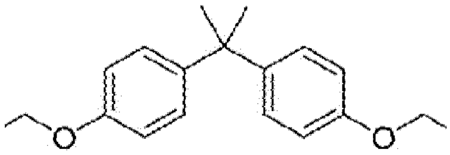 , 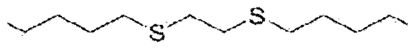

oder

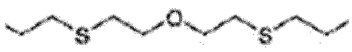

steht;
und mit der Ausnahme, dass dann, wenn $R_3$ und/oder $R_4$ für ein Wasserstoffatom stehen, $R_2$ nicht für

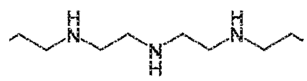

steht;
wobei $R_8$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, insbesondere 2 bis 60 Kohlenstoffatome, spezieller 2 bis 20 Kohlenstoffatome, noch spezieller 2 bis 15 Kohlenstoffatome, aufweist, oder eine Mischung davon steht; vorzugsweise $R_8$ für ein Alkylen steht; und
wobei u für eine ganze Zahl ≥ 1, vorzugsweise zwischen 1 und 10, weiter bevorzugt zwischen 1 und 5, steht.

**2.** Medizinische Vorrichtung nach Anspruch 1, wobei $R_1$ für Polypropylenoxid, Polyethylenoxid, Polybutylenoxid, Polytetrahydrofuran oder eine Mischung davon steht.

**3.** Medizinische Vorrichtung nach Anspruch 1, umfassend Polyhydroxyurethan (PHU) auf der Basis von Formel (1), wobei $R_1$ für Polypropylenoxid, Polyethylenoxid, Polybutylenoxid, Polytetrahydrofuran oder eine Mischung davon steht und $R_2$ für ein Alkylen steht.

**4.** Medizinische Vorrichtung nach Anspruch 1, wobei das Polyhydroxyurethan auf Basis von Formel (1) erhalten wird durch thermische Polyaddition von Polycyclocarbonat der Formel (2)

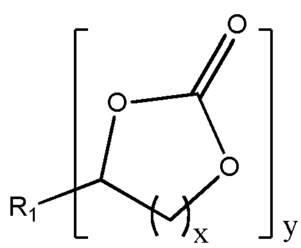 (2)

wobei $R_1$ für eine Kohlenstoffbindung zwischen einem Cyclocarbonatring oder für eine lineare oder verzweigte Kohlenwasserstoffkette, die unsubstituiert oder substituiert sein kann, und wobei ein oder mehrere Kohlenwasserstoffgruppen der Kohlenwasserstoffkette durch ein Heteroatom, ein Keton, ein Cycloalkyl, einen Heterocyclus, ein Aryl oder ein Heteroaryl ersetzt sein können, wobei jede dieser Gruppen substituiert oder unsubstituiert sein kann, wobei die Kohlenwasserstoffkette 2 bis 40 Kohlenstoffatome aufweist, steht; vorzugsweise $R_1$ für Polypropylenoxid, Polyethylenoxid, Polybutylenoxid, Polytetrahydrofuran oder eine Mischung davon steht;
wobei x für eine ganze Zahl zwischen 1 und 4 steht;
wobei y für eine ganze Zahl größer oder gleich 2 steht; mit einem Polyamin der Formel (3)

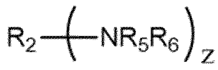 (3)

wobei z für eine ganze Zahl zwischen 2 und 6 steht;
wobei $R_2$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, insbesondere 2 bis 60 Kohlenstoffatome, spezieller 2 bis 20 Kohlenstoffatome, noch spezieller 2 bis 15 Kohlenstoffatome, aufweist, steht; vorzugsweise $R_2$ für ein Alkylen steht;
wobei $R_5$ und $R_6$ gleich oder verschieden sind und für H , eine gerade oder verzweigte gesättigte Kohlenwasserstoffkette $C_{1-6}$-Alkyl mit 1 bis 6 Kohlenstoffatomen stehen; vorzugsweise $R_5$ und $R_6$ für Wasserstoff stehen;
mit der Ausnahme, dass dann, wenn $R_5$ und/oder $R_6$ für Wasserstoff stehen, $R_2$ nicht für

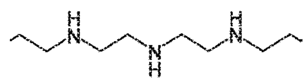

steht;
mit der Ausnahme, dass dann, wenn $R_5$ und/oder $R_6$ für ein Wasserstoffatom stehen, $R_1$ nicht für

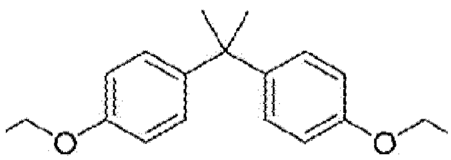 , 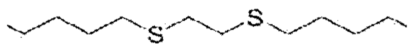

oder

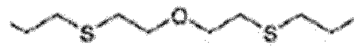

steht.

**5.** Medizinische Vorrichtung nach Anspruch 4, **dadurch gekennzeichnet, dass** es sich bei dem Polycylocarbonat der Formel (2) um ein Poly(propylenglykol)biscyclocarbonat (PPGbisCC) der Formel (5) handelt, wobei m für eine ganze Zahl ≥ 1, vorzugsweise zwischen 1 und 100, ganz besonders bevorzugt zwischen 1 und 10, steht;

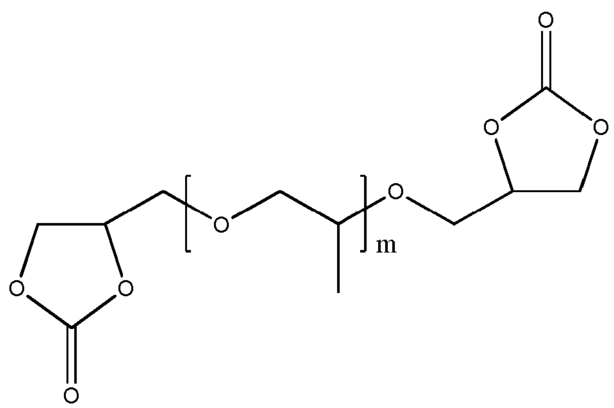 (5)

und es sich bei dem Polyamin um 4-4'-Methylenbis(cyclohexylamin) (MBCHA) der Formel (4)

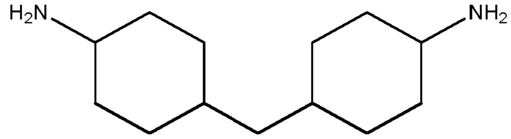
(4)

handelt.

6. Medizinische Vorrichtung nach Anspruch 5, **dadurch gekennzeichnet, dass** es sich bei dem Polyamin um 4,4'-Methylenbis(cyclohexylamin) der Formel (4) in Kombination mit Tris(2-aminoethyl)amin (TAEA) der Formel (6) handelt.

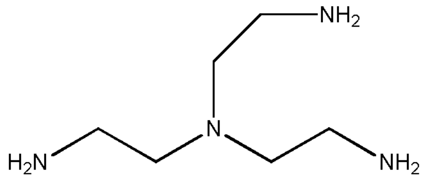
(6)

7. Medizinische Vorrichtung nach Anspruch 4, **dadurch gekennzeichnet, dass** es sich bei dem Polycyclocarbonat um Polytetrahydrofuranbiscyclocarbonat der Formel (8) handelt, wobei p für eine ganze Zahl ≥ 1, vorzugsweise zwischen 1 und 100, ganz besonders bevorzugt zwischen 1 und 10, steht;

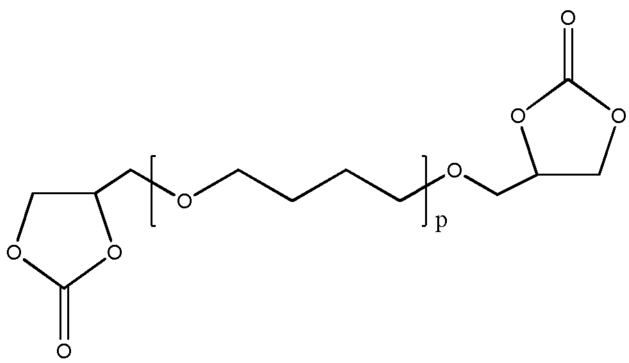
(8)

und es sich bei dem Polyamin um 4-4'-Methylenbis(cyclohexylamin) (MBCHA) der Formel (4)

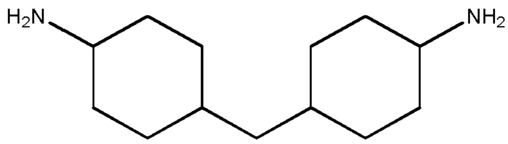
(4)

handelt.

8. Medizinische Vorrichtung nach Anspruch 7, **dadurch gekennzeichnet, dass** es sich bei dem Polyamin um 4-4'-Methylenbis(cyclohexylamin) der Formel (4) in Kombination mit Tris(2-aminoethyl)amin (TAEA) der Formel (6) handelt.

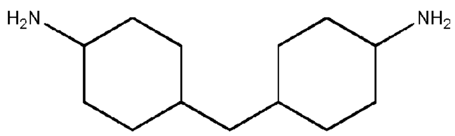
(4)
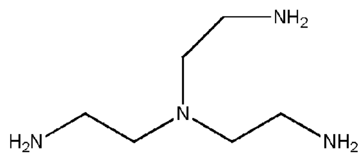
(6).

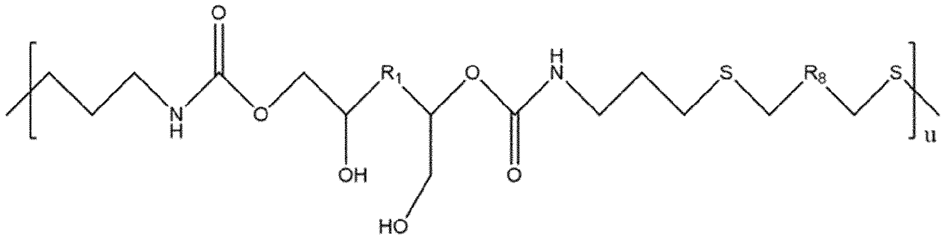

(9).

9. Medizinische Vorrichtung nach Anspruch 1, wobei das Polyhydroxyurethan (PHU) auf Basis von Formel (9) erhalten wird durch thermische Addition von Polycarbonat der Formel (2)

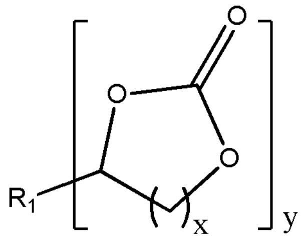

(2)

wobei $R_1$ für eine Kohlenstoffbindung zwischen einem Cyclocarbonatring oder für eine lineare oder verzweigte Kohlenwasserstoffkette, die unsubstituiert oder substituiert sein kann, und wobei ein oder mehrere Kohlenwasserstoffgruppen der Kohlenwasserstoffkette durch ein Heteroatom, ein Keton, ein Cycloalkyl, einen Heterocyclus, ein Aryl oder ein Heteroaryl ersetzt sein können, wobei jede dieser Gruppen substituiert oder unsubstituiert sein kann, wobei die Kohlenwasserstoffkette 2 bis 60 Kohlenstoffatome aufweist, steht;
wobei y für eine ganze Zahl gleich oder größer als 2 steht;
wobei x für eine ganze Zahl zwischen 1 und 4 steht;
mit einem Polyamin der Formel (15) unter Erhalt eines Polycarbamathydroxyderivats, das auf Licht ansprechende Gruppen trägt:

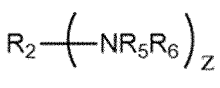

(15);

wobei $R_2$ für eine lineare oder verzweigte ungesättigte Kohlenwasserstoffkette mit 2 bis 40 Kohlenstoffatomen steht, wobei die lineare oder verzweigte ungesättigte Kohlenwasserstoffkette mindestens eine auf Licht ansprechende Gruppe trägt;
wobei $R_5$ für Wasserstoff steht und $R_6$ für Wasserstoff, eine gerade oder verzweigte gesättigte Kohlenwasserstoffkette $C_{1-6}$-Alkyl mit 1 bis 6 Kohlenstoffatomen steht, vorzugsweise $R_6$ für Wasserstoff steht; und
wobei z für eine ganze Zahl $\geq 1$, vorzugsweise zwischen 1 und 6, steht;
und das Polycarbamathydroxyderivat, das auf Licht ansprechende Gruppen trägt,
weiter durch UV-Polyaddition mit einem Thiol der Formel (16), vorzugsweise einem Dithiol der Formel (11), und einem Photoinitiator umgesetzt wird;

$R_8$-$(SH)_k$ (16);

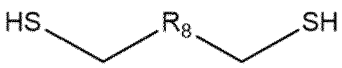

(11)

wobei $R_8$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, insbesondere 2 bis 60 Kohlenstoffatome, spezieller 2 bis 20 Kohlenstoffatome, noch spezieller 2 bis 15 Kohlenstoffatome, aufweist,

oder eine Mischung davon steht; vorzugsweise $R_8$ für ein Alkylen steht;
wobei k für eine ganze Zahl größer oder gleich 2, vorzugsweise zwischen 2 und 6, steht.

**10.** Medizinische Vorrichtung nach Anspruch 9, wobei es sich bei der auf Licht ansprechenden Gruppe um eine Allylgruppe handelt und es sich bei dem Polycarbamathydroxyderivat, das auf Licht ansprechende Gruppen trägt, um ein Polyallylcarbamathydroxyderivat der Formel (10)

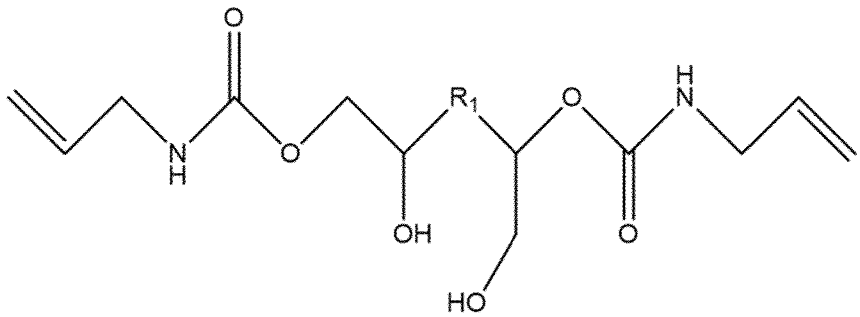

(10)

handelt, wobei $R_1$ für eine lineare oder verzweigte Kohlenwasserstoffkette, die unsubstituiert oder substituiert sein kann, und wobei ein oder mehrere Kohlenwasserstoffgruppen der Kohlenwasserstoffkette durch ein Heteroatom, ein Keton, ein Cycloalkyl, einen Heterocyclus, ein Aryl oder ein Heteroaryl ersetzt sein können, wobei jede dieser Gruppen substituiert oder unsubstituiert sein kann, wobei die Kohlenwasserstoffkette 2 bis 60 Kohlenstoffatome aufweist, steht; vorzugsweise $R_1$ für Polypropylenoxid, Polyethylenoxid, Polybutylenoxid, Polytetrahydrofuran oder eine Mischung davon steht;
und es sich bei dem Thiol um ein Dithiol der Formel (11)

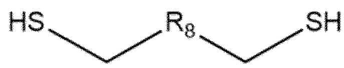

(11)

handelt, wobei $R_8$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, insbesondere 2 bis 60 Kohlenstoffatome, spezieller 2 bis 20 Kohlenstoffatome, noch spezieller 2 bis 15 Kohlenstoffatome, aufweist, oder eine Mischung davon steht; vorzugsweise $R_8$ für ein Alkylen steht.

**11.** Medizinische Vorrichtung nach Anspruch 10, **dadurch gekennzeichnet, dass** es sich bei dem Polyallylcarbamathydroxyderivat um ein Polyallylcarbamathydroxypropylenoxid der Formel (14)

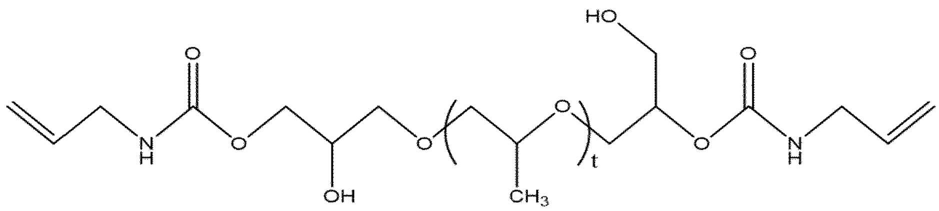

(14)

handelt, wobei $t \geq 1$, vorzugsweise t zwischen 1 und 1000 und ganz besonders bevorzugt zwischen 1 und 20 steht.

**12.** Medizinische Vorrichtung nach einem der Ansprüche 1 bis 11, bei der es sich um einen Katheter oder eine künstliche Herzklappe handelt.

**13.** Verfahren zur Herstellung einer implantierbaren medizinischen Vorrichtung, die Polyhydroxyurethan umfasst, nach einem der Ansprüche 1-11, **dadurch gekennzeichnet, dass** das Verfahren Schritt a) oder Schritt b) umfasst, die jeweils einer thermischen Polymerisation bzw. UV-Polymerisation oder einer Kombination davon entsprechen:

a) Mischen eines Polycyclocarbonats der Formel (2) mit einem Polyamin der Formel (3) und gegebenenfalls einem Katalysator unter Erhalt eines Polyhydroxyurethans auf Basis von Formel (1) durch thermische Polymerisation;

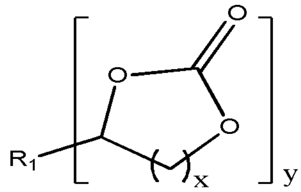
(2)

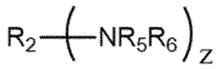
(3)

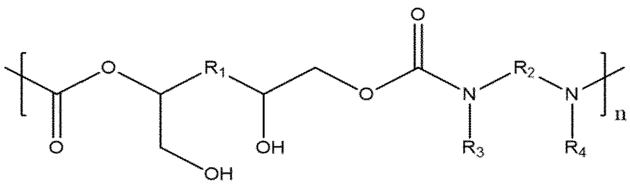
(1)

wobei $R_1$ für eine Kohlenstoffbindung zwischen einem Cyclocarbonatring oder für eine lineare oder verzweigte Kohlenwasserstoffkette, die unsubstituiert oder substituiert sein kann, und wobei ein oder mehrere Kohlenwasserstoffgruppen der Kohlenwasserstoffkette durch ein Heteroatom, ein Keton, ein Cycloalkyl, einen Heterocyclus, ein Aryl oder ein Heteroaryl ersetzt sein können, wobei jede dieser Gruppen substituiert oder unsubstituiert sein kann, wobei die Kohlenwasserstoffkette 2 bis 40 Kohlenstoffatome aufweist, steht; vorzugsweise $R_1$ für Polypropylenoxid, Polyethylenoxid, Polybutylenoxid, Polytetrahydrofuran oder eine Mischung davon steht;

wobei $R_2$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, vorzugsweise 2 bis 60 Kohlenstoffatome, weiter bevorzugt 2 bis 20 Kohlenstoffatome, noch weiter bevorzugt 2 bis 15 Kohlenstoffatome, aufweist, steht;

wobei $R_5$ und $R_6$ gleich oder verschieden sind und für H, eine gerade oder verzweigte gesättigte Kohlenwasserstoffkette $C_{1-6}$-Alkyl mit 1 bis 6 Kohlenstoffatomen stehen; vorzugsweise $R_5$ und $R_6$ für Wasserstoff stehen; mit der Ausnahme, dass dann, wenn $R_5$ und/oder $R_6$ für Wasserstoff stehen, $R_2$ nicht für

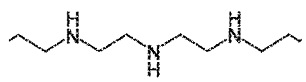

steht;

und mit der Ausnahme, dass dann, wenn $R_5$ und/oder $R_6$ für Wasserstoff stehen, $R_1$ nicht für

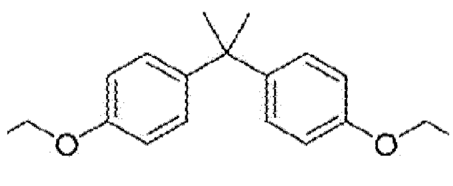
,
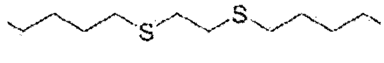

oder

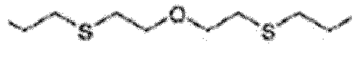

steht;

wobei $R_3$ und $R_4$ gleich oder verschieden sind und für H, eine gerade oder verzweigte gesättigte Kohlenwasserstoffkette $C_{1-6}$-Alkyl mit 1 bis 6 Kohlenstoffatomen; $C_{1-6}$-Alkoxyalkyl mit 1 bis 6 Kohlenstoffatomen stehen;

und wobei n für eine ganze Zahl ≥ 1 steht, vorzugsweise n zwischen 1 und 100, weiter bevorzugt zwischen 1 und 20, steht;

mit der Ausnahme, dass dann, wenn $R_3$ und/oder $R_4$ für ein Wasserstoffatom stehen, $R_1$ nicht für

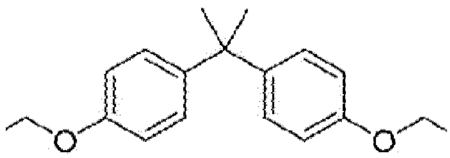

,

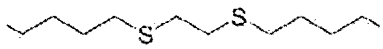

oder

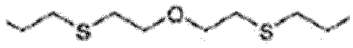

steht;

b) Mischen eines Polyvinylcarbamathydroxyderivats, das auf Licht ansprechende Gruppen trägt, vorzugsweise der Formel (10), mit einem Thiol der Formel (16), vorzugsweise der Formel (11), und einem Photoinitiator unter Erhalt eines Polyhydroxyurethans auf Basis von Formel (9) durch UV-Polymerisation;

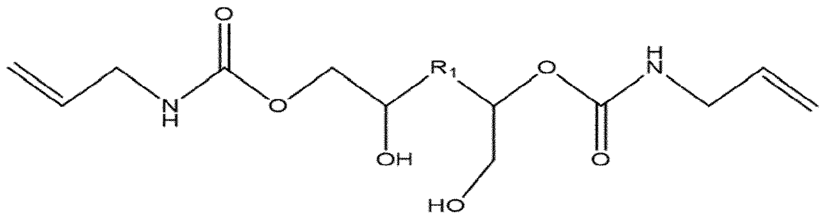

(10)

$R_8$-$(SH)_k$ (16);

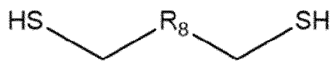

(11)

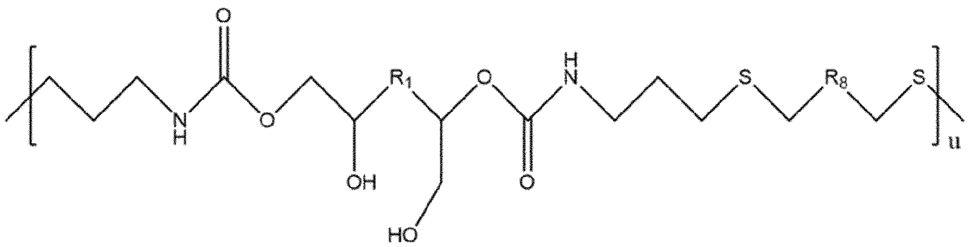

(9)

wobei $R_1$ für eine lineare oder verzweigte Kohlenwasserstoffkette, die unsubstituiert oder substituiert sein kann, und wobei ein oder mehrere Kohlenwasserstoffgruppen der Kohlenwasserstoffkette durch ein Heteroatom, ein Keton, ein Cycloalkyl, einen Heterocyclus, ein Aryl oder ein Heteroaryl ersetzt sein können, wobei jede dieser Gruppen substituiert oder unsubstituiert sein kann, wobei die Kohlenwasserstoffkette 2 bis 60 Kohlenstoffatome aufweist, steht; vorzugsweise $R_1$ für Polypropylenoxid, Polyethylenoxid, Polybutylenoxid, Polytetrahydrofuran oder eine Mischung davon steht;

wobei $R_8$ für ein Aryl oder ein Heteroaryl, wobei jede dieser Gruppen unsubstituiert oder substituiert sein

kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, insbesondere 2 bis 60 Kohlenstoffatome, spezieller 2 bis 20 Kohlenstoffatome, noch spezieller 2 bis 15 Kohlenstoffatome, aufweist, oder eine Mischung davon steht; vorzugsweise $R_8$ für ein Alkylen steht; und wobei k für eine ganze Zahl ≥ 2, vorzugsweise zwischen 2 und 6, steht;
wobei u für eine ganze Zahl ≥ 1, vorzugsweise zwischen 1 und 10, weiter bevorzugt zwischen 1 und 5, steht.

**14.** Verfahren zum Beschichten einer implantierbaren medizinischen Vorrichtung, die Polyhydroxyurethan umfasst, nach einem der Ansprüche 1-11, das Verfaren das Aufbringen auf eine Oberfläche der medizinischen Vorrichtung von mindestens eine Schicht aus Polyhydroxyurethan auf Basis von Formel (1) und/oder Formel (9) umfasst:

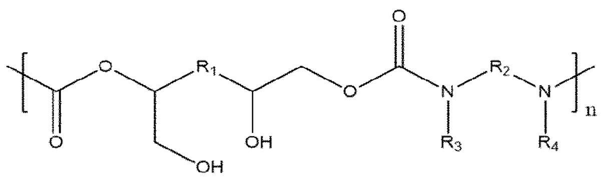

(1)

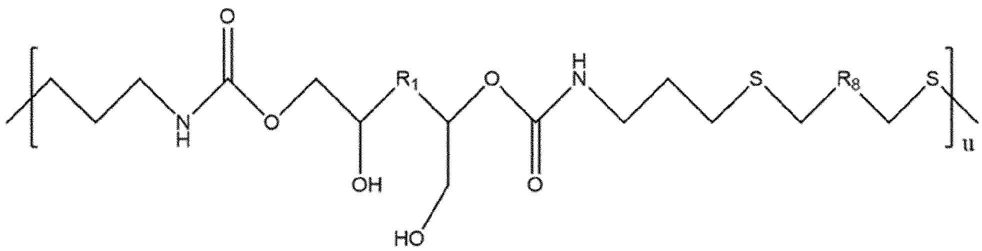

(9)

wobei $R_1$ für eine Kohlenstoffbindung zwischen einem Cyclocarbonatring oder für eine lineare oder verzweigte Kohlenwasserstoffkette, die unsubstituiert oder substituiert sein kann, und wobei ein oder mehrere Kohlenwasserstoffgruppen der Kohlenwasserstoffkette durch ein Heteroatom, ein Keton, ein Cycloalkyl, einen Heterocyclus, ein Aryl oder ein Heteroaryl ersetzt sein können, wobei jede dieser Gruppen substituiert oder unsubstituiert sein kann, wobei die Kohlenwasserstoffkette 2 bis 40 Kohlenstoffatome aufweist, steht; vorzugsweise $R_1$ für Polypropylenoxid, Polyethylenoxid, Polybutylenoxid, Polytetrahydrofuran oder eine Mischung davon steht;
wobei $R_2$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, vorzugsweise 2 bis 60 Kohlenstoffatome, weiter bevorzugt 2 bis 20 Kohlenstoffatome, noch weiter bevorzugt 2 bis 15 Kohlenstoffatome, aufweist, steht;
wobei $R_3$ und $R_4$ gleich oder verschieden sind und für H, eine gerade oder verzweigte gesättigte Kohlenwasserstoffkette $C_{1-6}$-Alkyl mit 1 bis 6 Kohlenstoffatomen; $C_{1-6}$-Alkoxyalkyl mit 1 bis 6 Kohlenstoffatomen stehen; mit der Ausnahme, dass dann, wenn $R_3$ und/oder $R_4$ für ein Wasserstoffatom stehen, $R_1$ nicht für

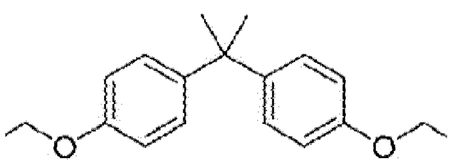

,

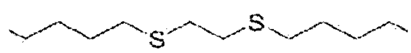

oder

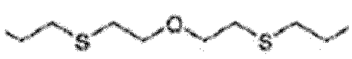

steht,
und mit der Ausnahme, dass dann, wenn $R_3$ und/oder $R_4$ für ein Wasserstoffatom stehen, $R_2$ nicht für

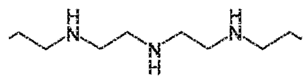

steht;
wobei $R_8$ für ein Aryl oder ein Heteroaryl, wobei jede dieser Gruppen unsubstituiert oder substituiert sein kann, oder eine lineare oder verzweigte Kohlenwasserstoffkette, die unsubstituiert oder substituiert sein kann, und wobei eine oder mehrere Kohlenwasserstoffgruppen der Kohlenwasserstoffkette durch ein Heteroatom, ein Cycloalkyl oder einen Heterocyclus ersetzt sein können, wobei jede dieser Gruppen unsubstituiert oder substituiert sein kann, wobei die Kohlenwasserstoffkette mindestens 2 Kohlenstoffatome, insbesondere 2 bis 60 Kohlenstoffatome, spezieller 2 bis 20 Kohlenstoffatome, noch spezieller 2 bis 15 Kohlenstoffatome, aufweist, oder eine Mischung davon steht; vorzugsweise $R_8$ für ein Alkylen steht;
wobei u für eine ganze Zahl ≥ 1, vorzugsweise zwischen 1 und 10, weiter bevorzugt zwischen 1 und 5, steht;
und wobei n für eine ganze Zahl ≥ 1 steht, vorzugsweise n zwischen 1 und 100, weiter bevorzugt zwischen 1 und 20, steht.

**15.** Medizinische Vorrichtung nach einem der Ansprüche 1 bis 12, die ferner ein bioaktives Molekül, vorzugsweise ein antibakterielles Mittel oder ein Antithrombotikum, umfasst.

**Revendications**

**1.** Dispositif médical implantable comprenant un ou plusieurs polyhydroxyuréthanes (PHU) 1 **caractérisé en ce que** le ou les polyhydroxyuréthanes (PHU) sont basés sur la formule (1) ou sur la formule (9)

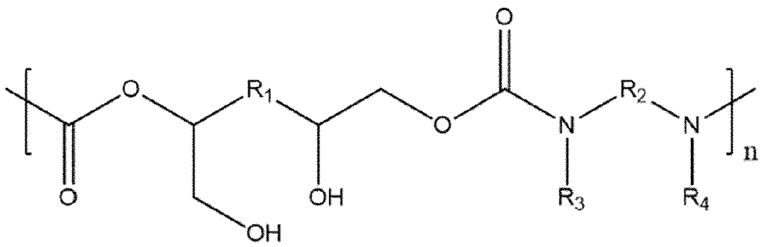

(1)

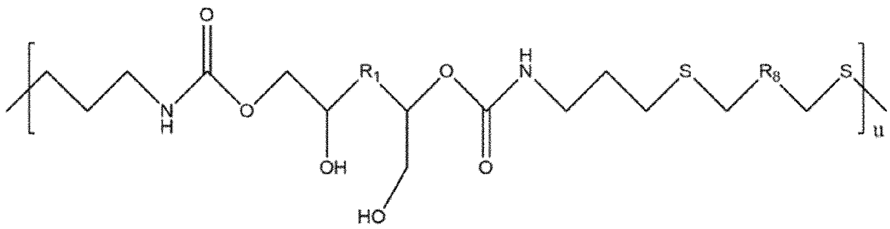

(9)

$R_1$ étant une chaîne hydrocarbonée linéaire ou ramifiée, qui peut être non substituée ou substituée et un ou plusieurs groupes hydrocarbonés de ladite chaîne hydrocarbonée pouvant être remplacés par un hétéroatome, une cétone, un cycloalkyle, un hétérocycle, un aryle ou un hétéroaryle, chacun desquels pouvant être substitué ou non substitué, ladite chaîne hydrocarbonée ayant de 2 à 60 atomes de carbone ;
$R_2$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, préférablement de 2 à 60 atomes de carbone, plus préférablement de 2 à 20 atomes de carbone, encore plus préférablement de 2 à 15 atomes de carbone, ou un mélange correspondant ;
préférablement $R_2$ étant un alkylène ;

$R_3$ et $R_4$ étant identiques ou différents et représentant H, une chaîne hydrocarbonée saturée linéaire ou ramifiée $C_{1-6}$-alkyle ayant 1 à 6 atomes de carbone ; $C_{1-6}$-alcoxy alkyle ayant 1 à 6 atomes de carbone
et n étant un entier ≥ 1, préférablement n étant compris entre 1 et 100, plus préférablement compris entre 1 et 20 ;
à l'exception que si $R_3$ et/ou $R_4$ représentent un atome d'hydrogène, alors $R_1$ n'est pas

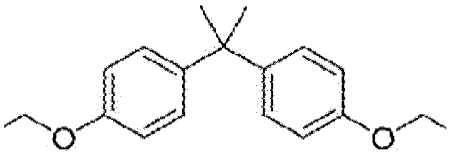
,
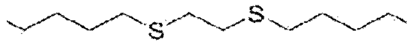
,

ou

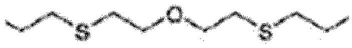
;

et à l'exception que si $R_3$ et/ou $R_4$ représentent un atome d'hydrogène, alors $R_2$ n'est pas

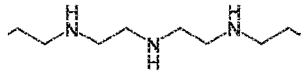
;

$R_8$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, en particulier de 2 à 60 atomes de carbone, plus particulièrement de 2 à 20 atomes de carbone, encore plus particulièrement de 2 à 15 atomes de carbone, ou un mélange correspondant ; préférablement $R_8$ étant un alkylène ; et
u étant un entier ≥ 1, préférablement compris entre 1 et 10, plus préférablement compris entre 1 et 5.

2. Dispositif médical selon la revendication 1, $R_1$ étant poly(oxyde de propylène), poly(oxyde d'éthylène), poly(oxyde de butylène), poly(tétrahydrofurane), ou un mélange correspondant.

3. Dispositif médical selon la revendication 1, comprenant un polyhydroxyuréthane (PHU) basé sur la formule (1), $R_1$ étant poly(oxyde de propylène), poly(oxyde d'éthylène), poly(oxyde de butylène), poly(tétrahydrofurane), ou un mélange correspondant et $R_2$ étant un alkylène.

4. Dispositif médical selon la revendication 1, le polyhydroxyuréthane basé sur la formule (1) étant obtenu par polyaddition thermique d'un polycyclocarbonate de formule (2)

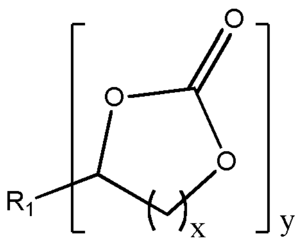
(2)

$R_1$ étant une liaison carbonée entre un cycle carbonate cyclique ou étant une chaîne hydrocarbonée linéaire ou ramifiée, qui peut être non substituée ou substituée et un ou plusieurs groupes hydrocarbonés de ladite chaîne hydrocarbonée pouvant être remplacés par un hétéroatome, une cétone, un cycloalkyle, un hétérocycle, un aryle ou un hétéroaryle, chacun desquels pouvant être substitué ou non substitué, ladite chaîne hydrocarbonée ayant de 2 à 40 atomes de carbone ; préférablement $R_1$ étant poly(oxyde de propylène), poly(oxyde d'éthylène), poly(oxyde de butylène), poly(tétrahydrofurane) ou un mélange correspondant ;

x étant un entier compris entre 1 et 4 ;
y étant un entier supérieur ou égal à 2 ;
avec une polyamine de formule (3)

$$R_2\left(NR_5R_6\right)_z \quad (3)$$

z étant un entier compris entre 2 et 6 ;
$R_2$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, en particulier de 2 à 60 atomes de carbone, plus particulièrement de 2 à 20 atomes de carbone, encore plus particulièrement de 2 à 15 atomes de carbone ; préférablement $R_2$ étant un alkylène ;
$R_5$ et $R_6$ étant identiques ou différents et représentant H, une chaîne hydrocarbonée saturée linéaire ou ramifiée $C_{1-6}$-alkyle ayant 1 à 6 atomes de carbone ; préférablement $R_5$ et $R_6$ étant hydrogène ;
à l'exception que si $R_5$ et/ou $R_6$ sont hydrogène, alors $R_2$
n'est pas

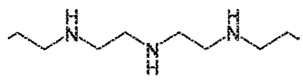 ;

à l'exception que si $R_5$ et/ou $R_6$ sont des atomes d'hydrogène, alors $R_1$ n'est pas

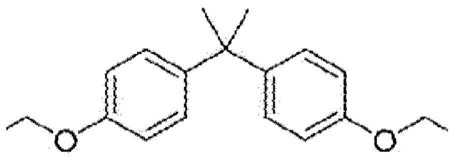 , 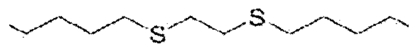 ,

ou

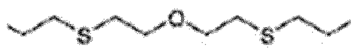 .

**5.** Dispositif médical selon la revendication 4, **caractérisé en ce que** le polycyclocarbonate de formule (2) est un poly(propylène glycol)-bis-cyclocarbonate (PPGbisCC) de formule (5), m étant un entier ≥ 1, préférablement compris entre 1 et 100, le plus préférablement compris entre 1 et 10 ;

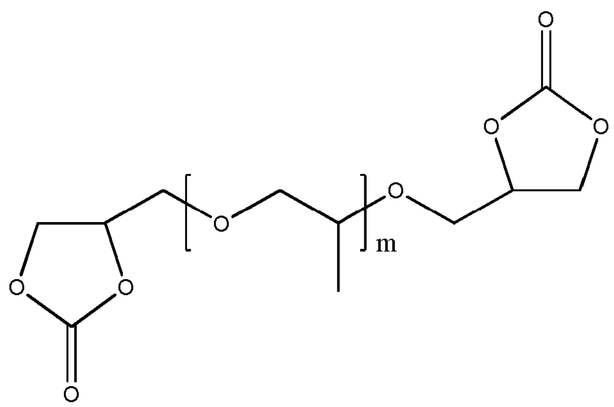

(5)

et la polyamine étant la 4-4'méthylène-bis-(cyclohexylamine) (MBCHA) de formule (4)

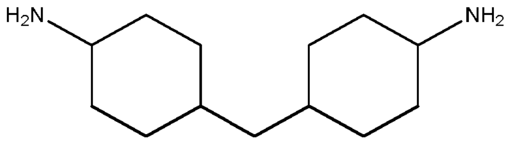

(4) .

6. Dispositif médical selon la revendication 5, **caractérisé en ce que** la polyamine est la 4,4'méthylène-bis-(cyclohexylamine) de formule (4) en combinaison avec la tris(2-aminoéthyl)amine (TAEA) de formule (6).

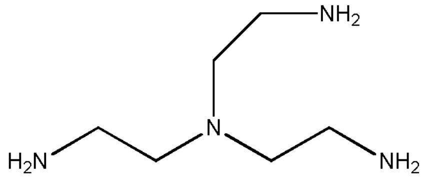

(6)

7. Dispositif médical selon la revendication 4, **caractérisé en ce que** le polycyclocarbonate est un polytétrahydrofuranebiscyclocarbonate de formule (8), p étant un entier ≥ 1, préférablement compris entre 1 et 100, le plus préférablement compris entre 1 et 10 ;

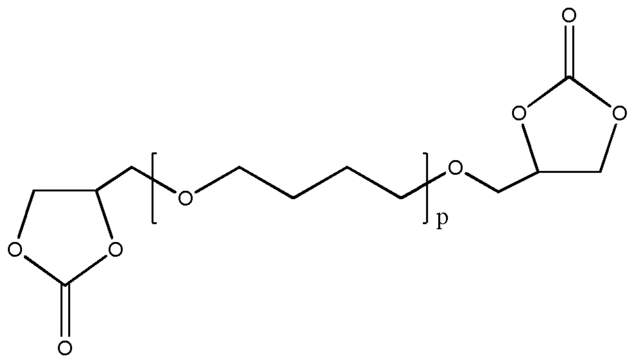

(8)

et la polyamine étant la 4-4'méthylène-bis-(cyclohexylamine) (MBCHA) de formule (4)

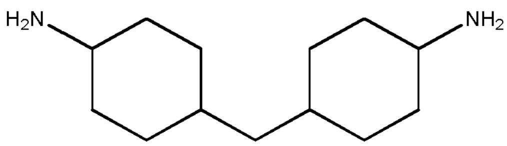

(4) .

8. Dispositif médical selon la revendication 7, **caractérisé en ce que** la polyamine est la 4-4'méthylène-bis-(cyclohexylamine) de formule (4) en combinaison avec la tris(2-aminoéthyl)amine (TAEA) de formule (6)

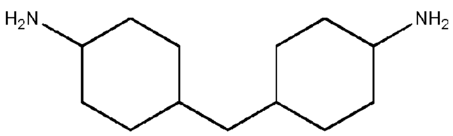

(4)

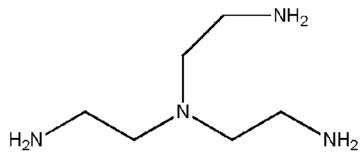

(6) .

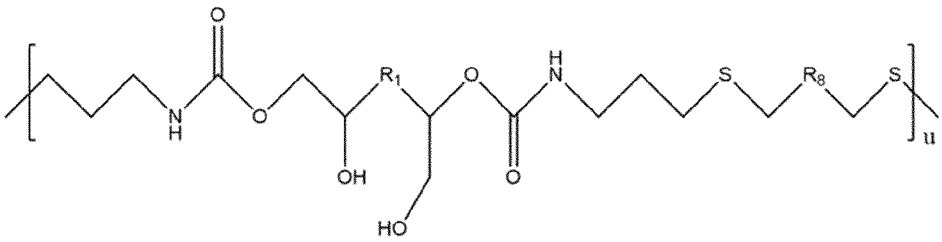

(9).

**9.** Dispositif médical selon la revendication 1, le polyhydroxyuréthane (PHU) basé sur la formule (9) étant obtenu par addition thermique du polycarbonate de formule (2)

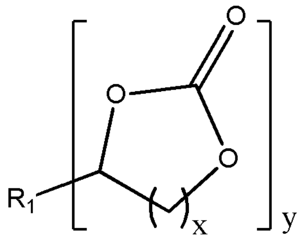

(2)

$R_1$ étant une liaison carbonée entre un cycle carbonate cyclique ou étant une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, une cétone, un cycloalkyle, un hétérocycle, un aryle ou un hétéroaryle, chacun desquels pouvant être substitué ou non substitué, ladite chaîne hydrocarbonée ayant de 2 à 60 atomes de carbone ;
y étant un entier égal ou supérieur à 2 ;
X étant un entier compris entre 1 et 4 ;
avec une polyamine de formule (15) pour obtenir un dérivé polycarbamatehydroxy portant des groupes photosensibles

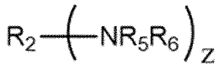

(15) ;

$R_2$ étant une chaîne insaturée hydrocarboné linéaire ou ramifiée comportant 2 à 40 atomes de carbone, ladite chaîne insaturée hydrocarbonée linéaire ou ramifiée portant au moins un groupe photosensible ;
$R_5$ étant hydrogène et $R_6$ étant hydrogène, une chaîne hydrocarbonée saturée linéaire ou ramifiée $C_{1-6}$ alkyle ayant 1 à 6 atomes de carbone, préférablement $R_6$ étant hydrogène ; et
z étant un entier $\geq 1$, préférablement compris entre 1 et 6 ;
et le dérivé polycarbamatehydroxy portant des groupes photosensibles
étant en outre mis à réagir par polyaddition UV avec un thiol de formule (16), préférablement un dithiol de formule (11) et un photoinitiateur ;

$R_8\text{-}(SH)_k$ (16) ;

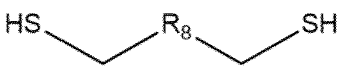

(11)

$R_8$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, en particulier de 2 à 60 atomes de carbone, plus particulièrement de 2 à 20 atomes de carbone, encore plus particulièrement de 2 à 15 atomes de carbone, ou un mélange correspondant ; préférablement $R_8$ étant un alkylène ;

k étant un entier supérieur ou égal à 2, préférablement compris entre 2 et 6.

**10.** Dispositif médical selon la revendication 9, le groupe photosensible étant un groupe allyle et le dérivé polycarbamatehydroxy portant des groupes photosensibles étant un dérivé polyallylcarbamatehydroxy de formule (10)

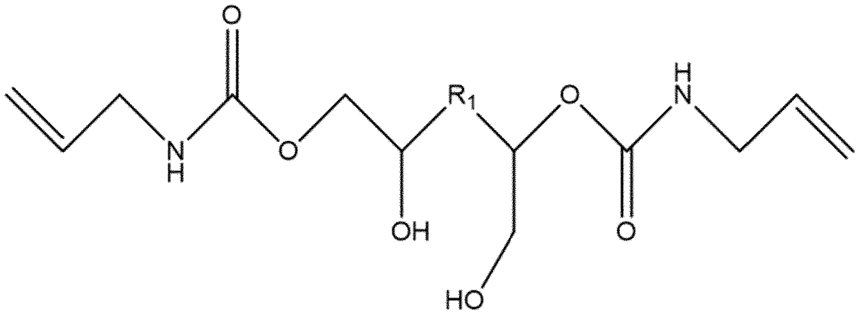

(10)

$R_1$ étant une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, une cétone, un cycloalkyle, un hétérocycle, un aryle ou un hétéroaryle, chacun desquels pouvant être substitué ou non substitué, ladite chaîne hydrocarbonée ayant de 2 à 60 atomes de carbone ; préférablement $R_1$ étant poly(oxyde de propylène), poly(oxyde d'éthylène), poly(oxyde de butylène), poly(tétrahydrofurane), ou un mélange correspondant ;
et le thiol étant un dithiol de formule (11)

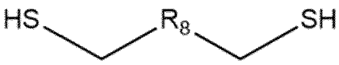

(11)

$R_8$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, en particulier de 2 à 60 atomes de carbone, plus particulièrement de 2 à 20 atomes de carbone, encore plus particulièrement de 2 à 15 atomes de carbone, ou un mélange correspondant ; préférablement $R_8$ étant un alkylène.

**11.** Dispositif médical selon la revendication 10 **caractérisé en ce que** le dérivé polyallylcarbamatehydroxy est un polyallylcarbamatehydroxyoxyde de propylène de formule (14)

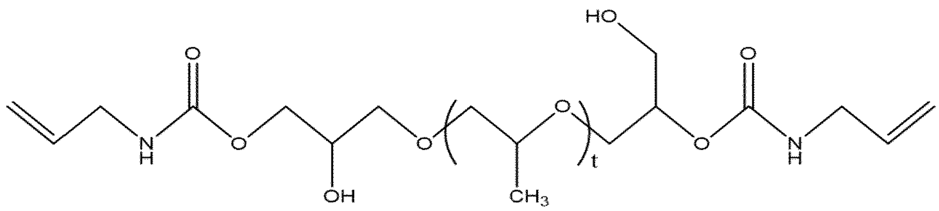

(14)

$t \geq 1$, préférablement t étant compris entre 1 et 1 000 et le plus préférablement compris entre 1 et 20.

**12.** Dispositif médical selon l'une quelconque des revendications 1 à 11, qui est un cathéter ou une valve cardiaque prothétique.

**13.** Procédé de fabrication d'un dispositif médical implantable comprenant un polyhydroxyuréthane selon l'une quelconque des revendications 1-11 ; **caractérisé en ce que** le procédé comprend l'étape (a) ou (b) correspondant respectivement à une polymérisation thermique ou par des UV ou une combinaison des deux :

a) mélange d'un polycyclocarbonate de formule (2) avec une polyamine de formule (3) et éventuellement un

catalyseur pour obtenir, par polymérisation thermique, un polyhydroxyuréthane basé sur la formule (1) ;

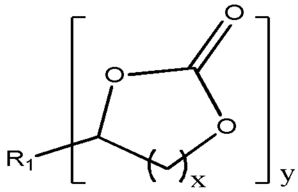 (2) 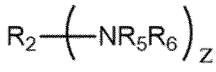 (3)

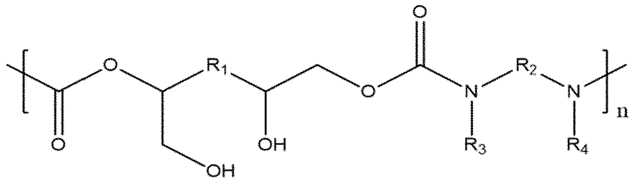

(1)

$R_1$ étant une liaison carbonée entre un cycle carbonate cyclique ou étant une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, une cétone, un cycloalkyle, un hétérocycle, un aryle ou un hétéroaryle, chacun desquels pouvant être substitué ou non substitué, ladite chaîne hydrocarbonée ayant de 2 à 40 atomes de carbone ; préférablement $R_1$ étant poly(oxyde de propylène), poly(oxyde d'éthylène), poly(oxyde de butylène), poly(tétrahydrofurane) ou un mélange correspondant ;
$R_2$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, préférablement de 2 à 60 atomes de carbone, plus préférablement de 2 à 20 atomes de carbone, encore plus préférablement de 2 à 15 atomes de carbone ;
$R_5$ et $R_6$ étant identiques ou différents et représentant H, une chaîne hydrocarbonée saturée linéaire ou ramifiée $C_{1-6}$-alkyle ayant 1 à 6 atomes de carbone ; préférablement $R_5$ et $R_6$ étant hydrogène, à l'exception que si $R_5$ et/ou
$R_6$ sont hydrogène alors $R_2$ n'est pas

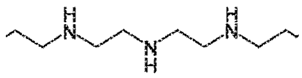 ;

et à l'exception que si $R_5$ et/ou $R_6$ sont hydrogène alors $R_1$ n'est pas

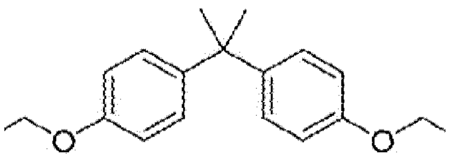, 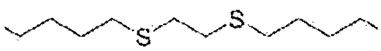,

ou

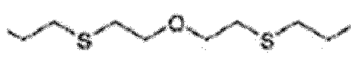 ;

$R_3$ et $R_4$ étant identiques ou différents et représentant H, une chaîne hydrocarbonée saturée linéaire ou ramifiée $C_{1-6}$-alkyle ayant 1 à 6 atomes de carbone ; $C_{1-6}$-alcoxy alkyle ayant 1 à 6 atomes de carbone ; et n étant un entier ≥ 1, préférablement n étant compris entre 1 et 100, plus préférablement compris entre 1 et 20 ;
à l'exception que si $R_3$ et/ou $R_4$ représentent un atome d'hydrogène, $R_1$ n'est pas

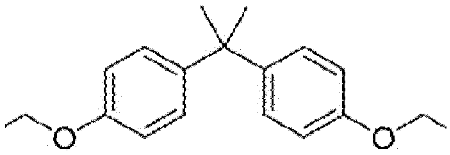 , 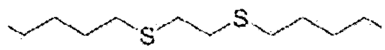 ,

ou

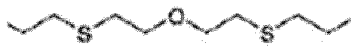 ;

b) mélange d'un dérivé polyvinylcarbamatehydroxy portant des groupes photosensibles, préférablement de formule (10) avec un thiol de formule (16), préférablement de formule (11) et un photoinitiateur pour obtenir, par polymérisation par des UV, un polyhydroxyuréthane basé sur la formule (9) ;

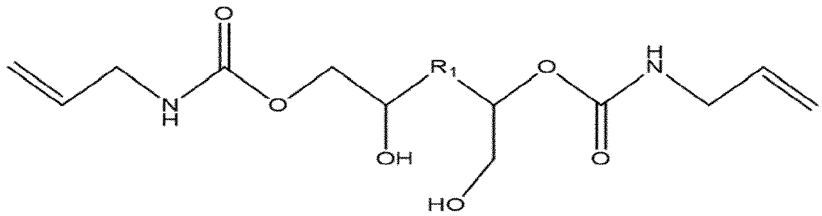

(10)

$R_8\text{-}(SH)_k$ (16) ;

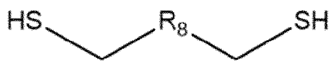

(11)

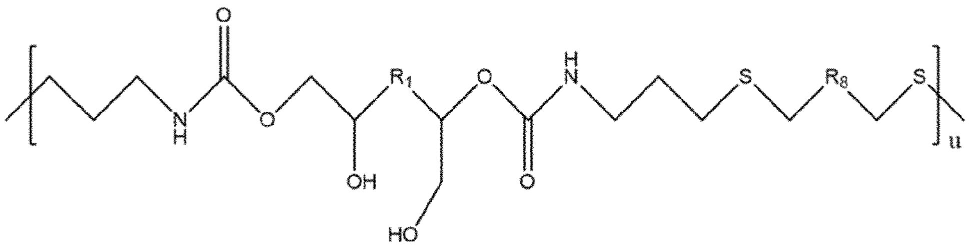

(9)

$R_1$ étant une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, une cétone, un cycloalkyle, un hétérocycle, un aryle ou un hétéroaryle, chacun desquels pouvant être substitué ou non substitué, ladite chaîne hydrocarbonée ayant de 2 à 60 atomes de carbone ; préférablement $R_1$ étant poly(oxyde de propylène), poly(oxyde d'éthylène), poly(oxyde de butylène), poly(tétrahydrofurane), ou un mélange correspondant ;

$R_8$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, en particulier de 2 à 60 atomes de carbone, plus particulièrement de 2 à 20 atomes de carbone, encore plus particulièrement de 2 à 15 atomes de carbone, ou un mélange correspondant ; préférablement $R_8$ étant un alkylène ; et

k étant un entier ≥ 2, préférablement compris entre 2 et 6 ;

u étant un entier ≥ 1, préférablement compris entre 1 et 10, plus préférablement compris entre 1 et 5.

**14.** Procédé de revêtement d'un dispositif médical implantable comprenant un polyhydroxyuréthane selon l'une quelconque des revendications 1-11 ; le procédé comprenant un dépôt sur une surface du dispositif médical d'au moins une couche de polyhydroxyuréthane basé sur la formule (1) et/ou sur la formule (9)

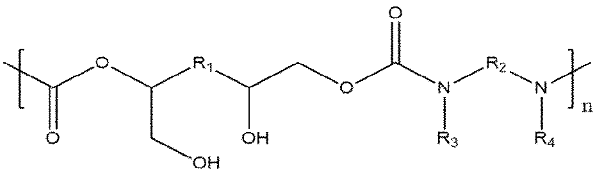

(1)

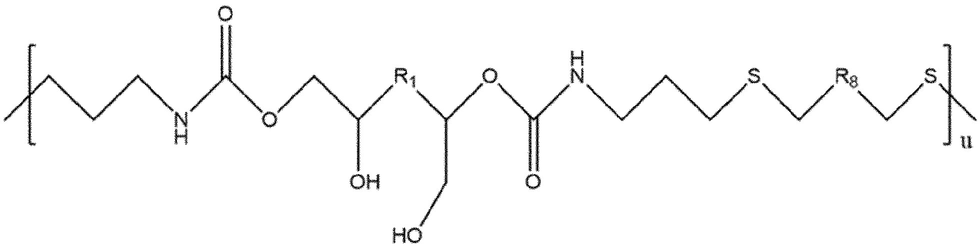

(9)

$R_1$ étant une liaison carbonée entre un cycle carbonate cyclique ou étant une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, une cétone, un cycloalkyle, un hétérocycle, un aryle ou un hétéroaryle, chacun desquels pouvant être substitué ou non substitué, ladite chaîne hydrocarbonée ayant de 2 à 40 atomes de carbone ; préférablement $R_1$ étant poly(oxyde de propylène), poly(oxyde d'éthylène), poly(oxyde de butylène), poly(tétrahydrofurane) ou un mélange correspondant ;

$R_2$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou un hétérocycle, chacun desquels pouvant être non substitué ou substitué, ladite chaîne hydrocarbonée ayant au moins 2 atomes de carbone, préférablement de 2 à 60 atomes de carbone, plus préférablement de 2 à 20 atomes de carbone, encore plus préférablement de 2 à 15 atomes de carbone ;

$R_3$ et $R_4$ étant identiques ou différents et représentant H, une chaîne hydrocarbonée saturée linéaire ou ramifiée $C_{1-6}$-alkyle ayant 1 à 6 atomes de carbone ; $C_{1-6}$-alcoxy alkyle ayant 1 à 6 atomes de carbone ;

à l'exception que si $R_3$ et/ou $R_4$ représentent un atome d'hydrogène, alors $R_1$ n'est pas

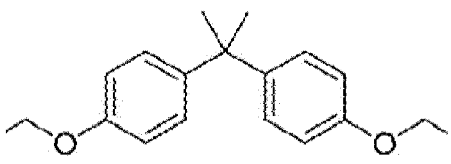

,

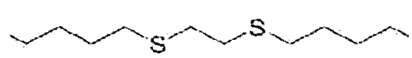

,

ou

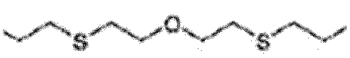

,

et à l'exception que si R3 et/ou $R_4$ représentent un atome d'hydrogène, alors $R_2$ n'est pas

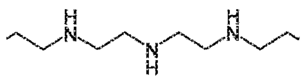

;

$R_8$ étant un aryle ou un hétéroaryle, chacun desquels pouvant être non substitué ou substitué, ou une chaîne hydrocarbonée linéaire ou ramifiée, qui peut être non substituée ou substituée, et un ou plusieurs groupes hydrocarbonés de ladite chaîne hydrocarbonée pouvant être remplacés par un hétéroatome, un cycloalkyle ou

un hétérocycle, chacun desquels pouvant être non substitué ou substitué, ladite chaîne hydrocarbonée ayant au moins 2 atomes de carbone, en particulier de 2 à 60 atomes de carbone, plus particulièrement de 2 à 20 atomes de carbone, encore plus particulièrement de 2 à 15 atomes de carbone, ou un mélange correspondant ; préférablement $R_8$ étant un alkylène ;
u étant un entier $\geq 1$, préférablement compris entre 1 et 10, plus préférablement compris entre 1 et 5 ;
et n étant un entier $\geq 1$, préférablement n étant compris entre 1 et 100, plus préférablement compris entre 1 et 20.

**15.** Dispositif médical selon l'une quelconque des revendications 1 à 12 qui comprend en outre une molécule bioactive, préférablement un agent antibactérien ou un agent antithrombotique.

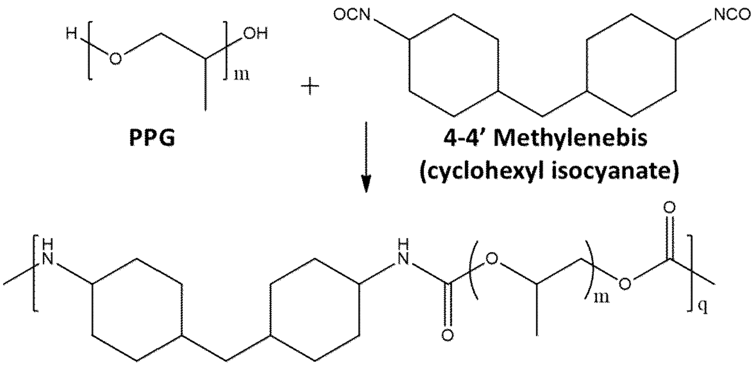


Polypropylene glycol based PU

**Figure 1**

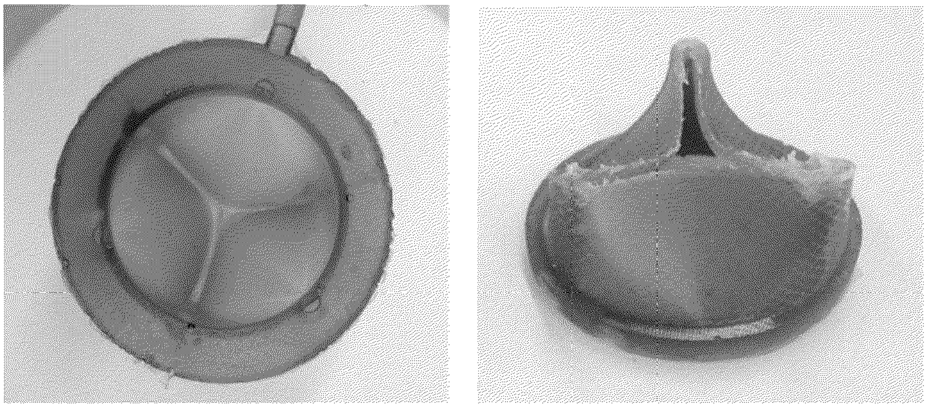

**Figure 2**

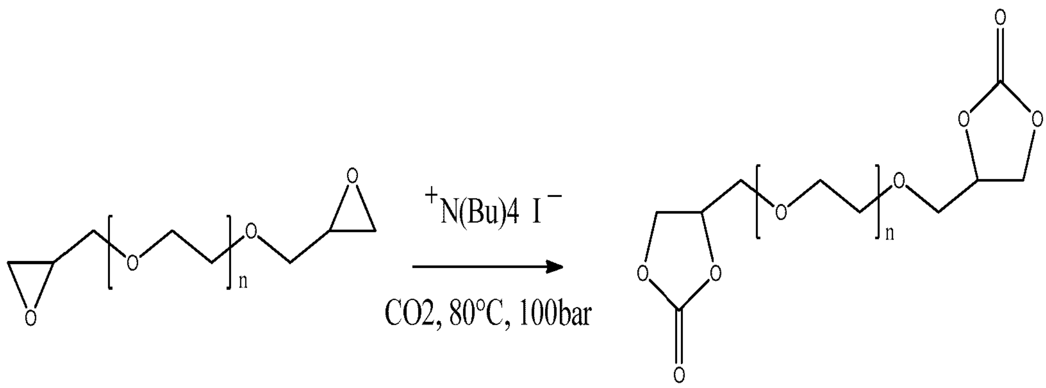


**Figure 3a**

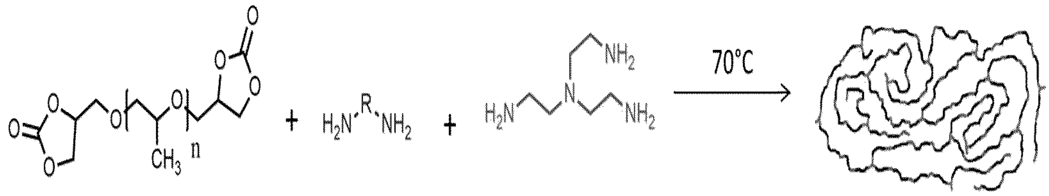


Figure 3b

Step 1

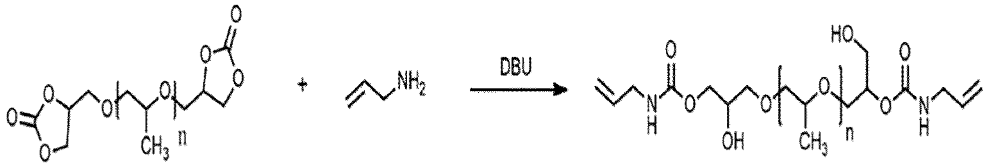


Step 2

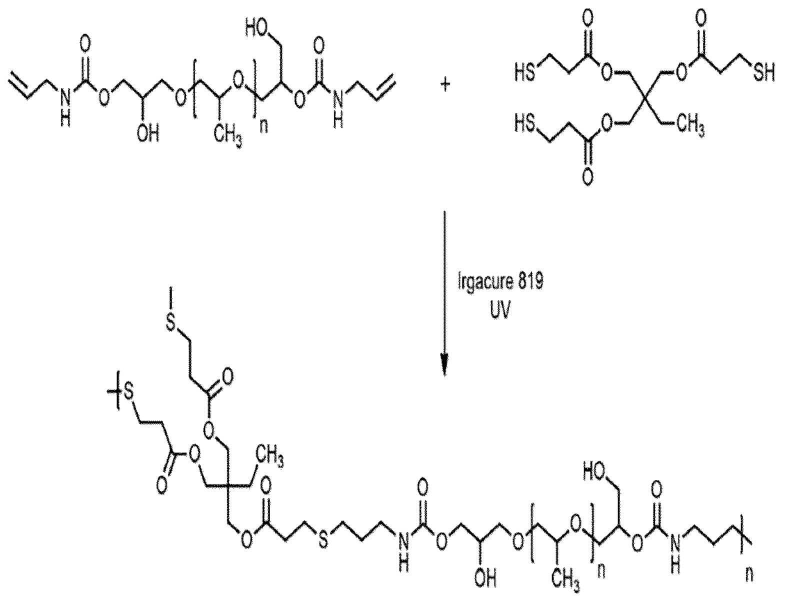


Figure 4

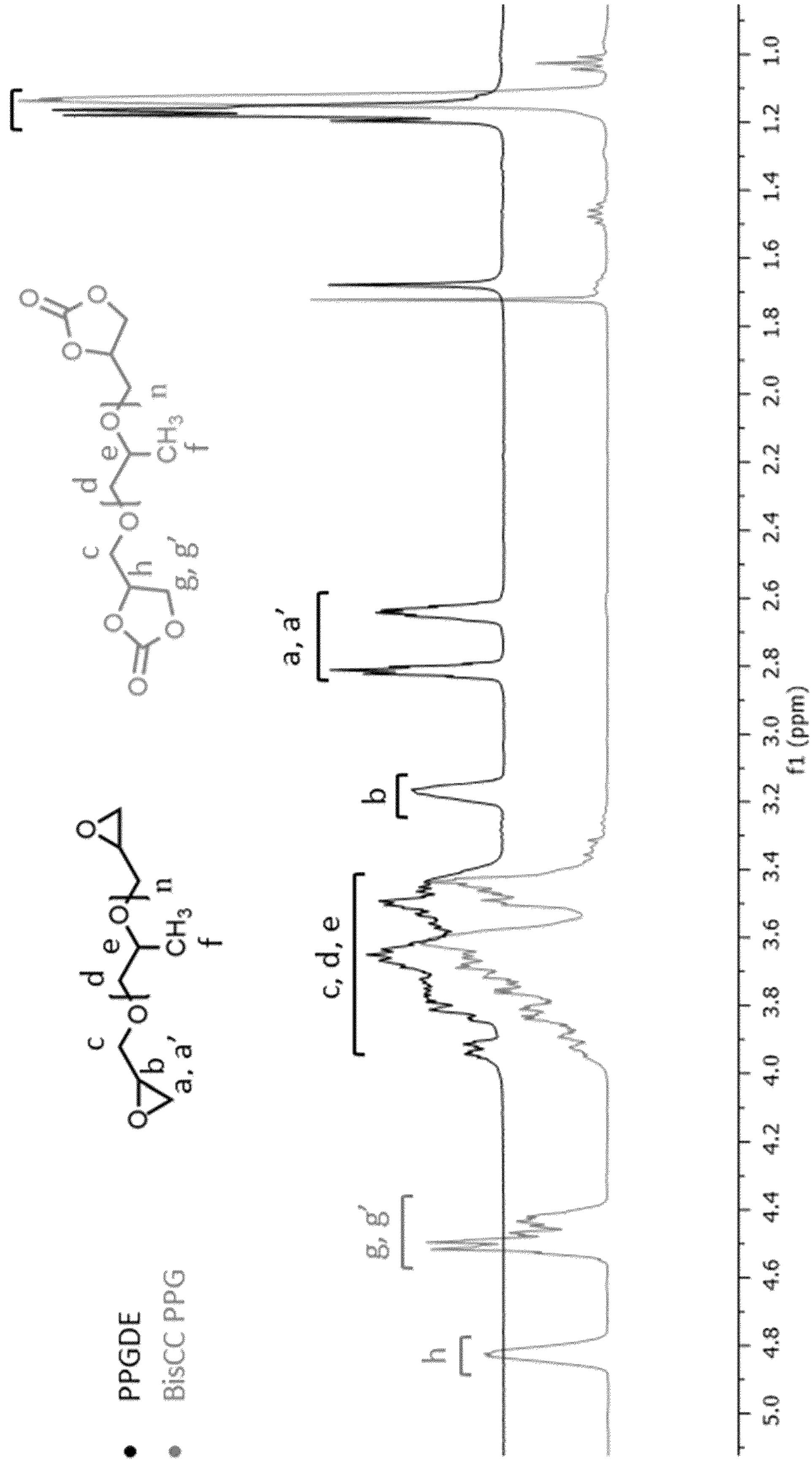
PPGDE
BisCC PPG
f
a, a'
b
c, d, e
g, g'
h
1.0
1.2
1.4
1.6
1.8
2.0
2.2
2.4
2.6
2.8
3.0
3.2
3.4
3.6
3.8
4.0
4.2
4.4
4.6
4.8
5.0
f1 (ppm)

Figure 5a

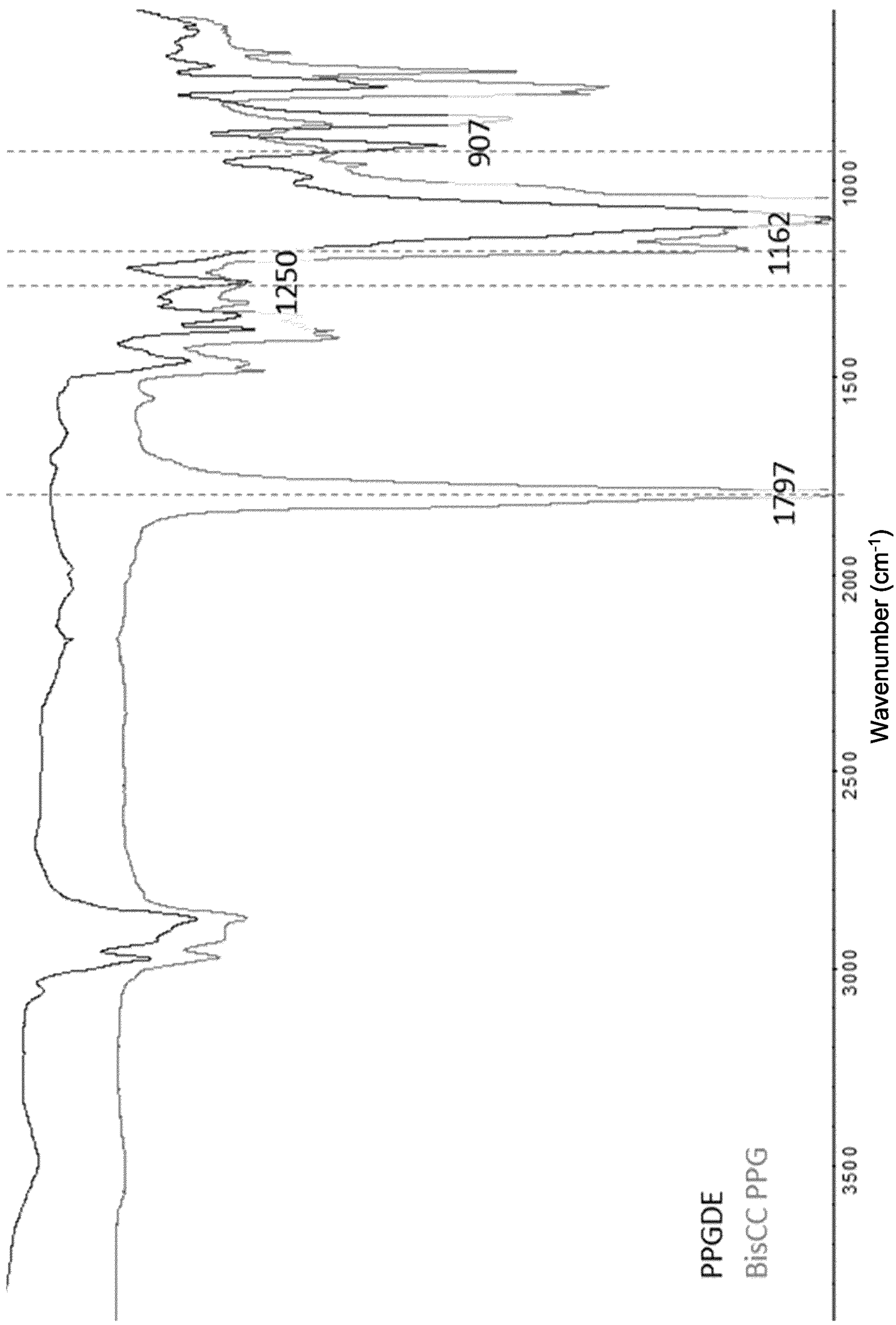
PPGDE
BisCC PPG
907
1162
1250
1797
1000
1500
2000
2500
3000
3500
Wavenumber (cm-1)

**Figure 5b**

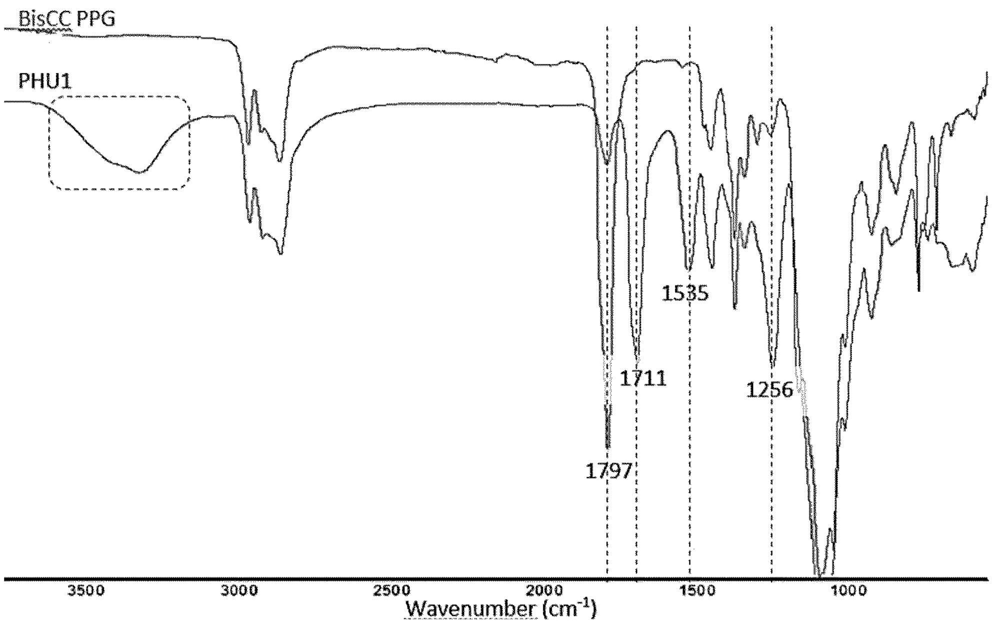
BisCC PPG
PHU1
1535
1711
1256
1797
3500
3000
2500
2000
1500
1000
Wavenumber (cm-1)

Figure 5c

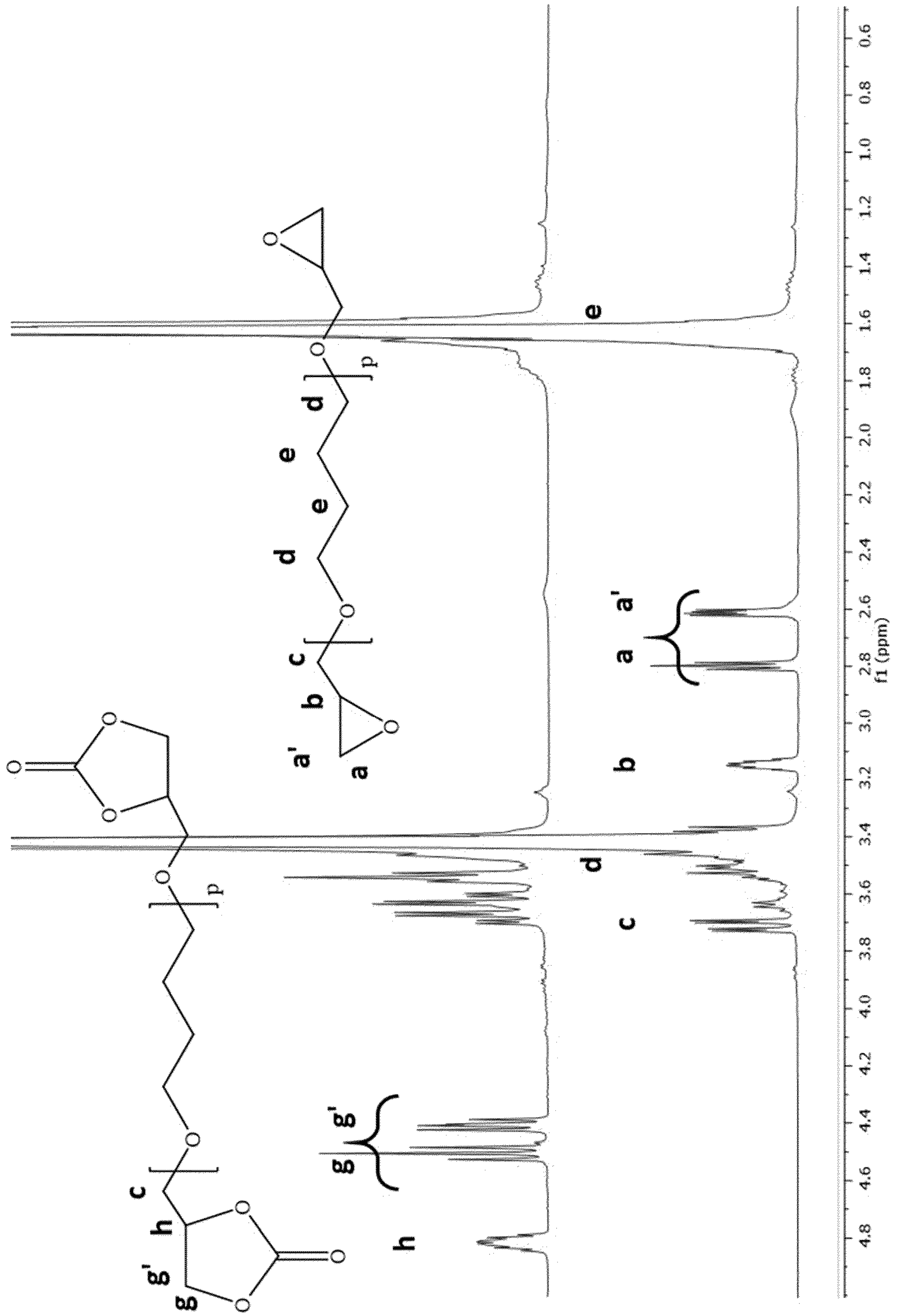


Figure 5d

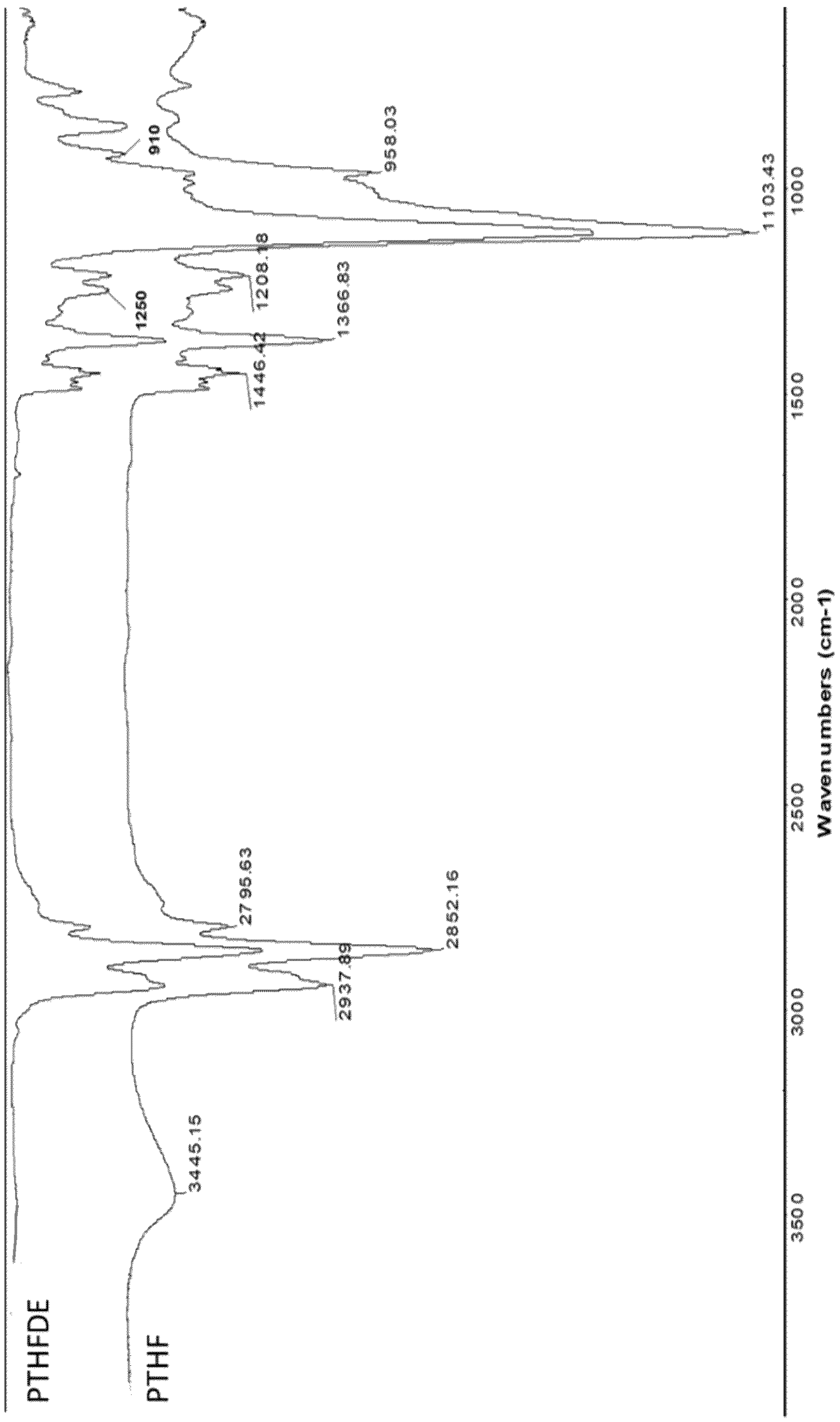
PTHFDE
PTHF
3445.15
2937.89
2852.16
2795.63
1446.42
1366.83
1250
1208.18
1103.43
958.03
910
3500
3000
2500
2000
1500
1000
Wavenumbers (cm-1)

Figure 5e

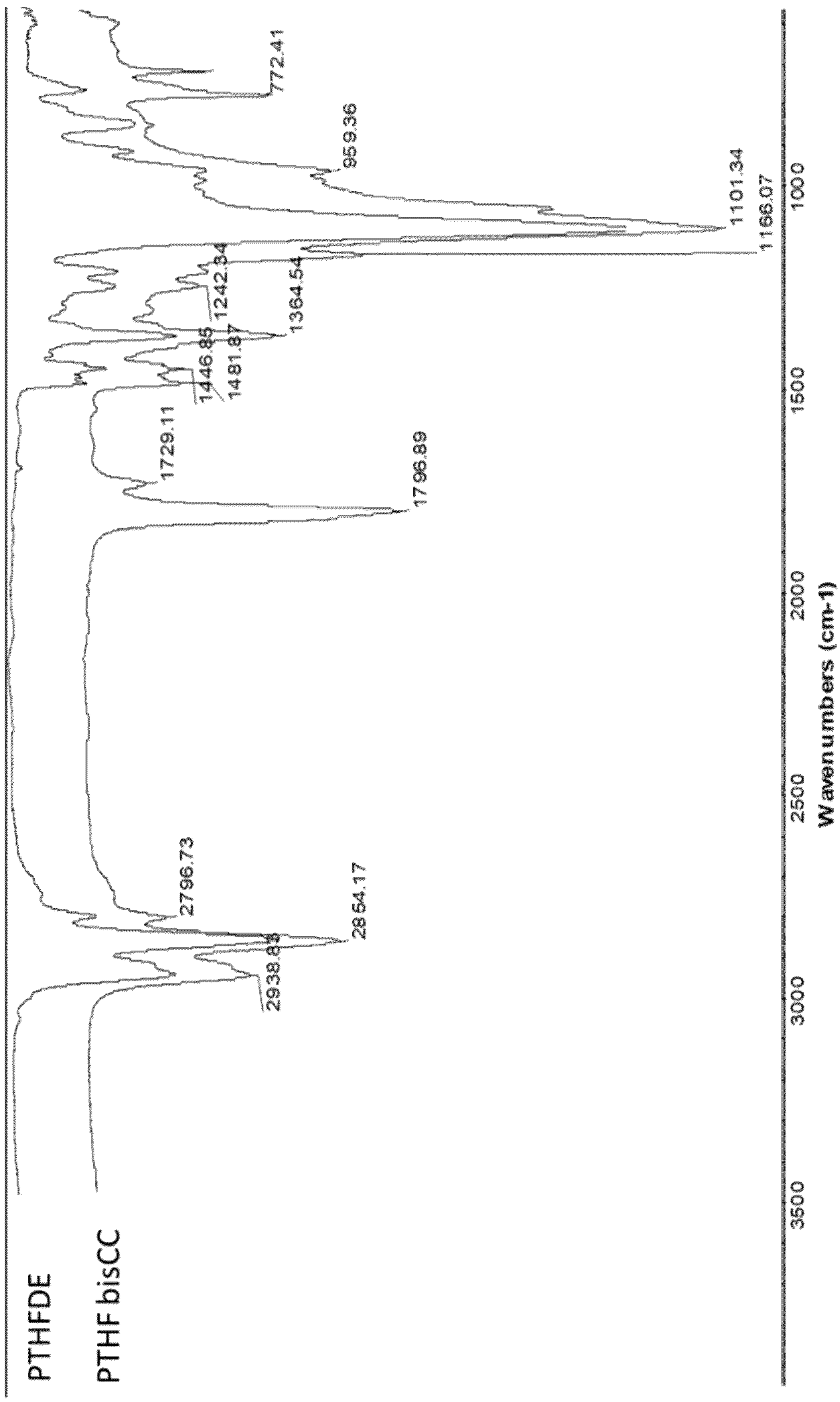
PTHFDE
PTHF bisCC
772.41
959.36
1101.34
1166.07
1242.34
1364.54
1446.85
1481.87
1729.11
1796.89
2796.73
2854.17
2938.85
1000
1500
2000
2500
3000
3500
Wavenumbers (cm-1)

Figure 5f

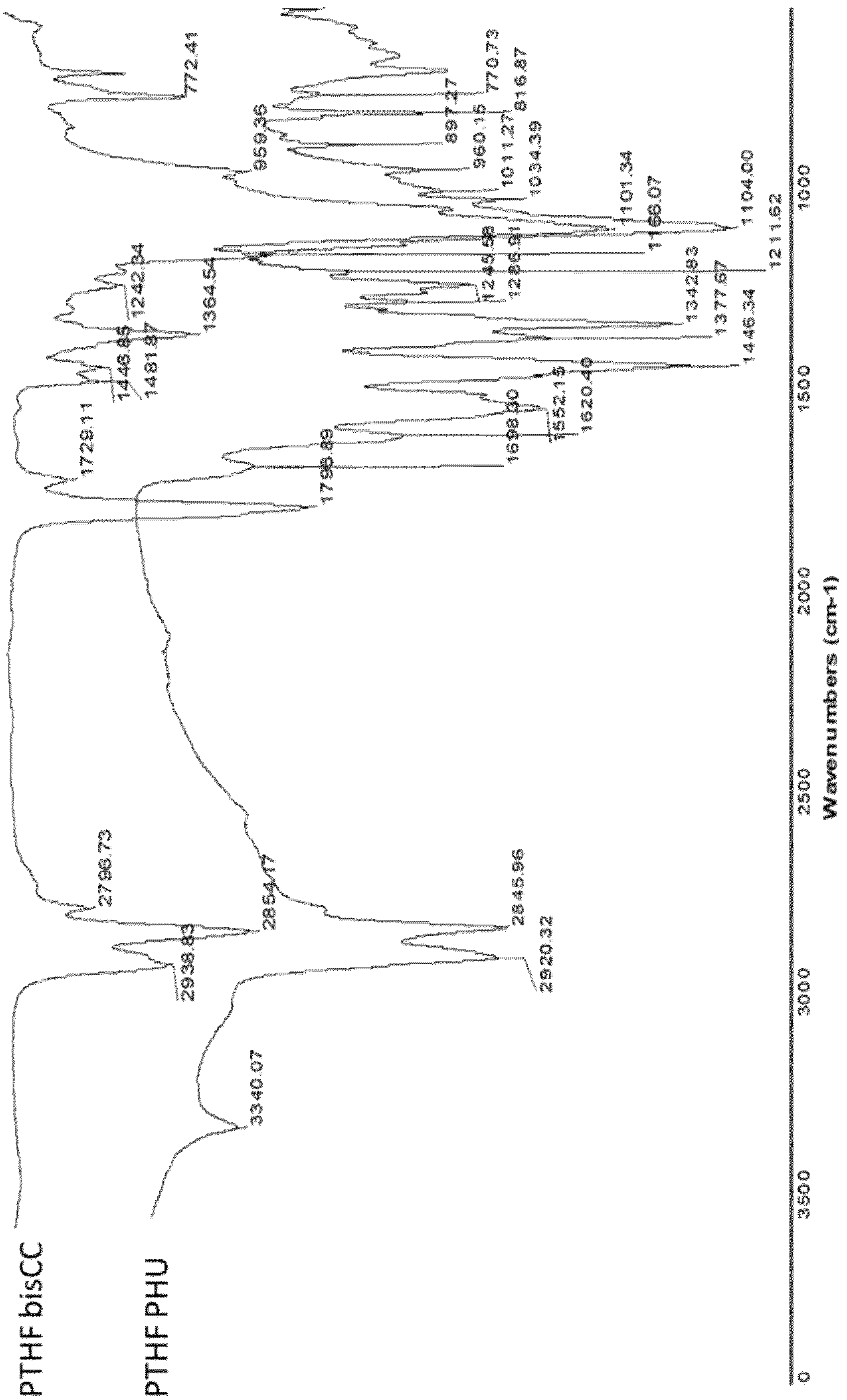
PTHF bisCC
PTHF PHU
Wavenumbers (cm-1)
0
500
1000
1500
2000
2500
3000
3500
772.41
959.36
1242.34
1364.54
1446.85
1481.87
1729.11
1796.89
2796.73
2854.17
2938.83
3340.07
2845.96
2920.32
770.73
816.87
897.27
960.15
1011.27
1034.39
1101.34
1166.07
1104.00
1211.62
1245.58
1286.91
1342.83
1377.67
1446.34
1552.15
1620.40
1698.30

Figure 5g

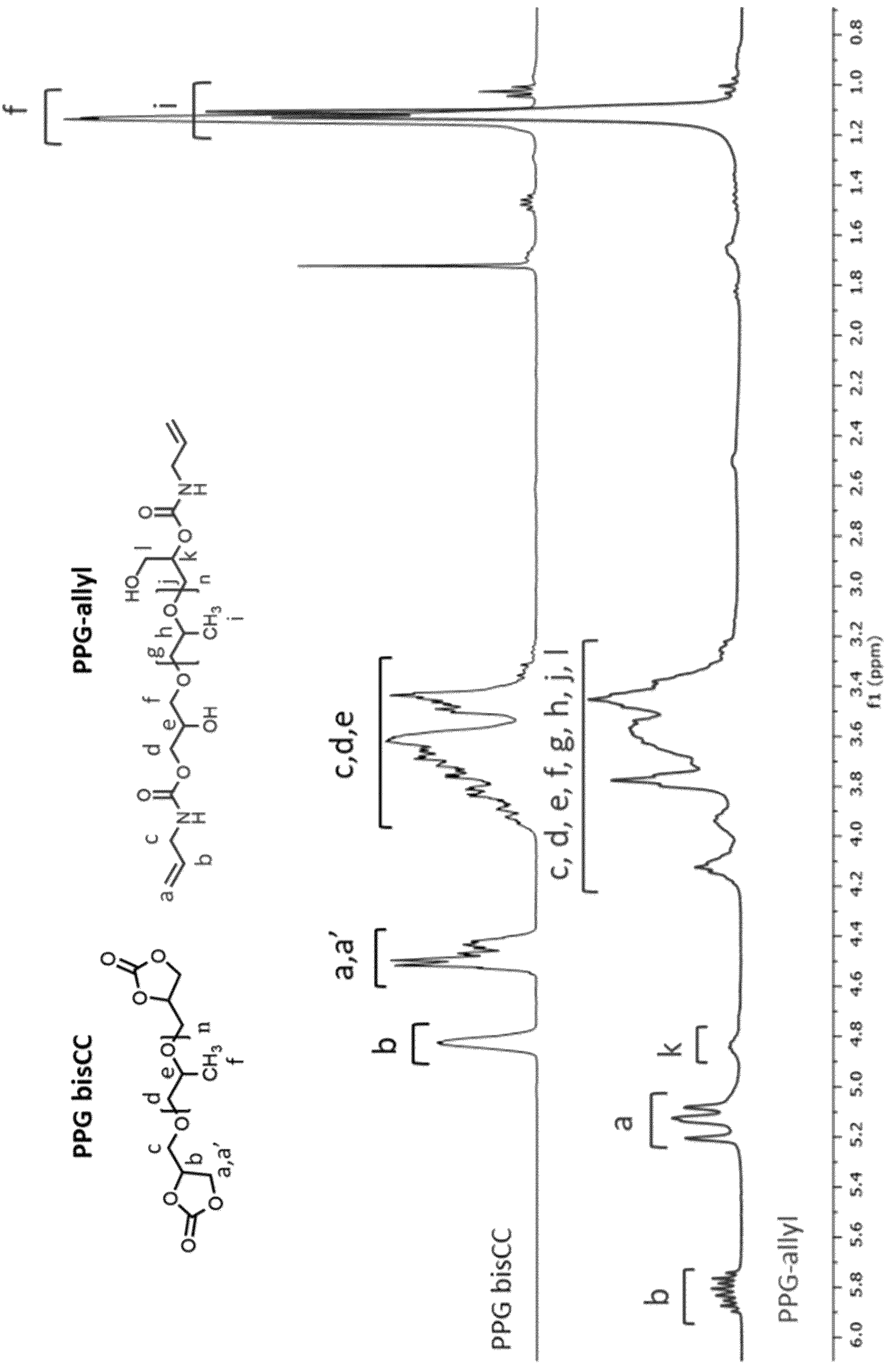


**Figure 5h**

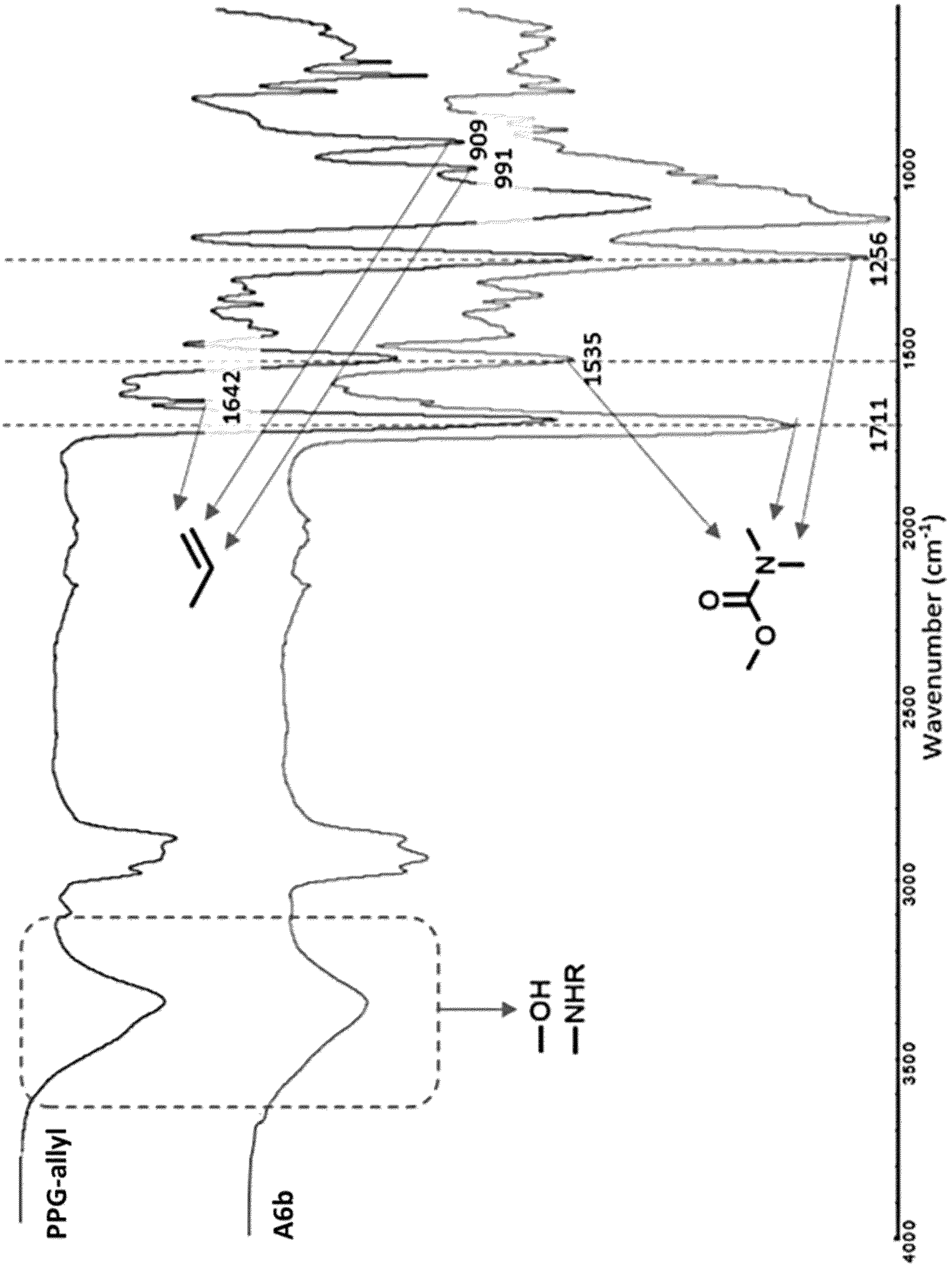
PPG-allyl
A6b
909
991
1642
1535
1256
1711
—OH
—NHR
1000
1500
2000
2500
3000
3500
4000
Wavenumber (cm-1)

**Figure 5i**

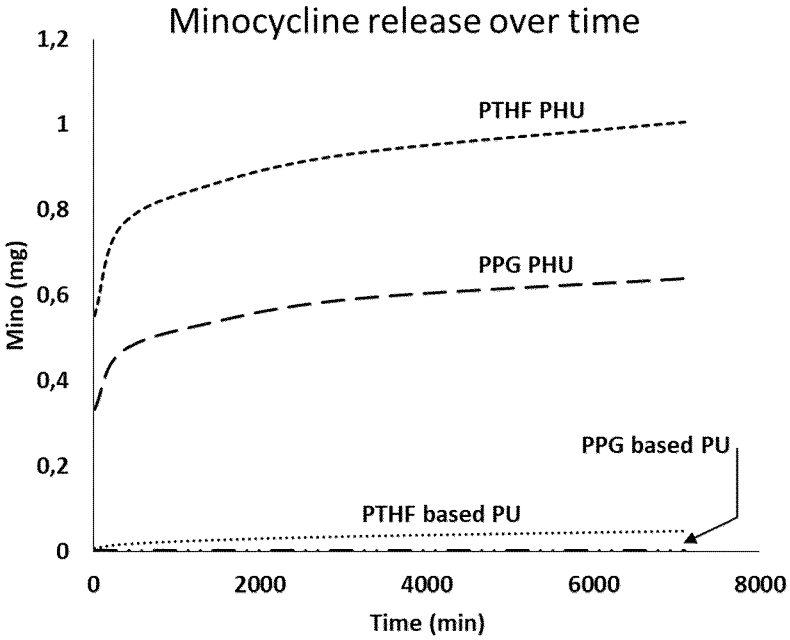


Figure 6

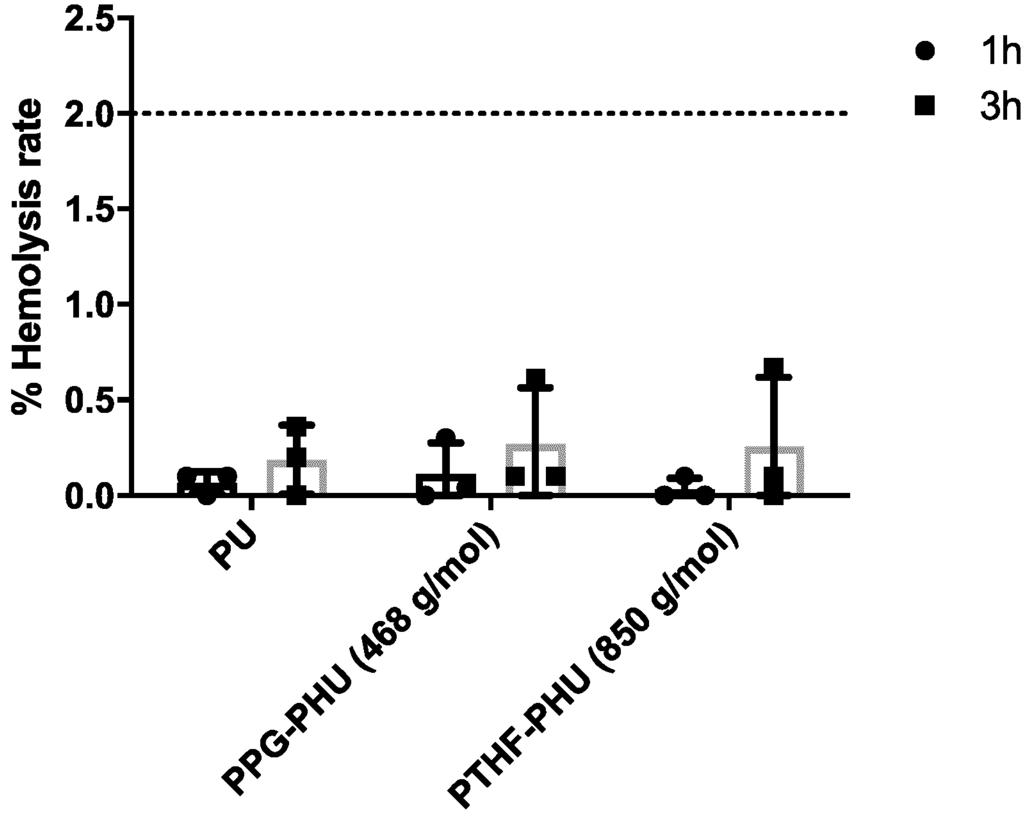


Figure 7

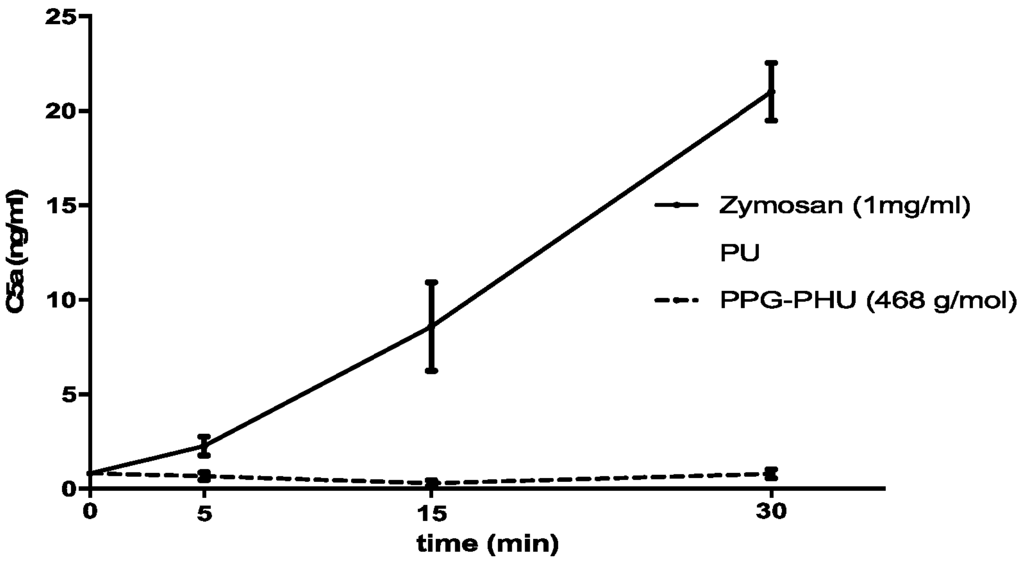


Figure 8

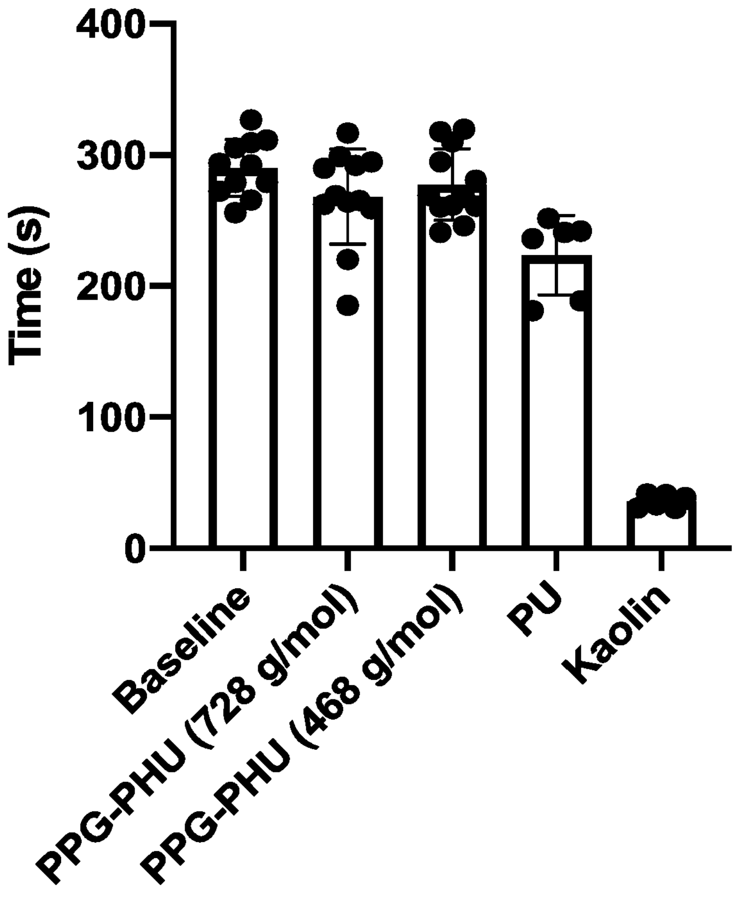


Figure 9

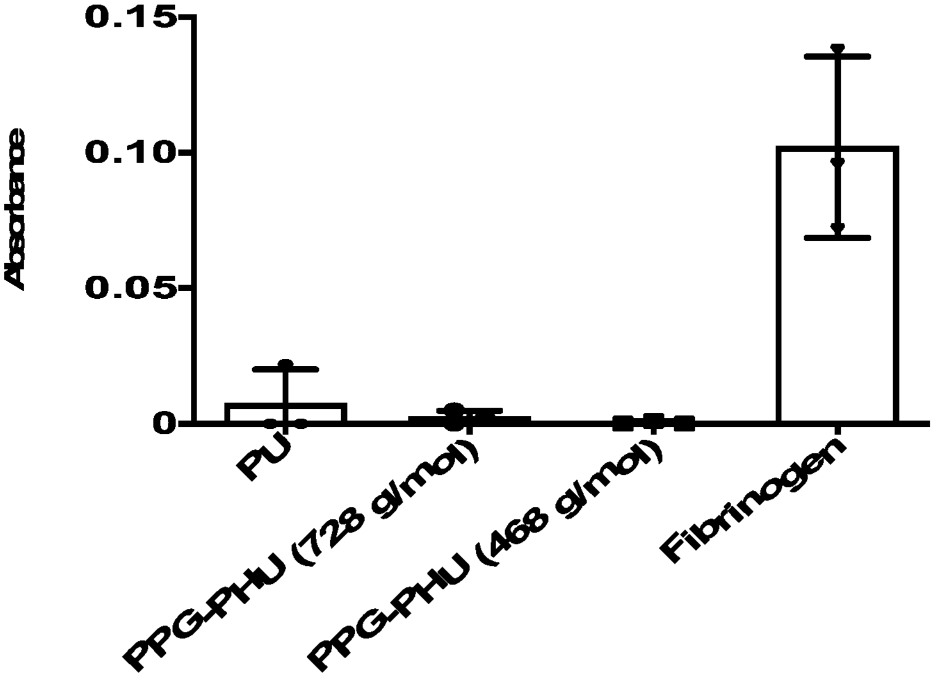


Figure 10

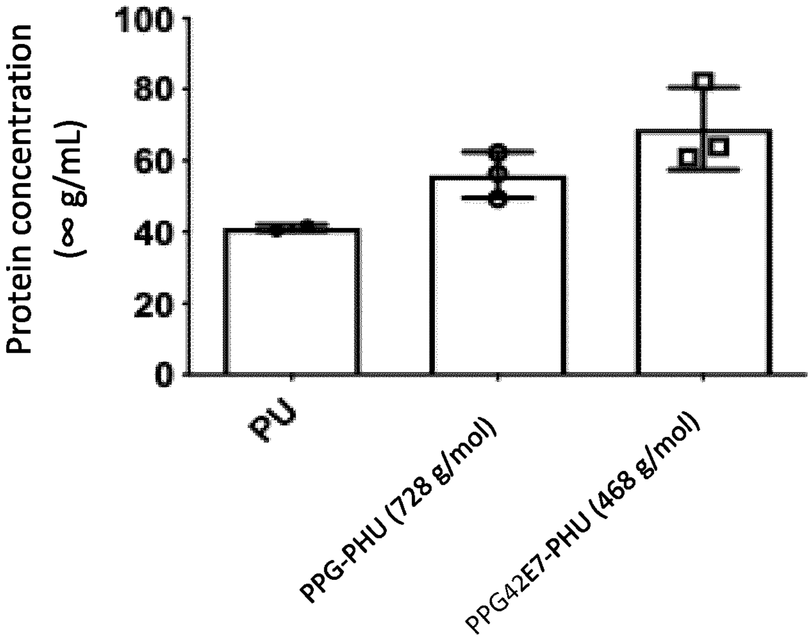


Figure 11

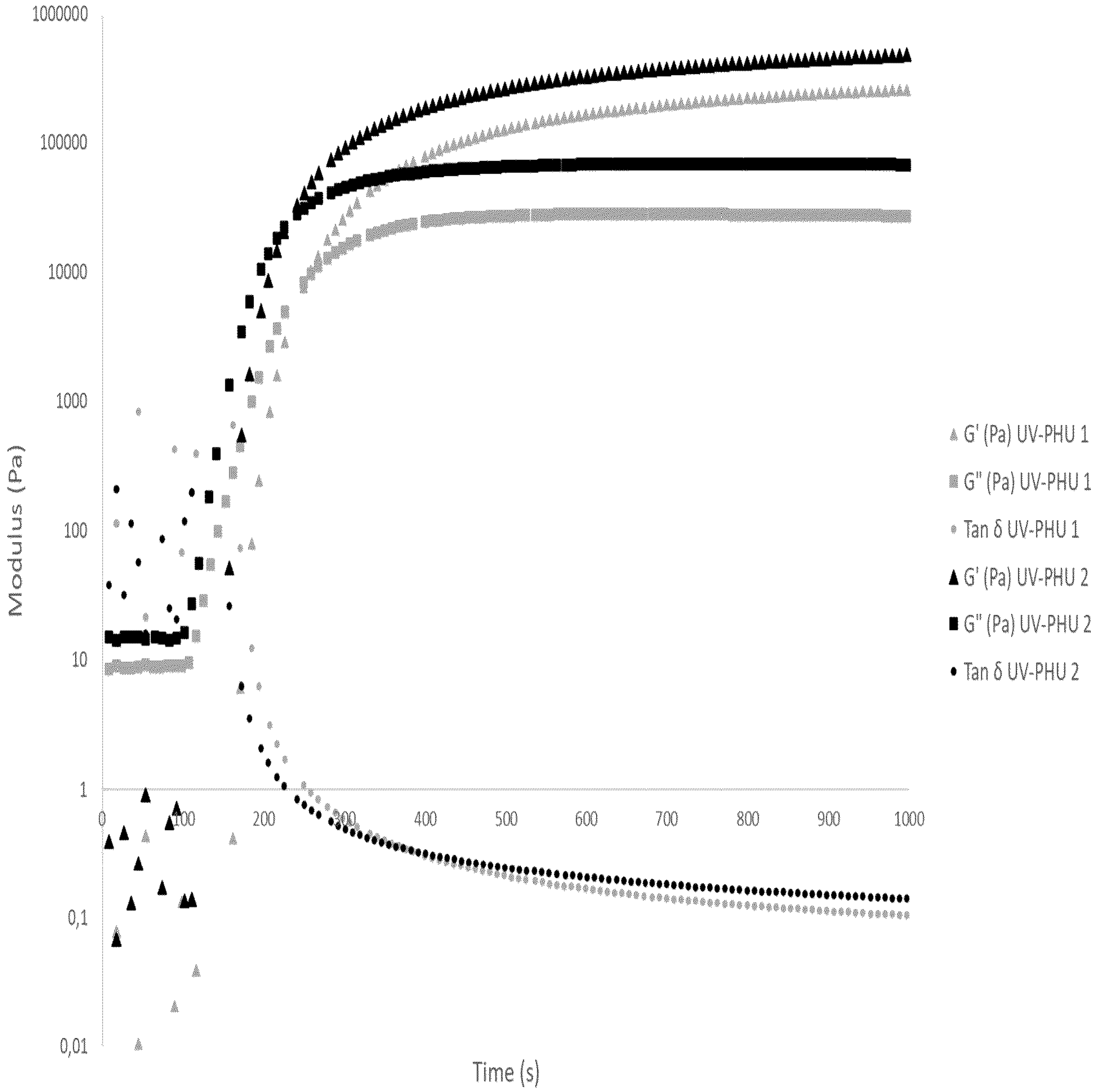
1000000
100000
10000
1000
100
10
1
0,1
0,01
Modulus (Pa)
0
100
200
300
400
500
600
700
800
900
1000
Time (s)
G' (Pa) UV-PHU 1
G'' (Pa) UV-PHU 1
Tan δ UV-PHU 1
G' (Pa) UV-PHU 2
G'' (Pa) UV-PHU 2
Tan δ UV-PHU 2

**Figure 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014185856 A
- WO 2013060950 A
- EP 3199569 A1
- WO 2021004993 A
- WO 2019158655 A
- EP 3292867 B1


### Non-patent literature cited in the description


- **HANNES BLATTMAN et al.** *Macromolecular Rapid Communication,* 30 July 2014, 1238-1254
- **GABRIEL ROCKIKI.** *Polymer for AdvancesTechnology,* 13 May 2015, 707-761
- **L.-N. HE.** One-pot stepwise synthesis of cyclic carbonates directly from olefins with CO2 promoted by K2S2O8/NaBr. *J. CO2 Util.,* 2016, vol. 16, 313-317
- **R. WANG.** Direct Synthetic Processes for Cyclic Carbonates from Olefins and CO. *Catal. Surv. from Asia,* 2011, vol. 15, 49-54
- **C. DETREMBLEUR.** Organocatalyzed coupling of carbon dioxide with epoxides for the synthesis of cyclic carbonates: catalyst design and mechanistic studies. *Catal. Sci. Technol.,* 2017, vol. 7, 2651
- **BLAIN et al.** *Green Chemistry,* 2014, vol. 16, 4286
- **TOTEA G et al.** *Cent. Eur. J. Chem.,* 2014, vol. 12 (7), 796-803